# EUROPEAN PATENT APPLICATION

(11) **EP 4 786 469 A1**
(43) Date of publication of application: **05.08.2026**
(21) Application number: 24870960.2
(22) Date of filing: 27.09.2024
(51) Int. Cl.: C07D 417/14, C07D 401/14, C07D 471/04, C12N 15/113, A61K 31/713, A61P 11/00

(54) **TRIAZOLE-CONTAINING COMPOUND, AND PREPARATION METHOD AND USE THEREFOR**

(30) Priority: 27.09.2023 CN 202311260117; 05.06.2024 CN 202410720335
(71) Applicant: Tuojie Biotech (Shanghai) Co., Ltd., Shanghai 201203 (CN)
(72) Inventor: TAN, Liang, Shanghai 201203 (CN); LI, Yunfei, Shanghai 201203 (CN); LIU, Min, Shanghai 201203 (CN); WANG, Yanhui, Shanghai 201203 (CN)
(74) Representative: Potter Clarkson
(86) International application number: PCT/CN2024/121746
(87) International publication number: WO 2025/067423

(57) **Abstract**

Provided are a triazole-containing compound, and a preparation method and use therefor. The triazole-containing compound can be used for promoting the connection of a targeting group, a pharmacokinetic (PK) enhancer or a modifier or other presenting agents to an oligonucleotide, and has improved reaction yield, stability and biological activity.

## Description

### TECHNICAL FIELD

The present disclosure relates to the field of pharmaceuticals and specifically relates to a triazolyl-containing compound, a preparation method therefor, and use thereof.

### BACKGROUND

Synthetic oligonucleotides, such as antisense compounds, aptamers, ribozymes, and RNA interference (RNAi) agents or molecules, are increasingly used for biomedical research, diagnosis, and treatment. These synthetic oligonucleotides have been used to inhibit or knock down gene expression in a sequence-dependent manner *in vitro, in situ,* and *in vivo.* RNA interference is an effective way to silence gene expression. Statistically, about more than 80% of the disease-related proteins in humans are non-druggable proteins as they cannot be targeted by conventional small-molecule drugs and biomacromolecule formulations. By using the RNA interference technology, proper siRNAs can be designed according to the mRNAs encoding these proteins to specifically target and degrade the target mRNAs, thereby inhibiting the generation of the related proteins. Therefore, siRNAs have very important prospects for drug development. However, to achieve therapeutic RNA interference effects *in vivo,* it is necessary to deliver siRNA molecules to particular cells in the body.

A commonly used strategy is to link targeting groups or other pharmacologically active molecules, optionally by linking chains, to synthetic oligonucleotides, for *in vivo* delivery. The linking chains used should be able to adapt to different synthetic oligonucleotides, as well as different targeting groups or pharmacologically active molecules. In addition, the linking chains are non-toxic and do not produce toxic or other detrimental by-products. Moreover, the linking chains should have the characteristics of being readily cleavable at the targeted site (e.g., a target cell) and remaining stable at sites other than the targeted site (e.g., in circulation, subcutaneous spaces, or extracellular spaces).

### SUMMARY

The present disclosure provides an alkynyl-containing compound represented by formula I or a pharmaceutically acceptable salt thereof, 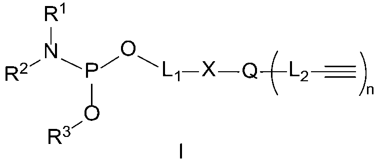
wherein R¹, R², and R³ are each independently C₁₋₆ alkyl optionally substituted with one or more substituents, and the substituents are halogen, hydroxy, or cyano;
L₁ is C₁₋₁₀ alkyl optionally interrupted by one or more groups selected from the group consisting of -O-, -S-, -N-, and -C(=O)-;
X is a bond, -C(=O), -C(=O)-NH-, or -NH-C(=O)-;
Q is 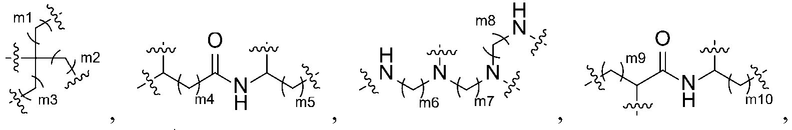 or 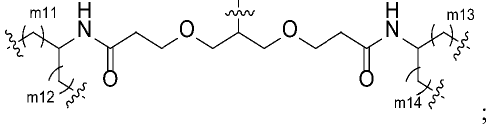
L₂ are each independently -O-(CH₂)ₚ₁-C(=O)-NH-(CH₂CH₂O)ₚ₂-(CH₂)ₚ₃, -C(=O)-NH-(CH₂CH₂O)ₚ₄-(CH₂)ₚ₅, or -C(=O)-(CH₂CH₂O)ₚ₆-(CH₂)ₚ₇;
n is 3 or 4;
m1, m2, m3, m4, m5, m6, m7, m8, m9, m10, m11, m12, m13, and m14 are each independently 0, 1, 2, 3, 4, 5, or 6;
p1, p2, p3, p4, p5, p6, and p7 are each independently 0, 1, 2, 3, or 4;
provided that when Q is 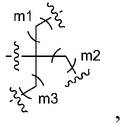 L₂ are not simultaneously -C(=O)-NH-(CH₂CH₂O)ₚ₄-(CH₂)ₚ₅.

In some embodiments, R¹, R², and R³ are each independently C₁₋₄ alkyl optionally substituted with one or more substituents, and the substituents are halogen, hydroxy, or cyano.

In some embodiments, R¹, R², and R³ are each independently methyl, ethyl, propyl, or isopropyl optionally substituted with one or more substituents, and the substituents are fluorine, chlorine, bromine, hydroxy, or cyano.

In some embodiments, R¹ and R² are both isopropyl.

In some embodiments, R³ is

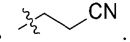

In some embodiments, Q is 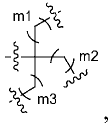 L₂ are each independently -O-(CH₂)ₚ₁-C(=O)-NH-(CH₂CH₂O)ₚ₂-(CH₂)ₚ₃, and m1, m2, m3, p1, p2, and p3 are as defined in formula I.

In some embodiments, Q is 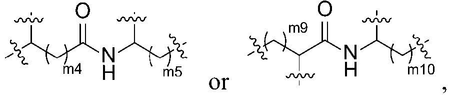 L₂ are each independently -C(=O)-NH-(CH₂CH₂O)ₚ₄-(CH₂)ₚ₅, and m4, m5, m9, m10, p4, and p5 are as defined in formula I.

In some embodiments, Q is 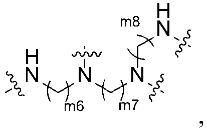 L₂ are each independently -C(=O)-(CH₂CH₂O)ₚ₆-(CH₂)ₚ₇, and m6, m7, m8, p6, and p7 are as defined in formula I.

In some embodiments, Q is 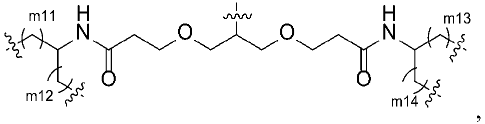 L₂ are each independently -O-(CH₂)ₚ₁-C(=O)-NH-(CH₂CH₂O)ₚ₂-(CH₂)ₚ₃, and m11, m12, m13, m14, p1, p2, and p3 are as defined in formula I.

In some embodiments, L₂ are identical.

In some embodiments, L₁ is C₁₋₁₀ alkyl optionally interrupted by one or more groups selected from the group consisting of -O-, -N-, and -C(=O)-.

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10).

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10), wherein the right side of -(CH₂)_{q1}-, -(CH₂)_{q2}-NH-C(=O)-(CH₂)_{q3}-, or -(CH₂)_{q4}-O-(CH₂)_{q5}- is linked to X.

In some embodiments, L₁ is -(CH₂)₆-, -(CH₂)₆-NH-C(=O)-(CH₂)₃-, or -(CH₂)₆-O-CH₂-. In some embodiments, L₁ is -(CH₂)₆-, -(CH₂)₆-NH-C(=O)-(CH₂)₃-, or -(CH₂)₆-O-CH₂-, wherein the right side of -(CH₂)₆-, -(CH₂)₆-NH-C(=O)-(CH₂)₃-, or -(CH₂)₆-O-CH₂- is linked to X.

In some embodiments, Q is 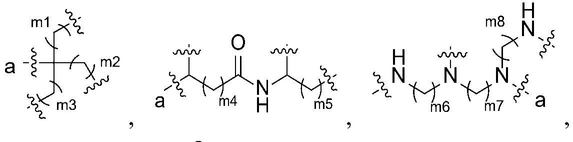 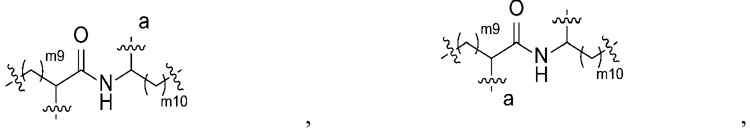 or 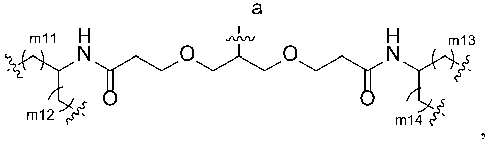 wherein end a is linked to X, and m1, m2, m3, m4, m5, m6, m7, m8, m9, m10, m11, m12, m13, and m14 are as defined in formula I.

In some embodiments, Q is

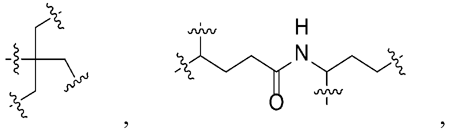

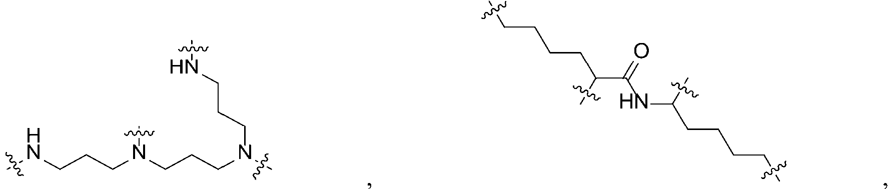

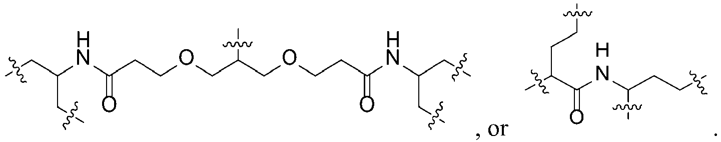

In some embodiments, Q is 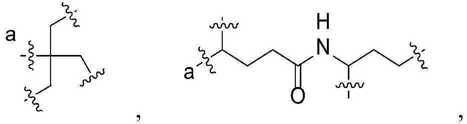 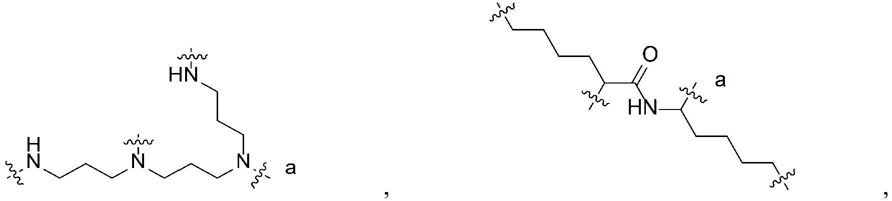 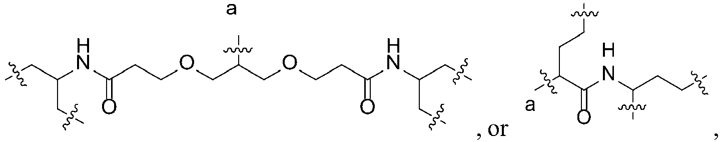 wherein end a is linked to X.

In some embodiments, L₂ are each independently

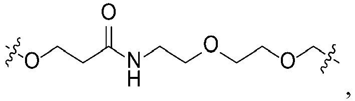

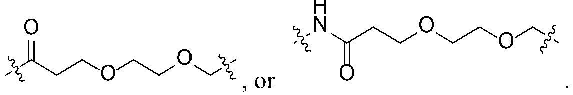

In some embodiments, L₂ are each independently 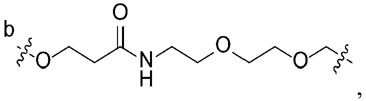 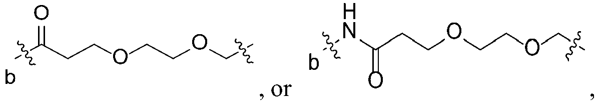 wherein end b is linked to Q.

The present disclosure provides an alkynyl-containing compound or a pharmaceutically acceptable salt thereof, wherein the alkynyl-containing compound is selected from the group consisting of: 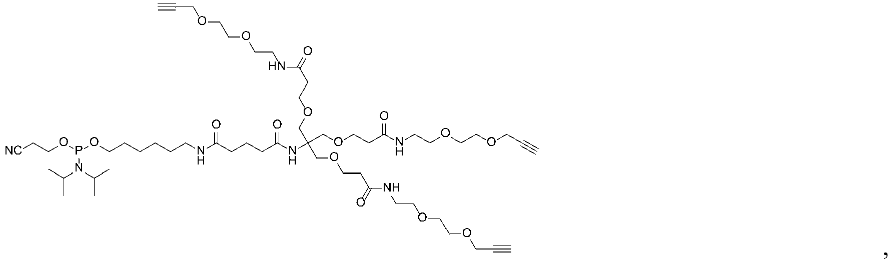 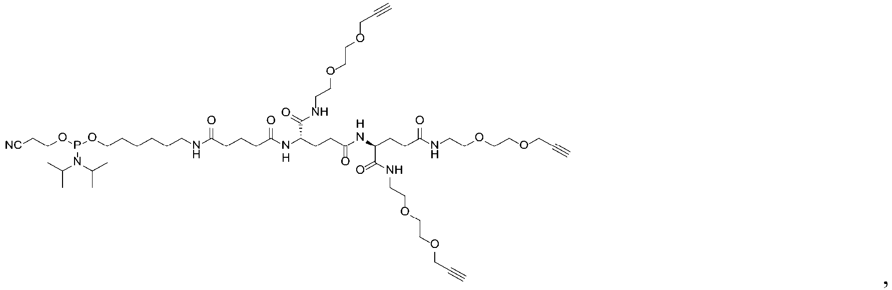 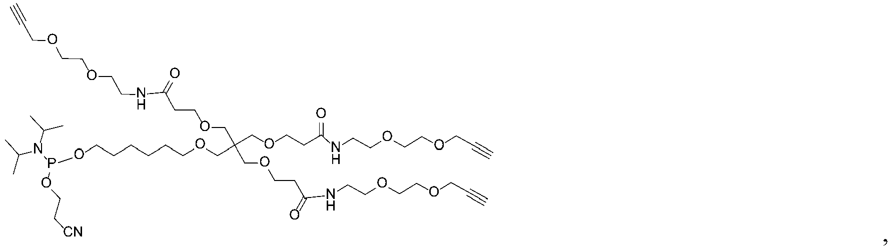 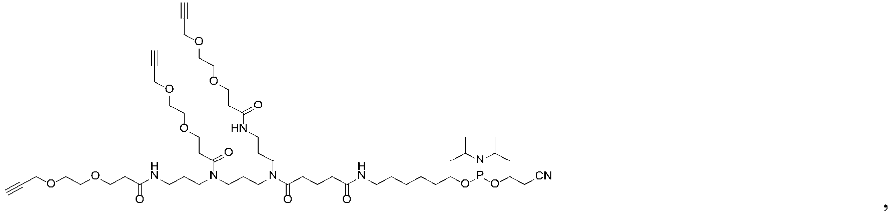 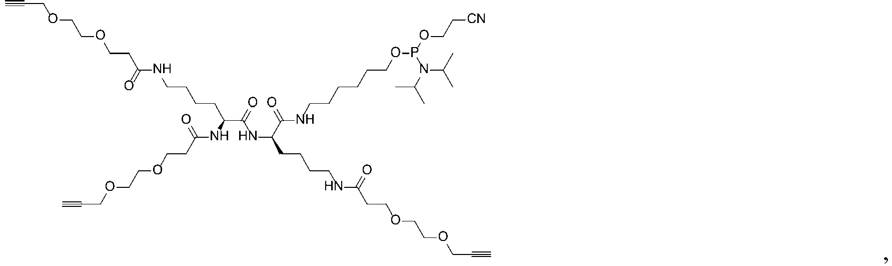 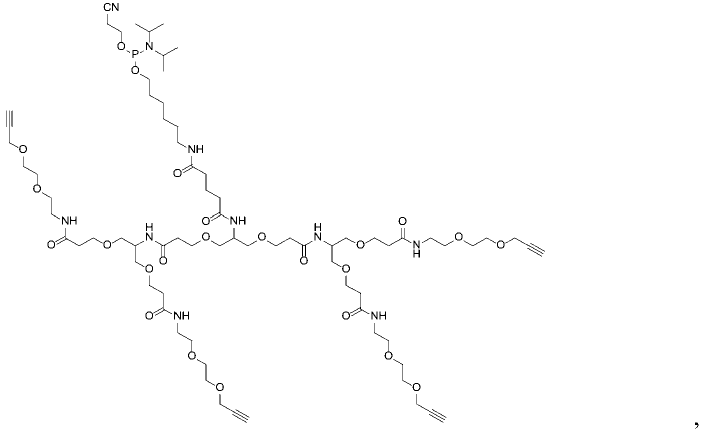 and

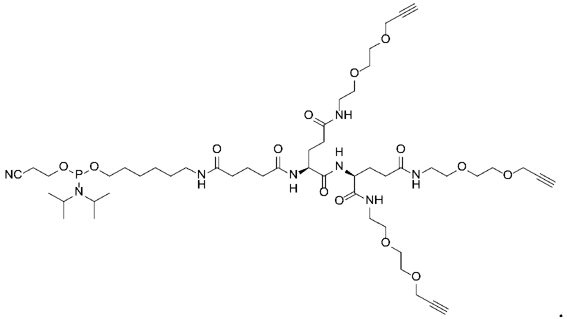

The present disclosure provides a conjugate of a nucleic acid and an alkynyl-containing moiety, or a pharmaceutically acceptable salt thereof, wherein the conjugate comprises a nucleic acid and one or more alkynyl-containing moieties, and the alkynyl-containing moieties are identical or different and have a structure represented by formula II, 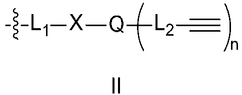
wherein L₁ is C₁₋₁₀ alkyl optionally interrupted by one or more groups selected from the group consisting of -O-, -S-, -N-, and -C(=O)-;
X is a bond, -C(=O), -C(=O)-NH-, or -NH-C(=O)-;
Q is 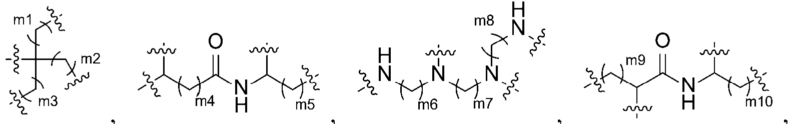 or 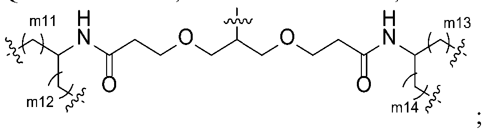
L₂ are each independently -O-(CH₂)ₚ₁-C(=O)-NH-(CH₂CH₂O)ₚ₂-(CH₂)ₚ₃, -C(=O)-NH-(CH₂CH₂O)ₚ₄-(CH₂)ₚ₅, or -C(=O)-(CH₂CH₂O)ₚ₆-(CH₂)ₚ₇;
n is 3 or 4;
m1, m2, m3, m4, m5, m6, m7, m8, m9, m10, m11, m12, m13, and m14 are each independently 0, 1, 2, 3, 4, 5, or 6;
p1, p2, p3, p4, p5, p6, and p7 are each independently 0, 1, 2, 3, or 4;
provided that when Q is 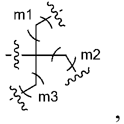 L₂ are not simultaneously -C(=O)-NH-(CH₂CH₂O)ₚ₄-(CH₂)ₚ₅.

In some embodiments, Q is 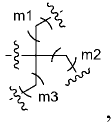 L₂ are each independently -O-(CH₂)ₚ₁-C(=O)-NH-(CH₂CH₂O)ₚ₂-(CH₂)ₚ₃, and m1, m2, m3, p1, p2, and p3 are as defined in formula II.

In some embodiments, Q is 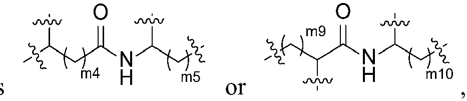 L₂ are each independently -C(=O)-NH-(CH₂CH₂O)ₚ₄-(CH₂)ₚ₅, and m4, m5, m9, m10, p4, and p5 are as defined in formula II.

In some embodiments, Q is 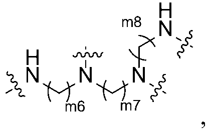 L₂ are each independently -C(=O)-(CH₂CH₂O)ₚ₆-(CH₂)ₚ₇, and m6, m7, m8, p6, and p7 are as defined in formula II.

In some embodiments, Q is 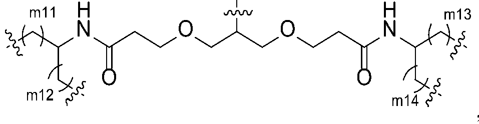 L₂ are each independently -O-(CH₂)ₚ₁-C(=O)-NH-(CH₂CH₂O)ₚ₂-(CH₂)ₚ₃, and m11, m12, m13, m14, p1, p2, and p3 are as defined in formula II.

In some embodiments, L₂ are identical.

In some embodiments, L₁ is C₁₋₁₀ alkyl optionally interrupted by one or more groups selected from the group consisting of -O-, -N-, and -C(=O)-.

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10).

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10), wherein the right side of -(CH₂)_{q1}-, -(CH₂)_{q2}-NH-C(=O)-(CH₂)_{q3}-, or -(CH₂)_{q4}-O-(CH₂)_{q5}- is linked to X.

In some embodiments, L₁ is -(CH₂)₆-, -(CH₂)₆-NH-C(=O)-(CH₂)₃-, or -(CH₂)₆-O-CH₂-. In some embodiments, L₁ is -(CH₂)₆-, -(CH₂)₆-NH-C(=O)-(CH₂)₃-, or -(CH₂)₆-O-CH₂-, wherein the right side of -(CH₂)₆-, -(CH₂)₆-NH-C(=O)-(CH₂)₃-, or -(CH₂)₆-O-CH₂- is linked to X.

In some embodiments, Q is 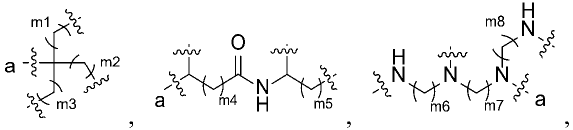 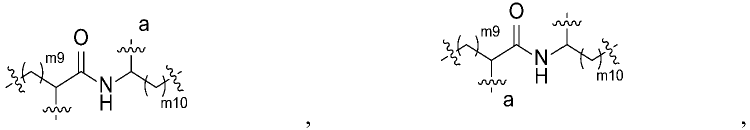 or 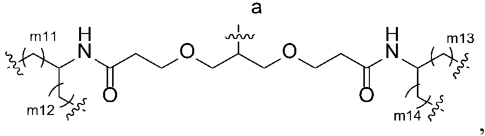 wherein end a is linked to X, and m1, m2, m3, m4, m5, m6, m7, m8, m9, m10, m11, m12, m13, and m14 are as defined in formula II.

In some embodiments, Q is

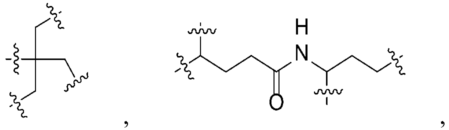

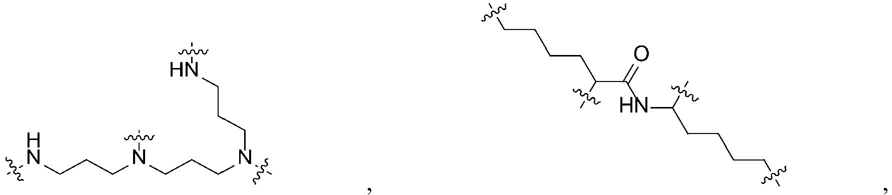

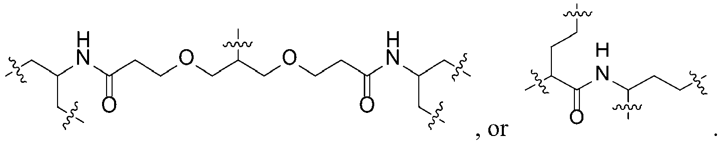

In some embodiments, Q is 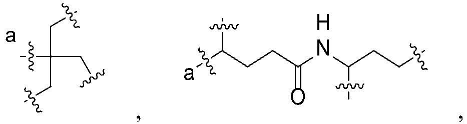 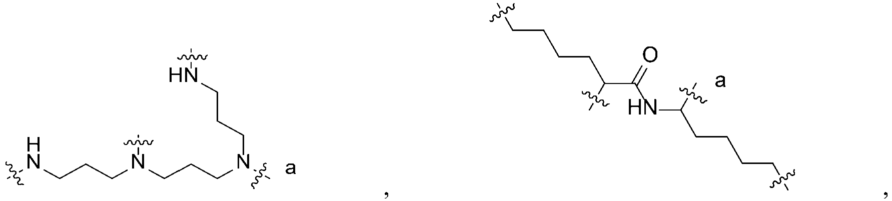 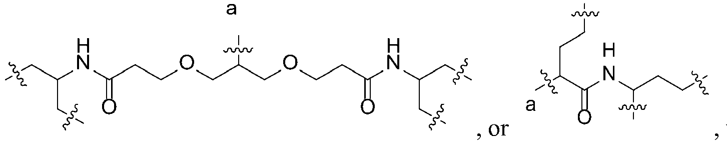 wherein end a is linked to X.

In some embodiments, L₂ are each independently

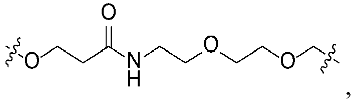

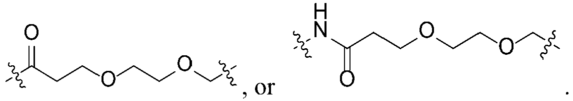

In some embodiments, L₂ are each independently 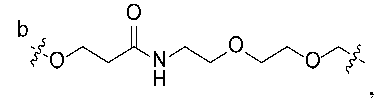 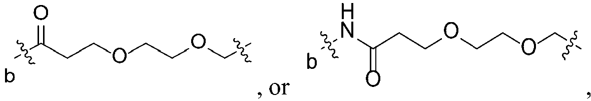 wherein end b is linked to Q.

In some embodiments, the nucleic acid and the alkynyl-containing moieties are linked by phosphoester groups, phosphorothioate groups, or phosphonic acid groups.

In some embodiments, the nucleic acid is linked to the alkynyl-containing moieties by phosphoester groups.

In some embodiments, the nucleic acid is linked to the alkynyl-containing moieties by phosphorothioate groups.

In some embodiments, the phosphoester groups are phosphodiester groups.

In some embodiments, the phosphorothioate groups are phosphorothioate diester groups. In some embodiments, the nucleic acid includes, but is not limited to, single-stranded oligonucleotides, single-stranded antisense oligonucleotides, short interfering RNAs (siRNAs, the siRNAs comprising partially complementary sense and antisense strands), double-stranded RNAs (dsRNAs), microRNAs (miRNAs), short hairpin RNAs (shRNAs), and Dicer substrates.

In some embodiments, a 3' end of the nucleic acid is conjugated to an alkynyl-containing moiety.

In some embodiments, a 5' end of the nucleic acid is conjugated to an alkynyl-containing moiety.

In some embodiments, a 3' end and a 5' end of the nucleic acid are both conjugated to alkynyl-containing moieties.

The aforementioned 3' end and/or 5' end of the nucleic acid conjugated to the alkynyl-containing moieties refer(s) to the 3' end and/or 5' end of a nucleic acid strand conjugated to the alkynyl-containing moieties.

In some specific embodiments, the nucleic acid is an siRNA in which the 5' end of the sense strand is conjugated to an alkynyl-containing moiety.

In some embodiments, the nucleic acid is a single-stranded nucleic acid.

In some embodiments, the single-stranded nucleic acid is the sense strand of an siRNA.

In some embodiments, the single-stranded nucleic acid is the antisense strand of an siRNA.

In some embodiments, the nucleic acid is a double-stranded nucleic acid.

In some embodiments, the double-stranded nucleic acid comprises at least one duplex region within which a first strand nucleic acid is at least partially complementary to a second strand nucleic acid, wherein:
(1) neither end of the first strand nucleic acid is conjugated to an alkynyl-containing moiety; and the 5' end of the second strand nucleic acid is conjugated to an alkynyl-containing moiety, and the 3' end is not conjugated to an alkynyl-containing moiety; or the 3' end of the second strand nucleic acid is conjugated to an alkynyl-containing moiety, and the 5' end is not conjugated to an alkynyl-containing moiety; or both the 3' end and the 5' end of the second strand nucleic acid are conjugated to alkynyl-containing moieties;
(2) neither end of the second strand nucleic acid is conjugated to an alkynyl-containing moiety; and the 5' end of the first strand nucleic acid is conjugated to an alkynyl-containing moiety, and the 3' end is not conjugated to an alkynyl-containing moiety; or the 3' end of the first strand nucleic acid is conjugated to an alkynyl-containing moiety, and the 5' end is not conjugated to an alkynyl-containing moiety; or both the 3' end and the 5' end of the first strand nucleic acid are conjugated to alkynyl-containing moieties;
(3) both the 5' ends of the first strand nucleic acid and the second strand nucleic acid are conjugated to alkynyl-containing moieties, and neither 3' end is conjugated to an alkynyl-containing moiety; or both the 3' ends of the first strand nucleic acid and the second strand nucleic acid are conjugated to alkynyl-containing moieties, and neither 5' end is conjugated to an alkynyl-containing moiety; or both the 3' ends and the 5' ends of the first strand nucleic acid and the second strand nucleic acid are conjugated to alkynyl-containing moieties; or both the 3' end of the first strand nucleic acid and the 5' end of the second strand nucleic acid are conjugated to alkynyl-containing moieties, and neither the 5' end of the first strand nucleic acid nor the 3' end of the second strand nucleic acid is conjugated to an alkynyl-containing moiety; or both the 5' end of the first strand nucleic acid and the 3' end of the second strand nucleic acid are conjugated to alkynyl-containing moieties, and neither the 3' end of the first strand nucleic acid nor the 5' end of the second strand nucleic acid is conjugated to an alkynyl-containing moiety;
(4) both the 3' end and the 5' end of the first strand nucleic acid are conjugated to alkynyl-containing moieties, and either the 5' end or the 3' end of the second strand nucleic acid is conjugated to an alkynyl-containing moiety; or both the 3' end and the 5' end of the second strand nucleic acid are conjugated to alkynyl-containing moieties, and either the 5' end or the 3' end of the first strand nucleic acid is conjugated to an alkynyl-containing moiety.

In some embodiments, the nucleic acid is an siRNA.

In some embodiments, the nucleic acid is an siRNA targeting RAGE.

In some embodiments, the nucleic acid is an siRNA targeting MUC5B.

In some embodiments, the nucleic acid is an siRNA targeting APP.

In some embodiments, the nucleic acid comprises one or more modified nucleotides.

In some embodiments, the conjugate of the nucleic acid and the alkynyl-containing moiety is represented by formula II-A, 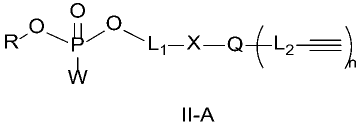 wherein R is the nucleic acid; W is OH, SH, O⁻, or S⁻; L₁, L₂, X, Q, and n are as defined in formula II.

In some embodiments, W is OH or O⁻.

In some embodiments, W is SH or S⁻.

The present disclosure provides a conjugate of a nucleic acid and an alkynyl-containing moiety, or a pharmaceutically acceptable salt thereof, wherein the conjugate of the nucleic acid and the alkynyl-containing moiety is selected from the group consisting of the following structures: 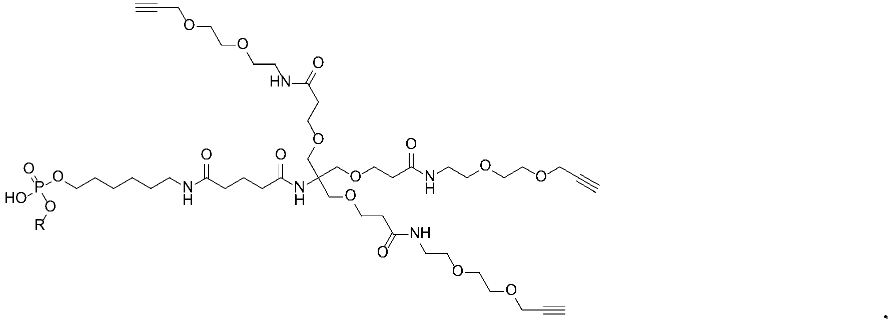 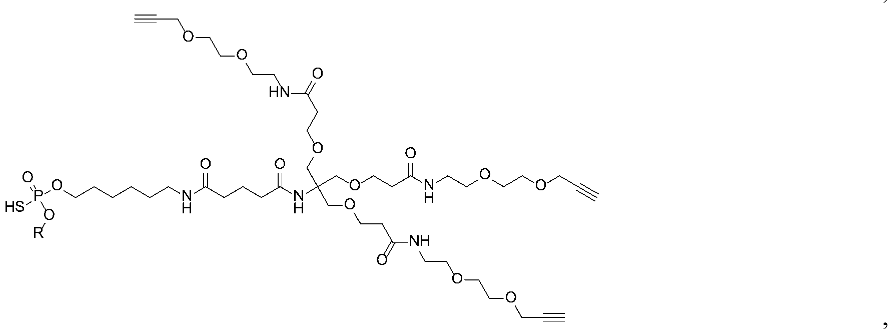 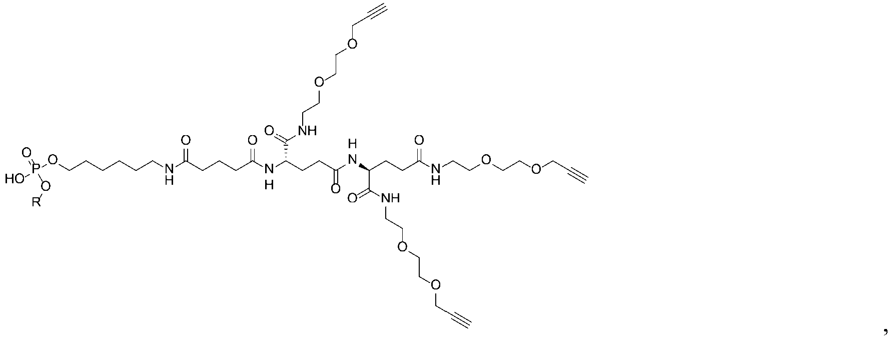 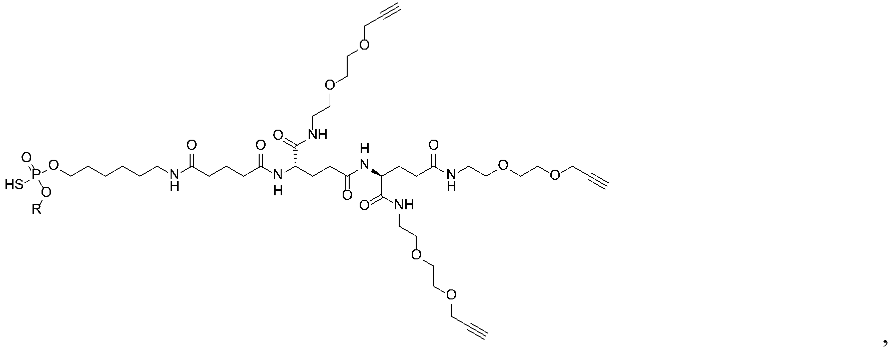 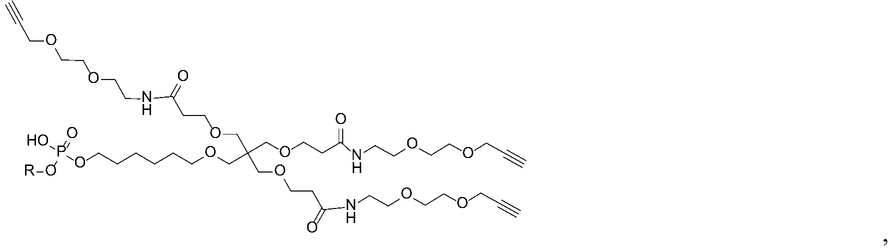 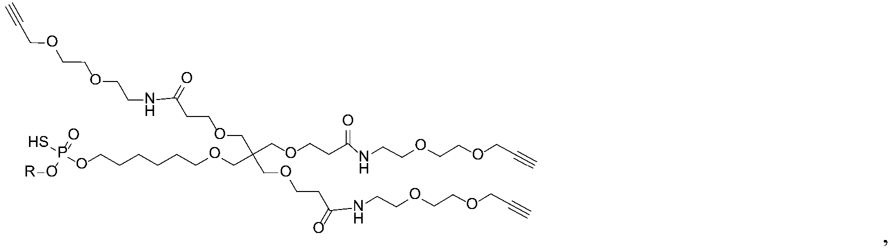 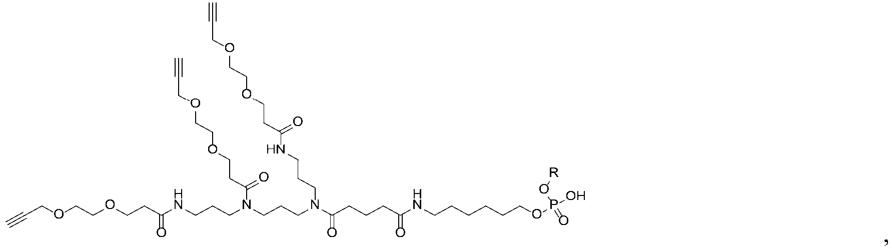 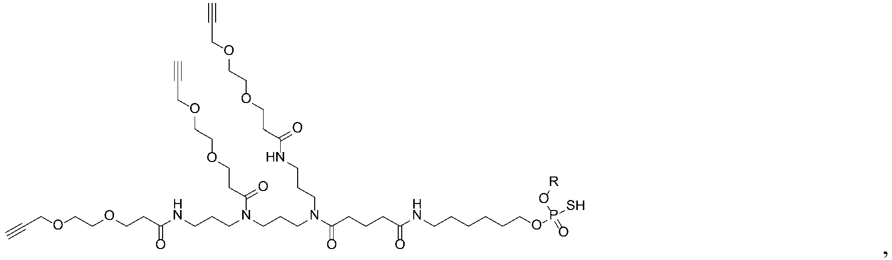 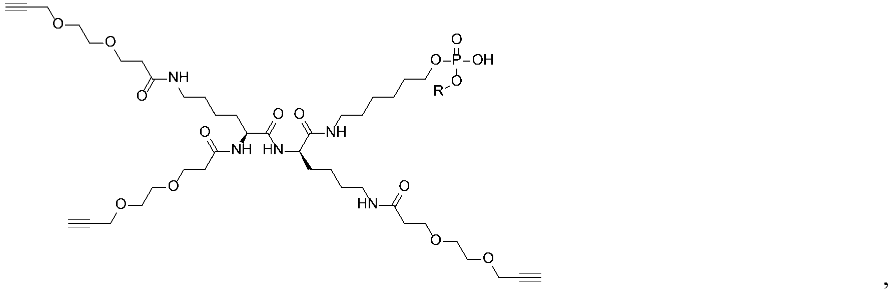 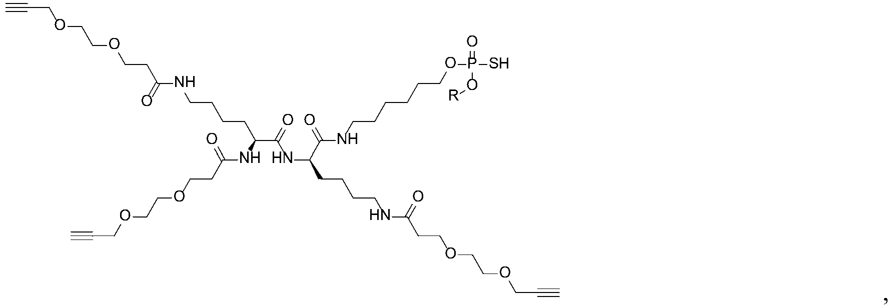 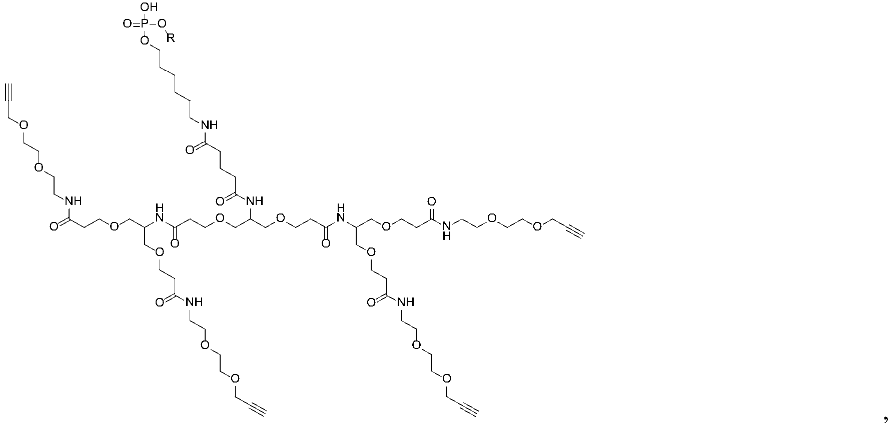 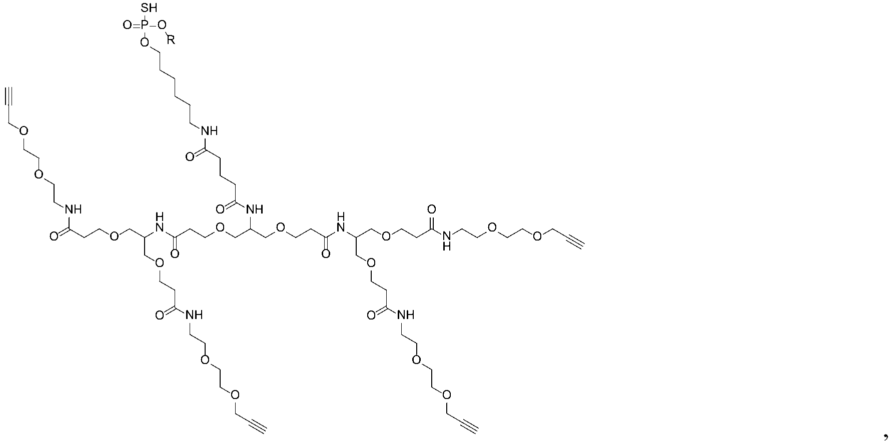 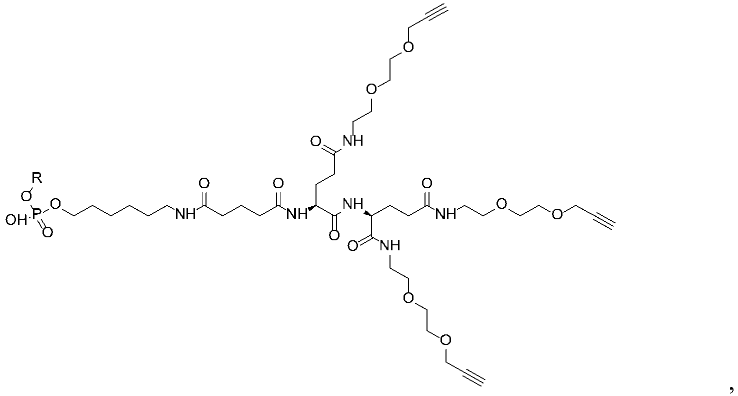 and 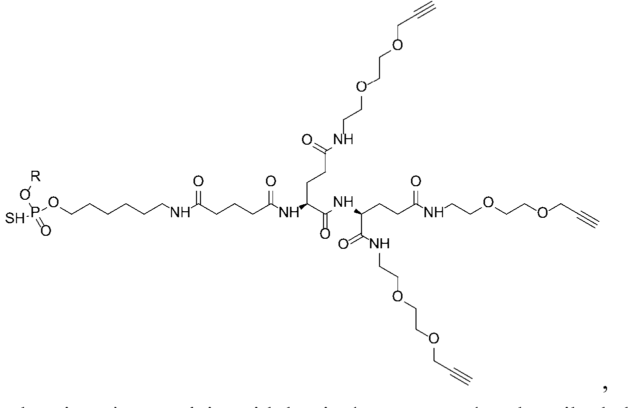 wherein R is a nucleic acid that is the same as that described above.

In some embodiments, a 5' end of the nucleic acid is conjugated to an alkynyl-containing moiety.

The present disclosure provides a targeting ligand represented by formula III or a pharmaceutically acceptable salt thereof, 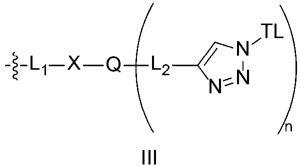
wherein L₁ is C₁₋₁₀ alkyl optionally interrupted by one or more groups selected from the group consisting of -O-, -S-, -N-, and -C(=O)-;
X is a bond, -C(=O), -C(=O)-NH-, or -NH-C(=O)-;
Q is 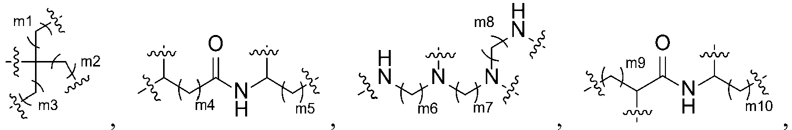 or 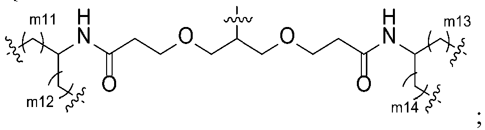
L₂ are each independently -O-(CH₂)ₚ₁-C(=O)-NH-(CH₂CH₂O)ₚ₂-(CH₂)ₚ₃, -C(=O)-NH-(CH₂CH₂O)ₚ₄-(CH₂)ₚ₅, or -C(=O)-(CH₂CH₂O)ₚ₆-(CH₂)ₚ₇;
TL are each independently a targeting group;
n is 3 or 4;
m1, m2, m3, m4, m5, m6, m7, m8, m9, ml0, m11, m12, m13, and m14 are each independently 0, 1, 2, 3, 4, 5, or 6;
p1, p2, p3, p4, p5, p6, and p7 are each independently 0, 1, 2, 3, or 4;
provided that when Q is 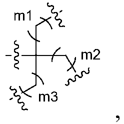 L₂ are not simultaneously -C(=O)-NH-(CH₂CH₂O)ₚ₄-(CH₂)ₚ₅.

In some embodiments, Q is 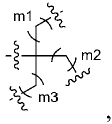 L₂ are each independently -O-(CH₂)ₚ₁-C(=O)-NH-(CH₂CH₂O)ₚ₂-(CH₂)ₚ₃, and m1, m2, m3, p1, p2, and p3 are as defined in formula III.

In some embodiments, Q is 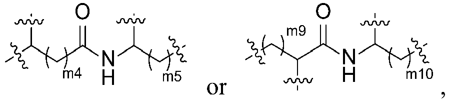 L₂ are each independently -C(=O)-NH-(CH₂CH₂O)ₚ₄-(CH₂)ₚ₅, and m4, m5, m9, m10, p4, and p5 are as defined in formula III.

In some embodiments, Q is 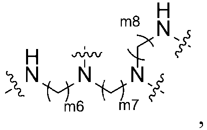 L₂ are each independently -C(=O)-(CH₂CH₂O)ₚ₆-(CH₂)ₚ₇, and m6, m7, m8, p6, and p7 are as defined in formula III.

In some embodiments, Q is 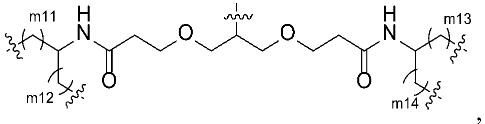 L₂ are each independently -O-(CH₂)ₚ₁-C(=O)-NH-(CH₂CH₂O)ₚ₂-(CH₂)ₚ₃, and m11, m12, m13, m14, p1, p2, and p3 are as defined in formula III.

In some embodiments, L₂ are identical.

In some embodiments, L₁ is C₁₋₁₀ alkyl optionally interrupted by one or more groups selected from the group consisting of -O-, -N-, and -C(=O)-.

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10).

In some embodiments, L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10 (e.g., 1, 2, 3, 4, 5, 6, 7, 8, 9, or 10), wherein the right side of -(CH₂)_{q1}-, -(CH₂)_{q2}-NH-C(=O)-(CH₂)_{q3}-, or -(CH₂)_{q4}-O-(CH₂)_{q5}- is linked to X.

In some embodiments, L₁ is -(CH₂)₆-, -(CH₂)₆-NH-C(=O)-(CH₂)₃-, or -(CH₂)₆-O-CH₂-. In some embodiments, L₁ is -(CH₂)₆-, -(CH₂)₆-NH-C(=O)-(CH₂)₃-, or -(CH₂)₆-O-CH₂-, wherein the right side of -(CH₂)₆-, -(CH₂)₆-NH-C(=O)-(CH₂)₃-, or -(CH₂)₆-O-CH₂- is linked to X.

In some embodiments, Q is 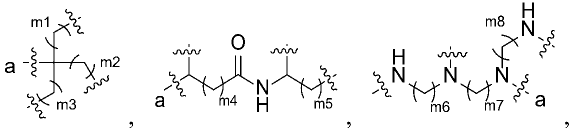 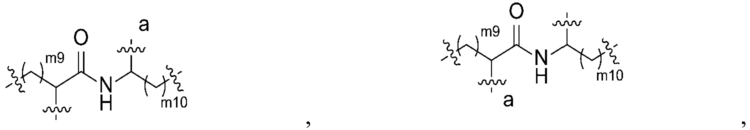 or 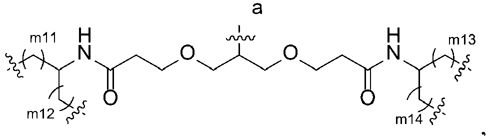 wherein end a is linked to X, and m1, m2, m3, m4, m5, m6, m7, m8, m9, m10, m11, m12, m13, and m14 are as defined in formula III.

In some embodiments, Q is

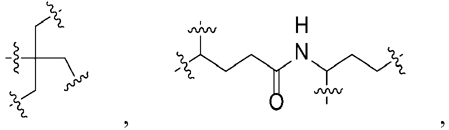

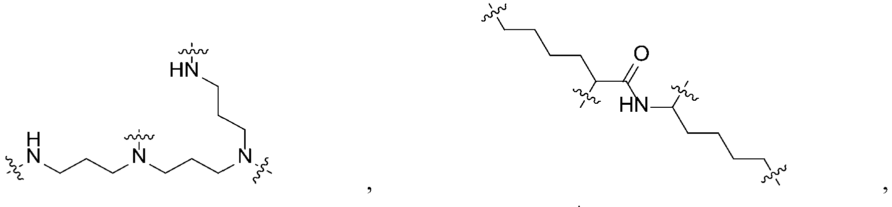

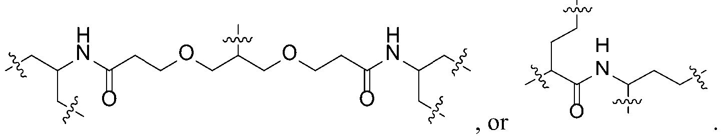

In some embodiments, Q is 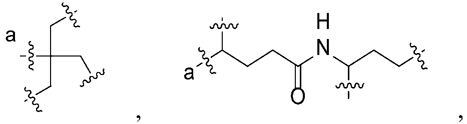 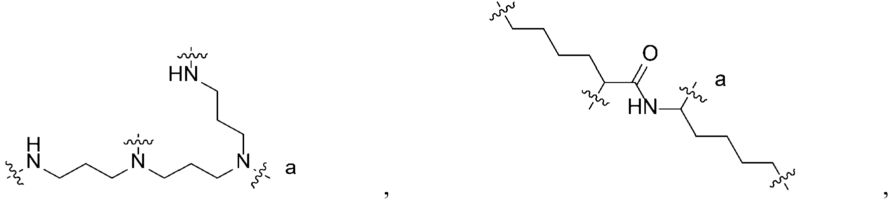 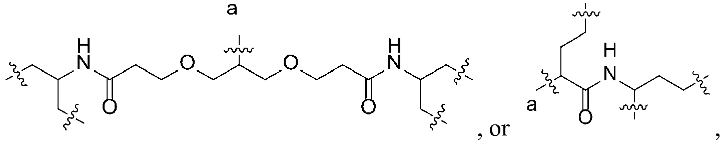 wherein end a is linked to X.

In some embodiments, L₂ are each independently

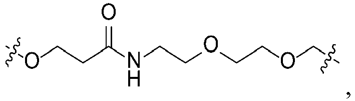

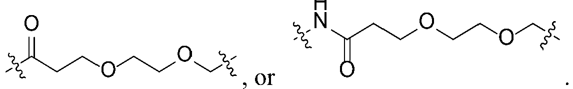

In some embodiments, L₂ are each independently 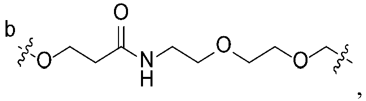 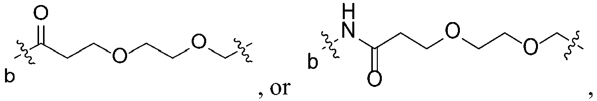 wherein end b is linked to Q.

In some embodiments, the targeting group is an integrin-targeting group.

In some embodiments, the integrin-targeting group is an αvβ6 integrin-targeting group.

In some embodiments, the targeting group TL is formula (III-a): 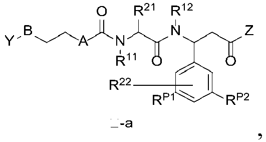 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 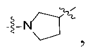 , and absent;
A is selected from the group consisting of -CH₂-, -NH-, -O-, and absent;
Z is selected from the group consisting of OR¹³, N(R¹³)₂, and SR¹³;
R²¹ is selected from the group consisting of H, C₁₋₆ alkyl, OH, COOH, CON(R⁵)₂, OR⁶, and formula (L) 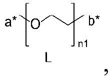 wherein a* represents a point of attachment to phenyl, b* represents a point of attachment to triazolyl in formula III, n1 is selected from the group consisting of integers from 2 to 20 (e.g., 2, 3, 4, 5, 6, 7, 8, 9, 10, 11, 12, 13, 14, 15, 16, 17, 18, 19, and 20), and the C₁₋₆ alkyl is optionally substituted with one or more halogens, hydroxy groups, or cyano groups;
each R⁵ and R⁶ is independently selected from the group consisting of H and C₁₋₆ alkyl, wherein the C₁₋₆ alkyl is optionally substituted with one or more halogens, hydroxy groups, or cyano groups;
R²², R^{P1}, and R^{P2} are each independently selected from the group consisting of H, halogen, 3- to 20-membered cycloalkylene, 6- to 14-membered arylene, 3- to 12-membered heterocycloalkylene, 5- to 14-membered heteroarylene, and formula (L) 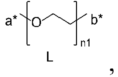
wherein a* represents a point of attachment to phenyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20); the cycloalkylene, arylene, heterocycloalkylene, and heteroarylene are optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
R¹¹ and R¹² are each independently selected from the group consisting of H and C₁₋₆ alkyl, and the C₁₋₆ alkyl is optionally substituted with one or more halogens, hydroxy groups, or cyano groups;
each R¹³ is independently selected from the group consisting of H, C₁₋₆ alkyl, and formula (L) 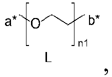 wherein a* represents a point of attachment to phenyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20); the C₁₋₆ alkyl is optionally substituted with one or more halogens, hydroxy groups, or cyano groups;
provided that one and only one of R²¹, R²², R¹³, R^{P1}, and R^{P2} is formula (L).

In some embodiments, R²² is formula (L), and R^{P1} and R^{P2} are each H.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

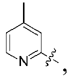

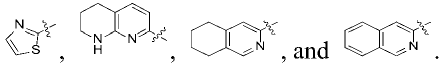

In some specific embodiments, group Y is selected from the group consisting of

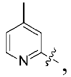

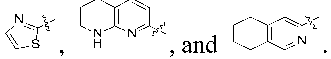

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A in the targeting group TL are 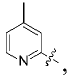 -NH-, and -NH-, respectively; or groups Y, B, and A are 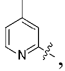 -NH-, and -O-, respectively; or groups Y, B, and A are 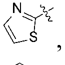 -NH-, and -CH₂-, respectively; or groups Y and A are 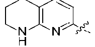 and -CH₂-, respectively, and B is absent; or group Y is 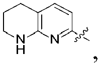 and B and A are absent; or groups Y, B, and A are 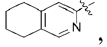 -NH-, and -CH₂-, respectively.

In some embodiments, the targeting group TL is formula (III-b): 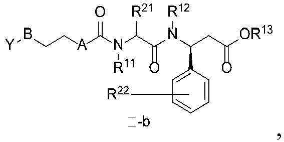 wherein Y is 5-14 membered heteroaryl; the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 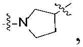 and absent;
A is selected from the group consisting of -CH₂-, -NH-, -O-, and absent;
R²¹ is selected from the group consisting of H, C₁₋₆ alkyl, OH, COOH, CH₂CH₂CH₂NH₂, CONHR⁵, OR⁶, and formula (L) 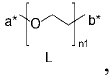 wherein a* represents a point of attachment to phenyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20);
R⁵ and R⁶ are each independently selected from the group consisting of H and C₁₋₆ alkyl, and the C₁₋₆ alkyl is optionally substituted with one or more halogens, hydroxy groups, or cyano groups;
R²² is selected from the group consisting of 3- to 20-membered cycloalkylene, 6- to 14-membered arylene, 3- to 12-membered heterocycloalkylene, 5- to 14-membered heteroarylene, and formula (L) 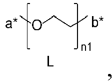 wherein a* represents a point of attachment to phenyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20); the cycloalkylene, arylene, heterocycloalkylene, and heteroarylene are optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5-to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano; provided that one and only one of R²¹ and R²² is formula (L);
R¹¹, R¹², and R¹³ are each independently selected from the group consisting of H and C₁₋₆ alkyl, and the C₁₋₆ alkyl is optionally substituted with one or more halogens, hydroxy groups, or cyano groups.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some embodiments, group Y is selected from the group consisting of

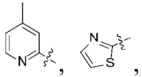

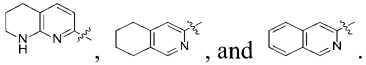

In some specific embodiments, group Y is selected from the group consisting of

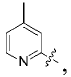

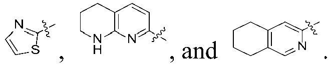

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A in the targeting group TL are 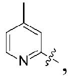 -NH-, and -NH-, respectively; or groups Y, B, and A are 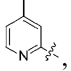 -NH-, and -O-, respectively; or groups Y, B, and A are 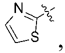 -NH-, and -CH₂-, respectively; or groups Y and A are 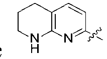 and -CH₂-, respectively, and B is absent; or group Y is 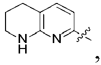 and B and A are absent; or groups Y, B, and A are 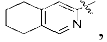 -NH-, and -CH₂-, respectively.

In some embodiments, the targeting group TL is formula (III-c): 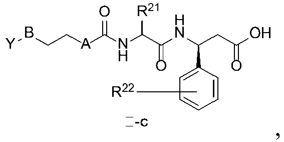 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 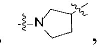 and absent;
A is selected from the group consisting of -CH₂-, -NH-, -O-, and absent;
R²¹ is formula (L) 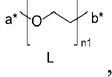 wherein a* represents a point of attachment to phenyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20);
R²² is selected from the group consisting of cycloalkyl, aryl, heteroaryl, and heterocycloalkyl having 2-10 carbon atoms, and the heteroaryl, cycloalkyl, aryl, and heterocycloalkyl are optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5-to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano. In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

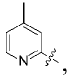

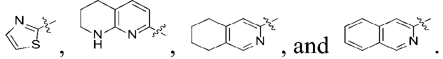

In some specific embodiments, group Y is selected from the group consisting of

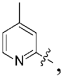

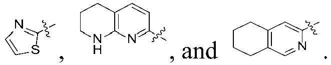

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A in the targeting group TL are 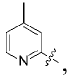 -NH-, and -NH-, respectively; or groups Y, B, and A are 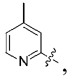 -NH-, and -O-, respectively; or groups Y, B, and A are 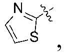 -NH-, and -CH₂-, respectively; or groups Y and A are 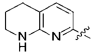 and -CH₂-, respectively, and B is absent; or group Y is 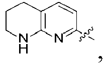 and B and A are absent; or groups Y, B, and A are 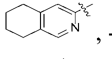 -NH-, and -CH₂-, respectively.

In some embodiments, the targeting group TL is formula (III-d): 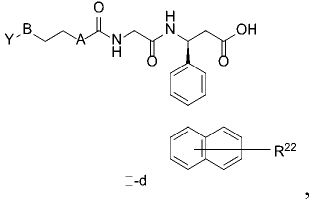 wherein R²² is formula (L) 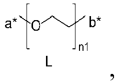 wherein a* represents a point of attachment to naphthyl, b* represents a point of attachment to triazolyl in formula III, and n1 is selected from the group consisting of integers from 2 to 20 (e.g., 2, 3, 4, 5, 6, 7, 8, 9, 10, 11, 12, 13, 14, 15, 16, 17, 18, 19, and 20); Y is 5-14 membered heteroaryl, and the heteroaryl is optionally substituted with one or more groups selected from the group consisting of: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more substituents selected from the group consisting of halogen, hydroxy, oxo, nitro, and cyano;
B is selected from the group consisting of -CH₂-, -NH-, 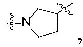 and absent;
A is selected from the group consisting of -CH₂-, -NH-, -O-, and absent.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

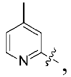

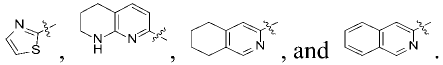

In some specific embodiments, group Y is selected from the group consisting of

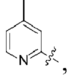

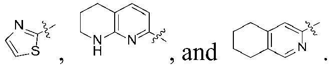

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A in the targeting group TL are 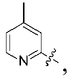 -NH-, and -NH-, respectively; or groups Y, B, and A are 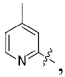 -NH-, and -O-, respectively; or groups Y, B, and A are 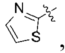 -NH-, and -CH₂-, respectively; or groups Y and A are 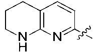 and -CH₂-, respectively, and B is absent; or group Y is 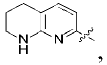 and B and A are absent; or groups Y, B, and A are 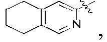 -NH-, and -CH₂-, respectively.

In some embodiments, n1 is selected from the group consisting of integers from 2 to 15.

In some embodiments, n1 is selected from the group consisting of integers from 2 to 10.

In some embodiments, n1 is selected from the group consisting of integers from 2 to 5.

In some specific embodiments, n1 is 2, 3, 4, 5, 6, 7, 8, 9, 10, 11, 12, 13, 14, 15, 16, 17, 18, 19, or 20.

In some embodiments, the targeting group TL is formula (III-e): 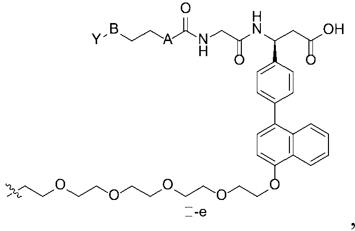 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 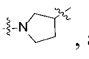 and absent;
A is selected from the group consisting of -CH₂-, -NH-, -O-, and absent.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

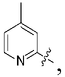

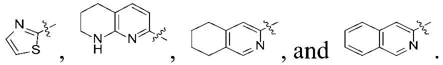

In some specific embodiments, group Y is selected from the group consisting of

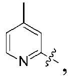

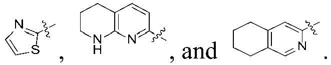

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A in the targeting group TL are 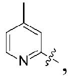 -NH-, and -NH-, respectively; or groups Y, B, and A are 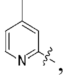 -NH-, and -O-, respectively; or groups Y, B, and A are 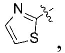 -NH-, and -CH₂-, respectively; or groups Y and A are 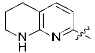 and -CH₂-, respectively, and B is absent; or group Y is 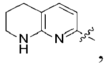 and B and A are absent; or groups Y, B, and A are 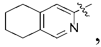 -NH-, and -CH₂-, respectively.

In some embodiments, the targeting group TL is formula (III-e-1): 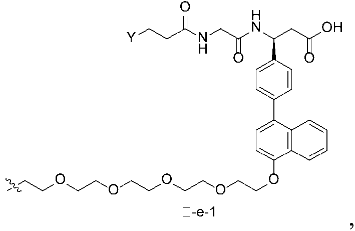 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano. In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

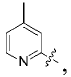

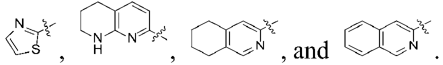

In some specific embodiments, group Y is selected from the group consisting of

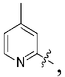

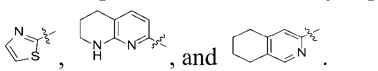

In some embodiments, the targeting group TL is formula (III-e-2): 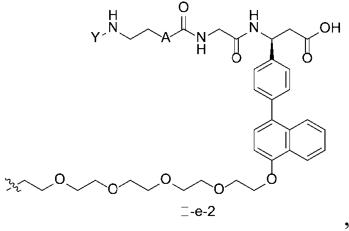 wherein A is selected from the group consisting of - O- and -CH₂-; Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

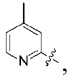

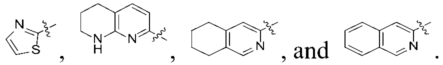

In some specific embodiments, group Y is selected from the group consisting of

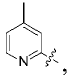

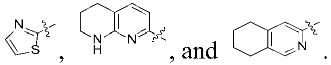

In some embodiments, group A of the targeting group TL is -CH₂-.

In some embodiments, group A of the targeting group TL is -O-.

In some specific embodiments, the targeting group TL is selected from the group consisting of (III-f)-(III-p): 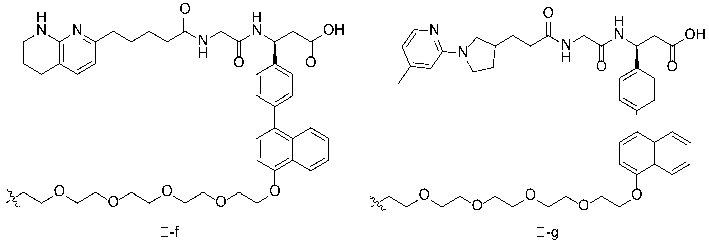 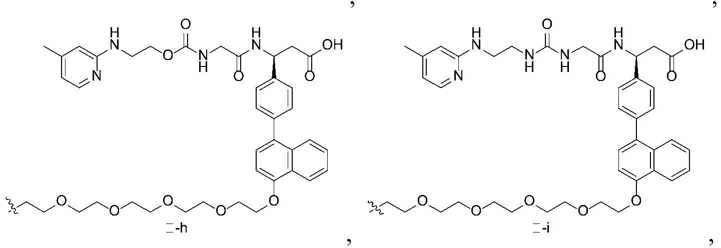 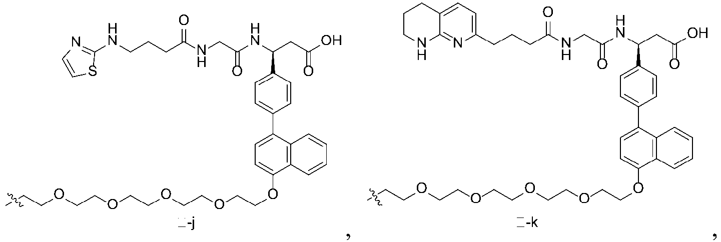 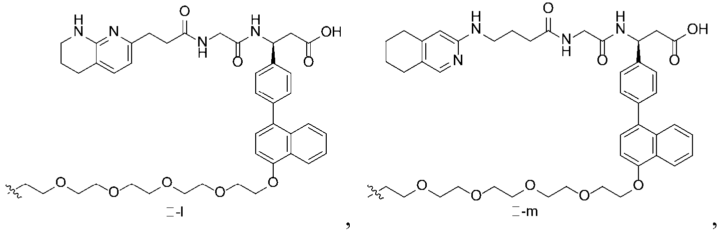 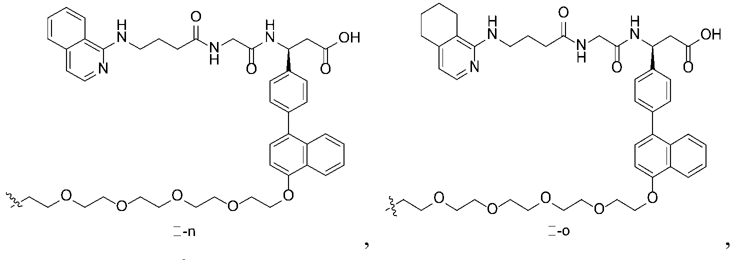 and

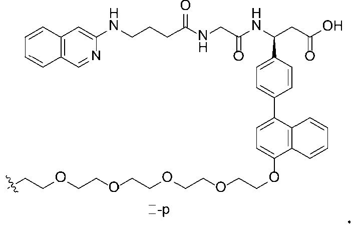

The present disclosure provides a targeting ligand or a pharmaceutically acceptable salt thereof, wherein the targeting ligand is selected from the group consisting of the following structures, and TL is the same as that described above: 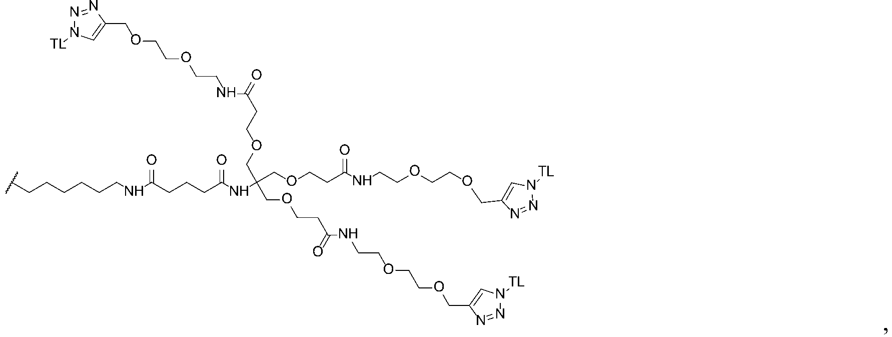 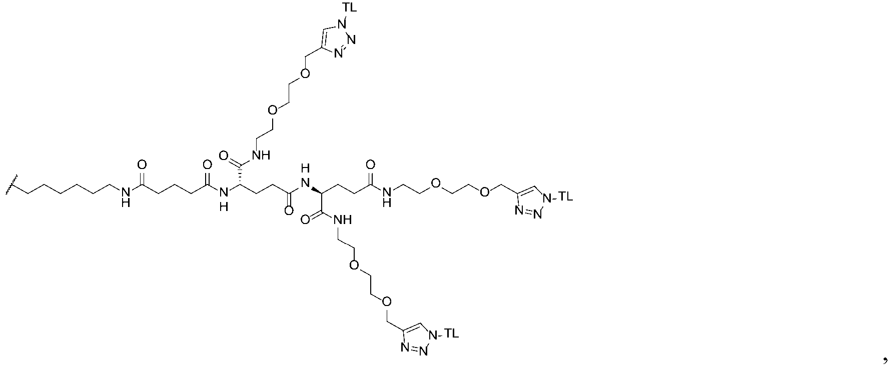 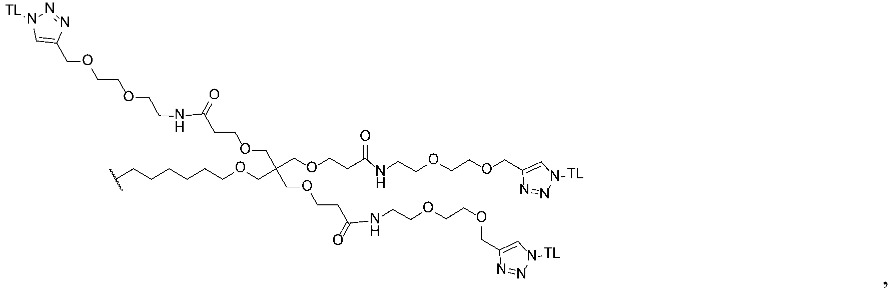 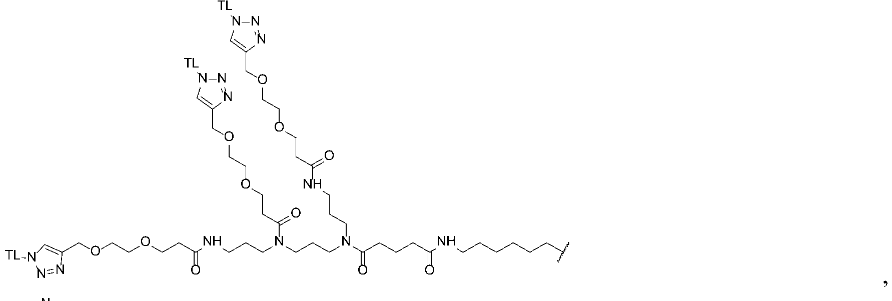 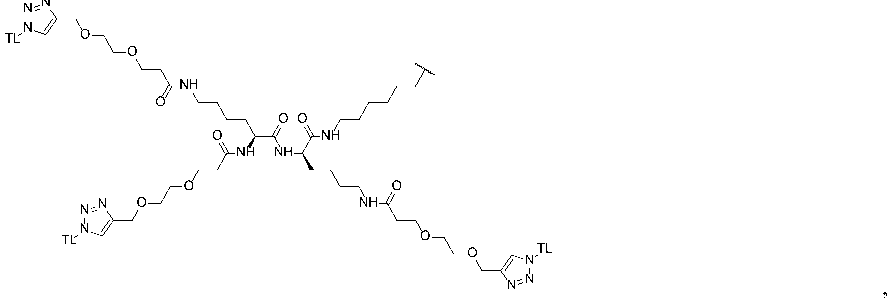 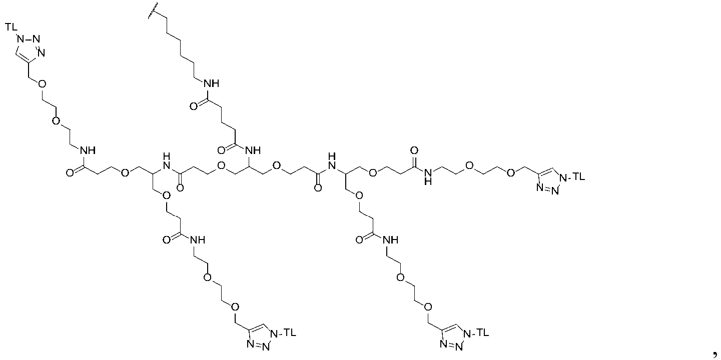 and

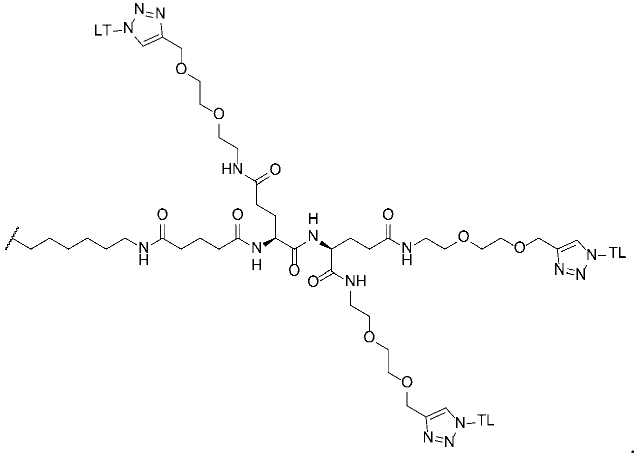

The present disclosure provides a targeting ligand or a pharmaceutically acceptable salt thereof, wherein the targeting ligand is selected from the group consisting of the following structures: 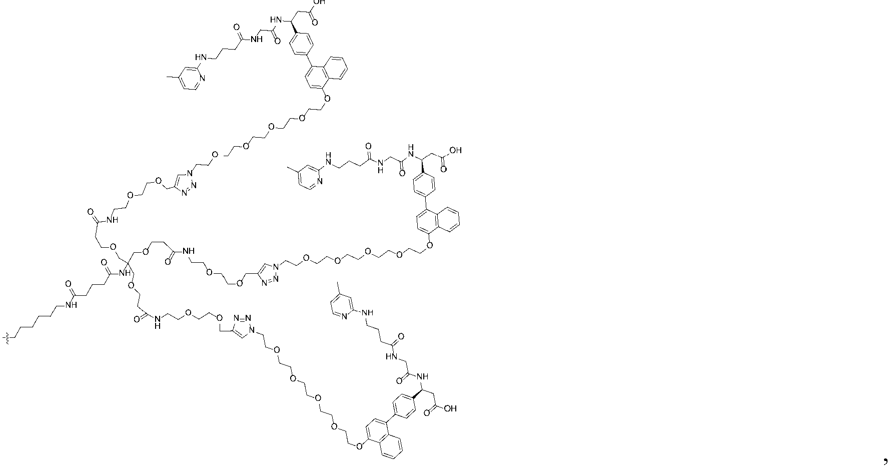 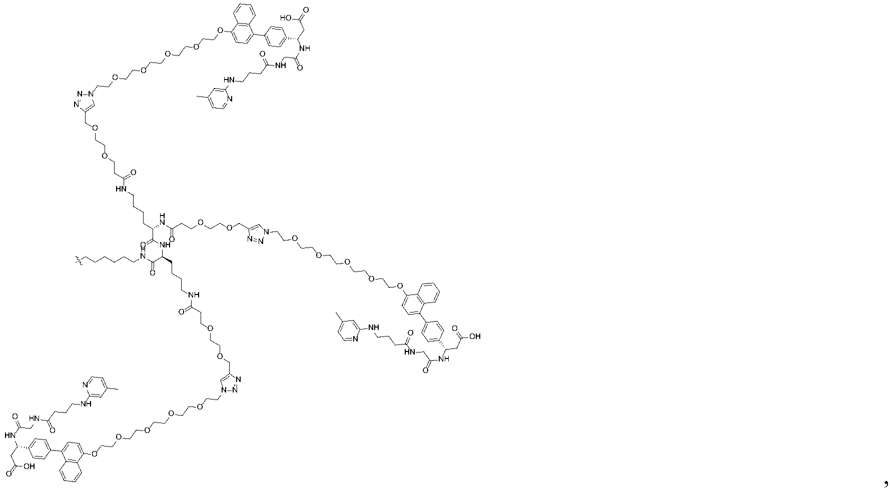 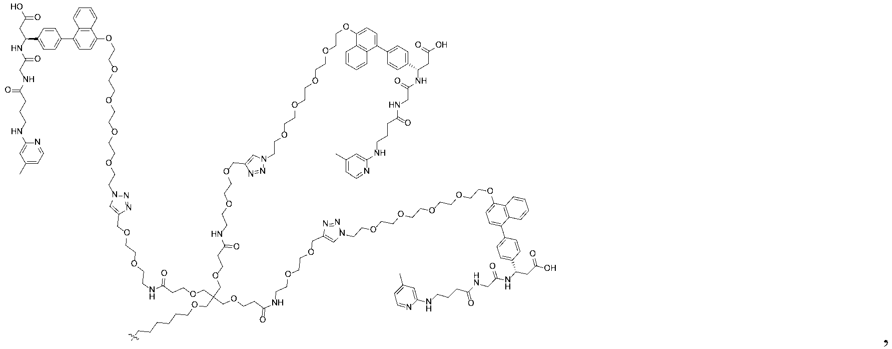 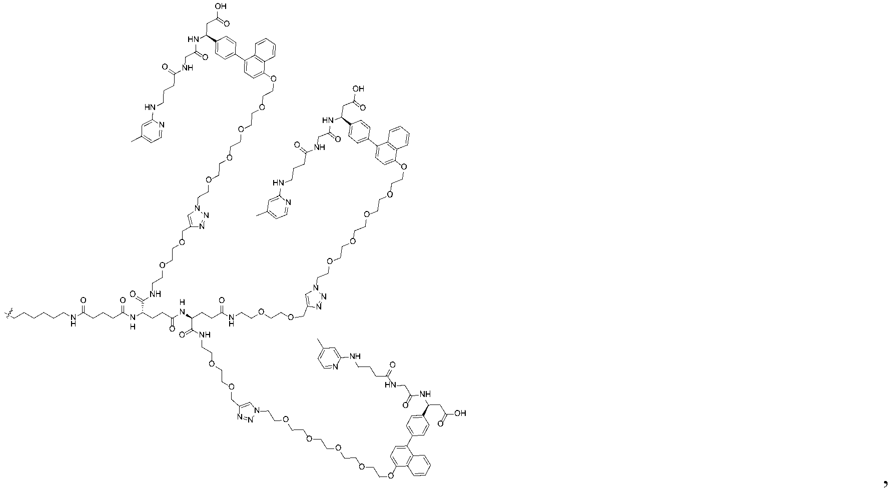 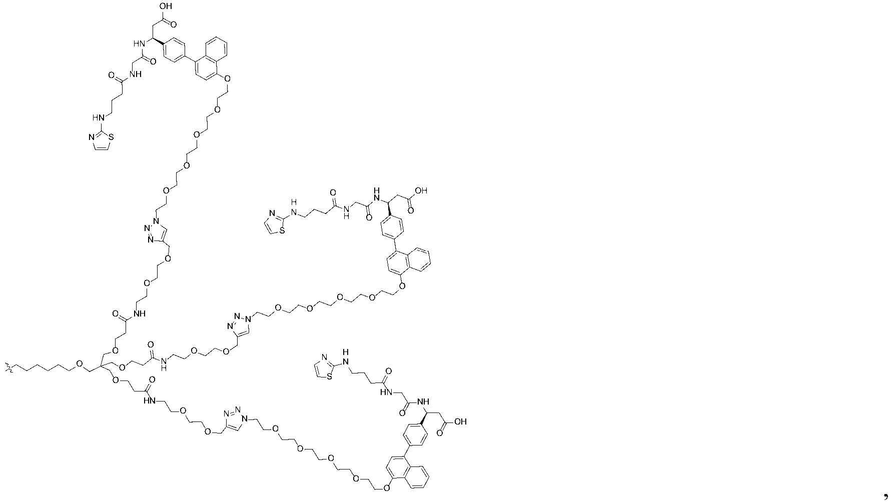 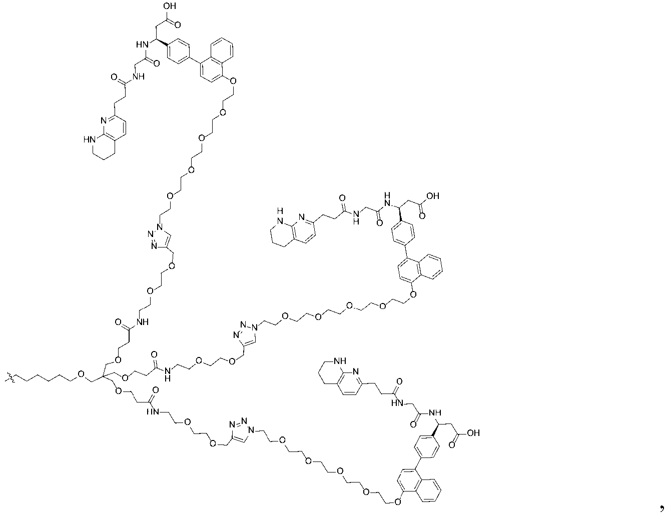 and

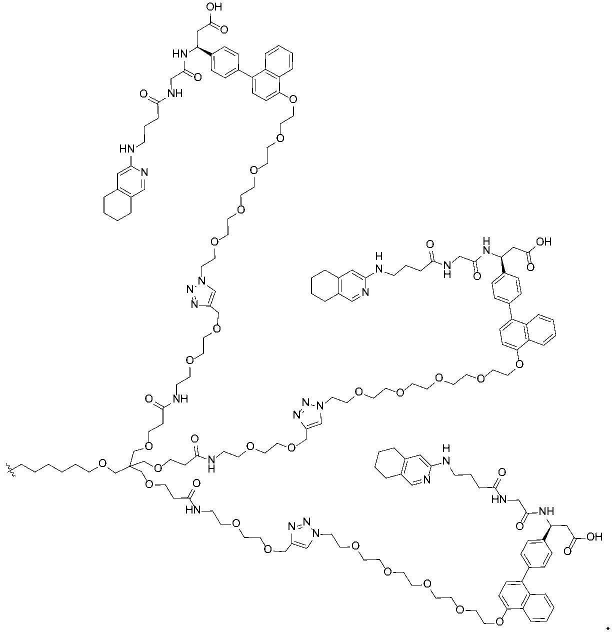

The present disclosure further provides a compound represented by formula (III-a') or a pharmaceutically acceptable salt thereof: 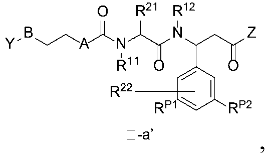 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 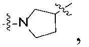 and absent;
A is selected from the group consisting of -CH₂-, -NH-, -O-, and absent, and when Y is 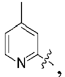 B and A are not simultaneously -NH- or -CH₂-;
Z is selected from the group consisting of OR¹³, N(R¹³)₂, and SR¹³;
R²¹ is selected from the group consisting of H, C₁₋₆ alkyl, OH, COOH, CON(R⁵)₂, OR⁶, and formula (L1) 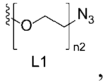 wherein n2 is selected from the group consisting of integers from 2 to 20 (e.g., 2, 3, 4, 5, 6, 7, 8, 9, 10, 11, 12, 13, 14, 15, 16, 17, 18, 19, and 20), and the wavy line represents a point of attachment to formula (III-a'); the C₁₋₆ alkyl is optionally substituted with one or more halogens, hydroxy groups, or cyano groups;
each R⁵ and R⁶ is independently selected from the group consisting of H and C₁₋₆ alkyl, and the C₁₋₆ alkyl is optionally substituted with one or more halogens, hydroxy groups, or cyano groups;
R²², R^{P1}, and R^{P2} are each independently selected from the group consisting of H, 3- to 20-membered cycloalkylene, 6- to 14-membered arylene, 3- to 12-membered heterocycloalkylene, 5- to 14-membered heteroarylene, and formula (L1) 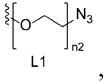
wherein n2 is selected from the group consisting of integers from 2 to 20 (e.g., 2, 3, 4, 5, 6, 7, 8, 9, 10, 11, 12, 13, 14, 15, 16, 17, 18, 19, and 20), and the wavy line represents a point of attachment to phenyl; the cycloalkylene, arylene, heterocycloalkylene, and heteroarylene are optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5-to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano; R¹¹ and R¹² are each independently selected from the group consisting of H and C₁₋₆ alkyl, and the C₁₋₆ alkyl is optionally substituted with one or more halogens, hydroxy groups, or cyano groups;
each R¹³ is independently selected from the group consisting of H, C₁₋₆ alkyl, and formula (L1) 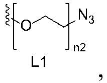 wherein n2 is selected from the group consisting of integers from 2 to 20 (e.g., 2, 3, 4, 5, 6, 7, 8, 9, 10, 11, 12, 13, 14, 15, 16, 17, 18, 19, and 20), and the wavy line represents a point of attachment to phenyl; the C₁₋₆ alkyl is optionally substituted with one or more halogens, hydroxy groups, or cyano groups;
provided that one and only one of R²¹, R²², R¹³, R^{P1}, and R^{P2} is formula (L1).

In some embodiments, R²² is formula (L1), and R^{P1} and R^{P2} are each H.

In some embodiments, n2 is selected from the group consisting of integers from 2 to 15.

In some embodiments, n2 is selected from the group consisting of integers from 2 to 10.

In some embodiments, n2 is selected from the group consisting of integers from 2 to 5.

In some specific embodiments, n2 is 2, 3, 4, 5, 6, 7, 8, 9, 10, 11, 12, 13, 14, 15, 16, 17, 18, 19, or 20.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

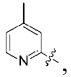

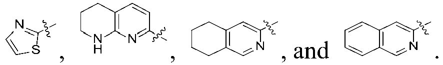

In some specific embodiments, group Y is selected from the group consisting of

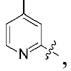

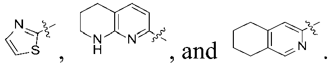

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A are 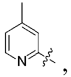 -NH-, and -NH-, respectively; or groups Y, B, and A are 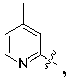 -NH-, and -O-, respectively; or groups Y, B, and A are 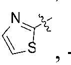 -NH-, and -CH₂-, respectively; or groups Y and A are 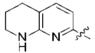 and -CH₂-, respectively, and B is absent; or group Y is 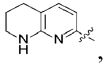 and B and A are absent; or groups Y, B, and A are 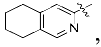 -NH-, and -CH₂-, respectively.

The present disclosure further provides a compound represented by formula (III-d') or a pharmaceutically acceptable salt thereof: 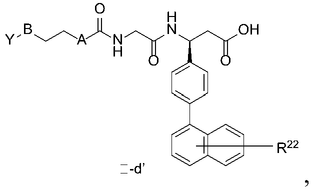 wherein R²² is formula (L1) 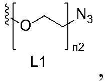 wherein n2 is selected from the group consisting of integers from 2 to 20 (e.g., 2, 3, 4, 5, 6, 7, 8, 9, 10, 11, 12, 13, 14, 15, 16, 17, 18, 19, and 20), and the wavy line represents a point of attachment to naphthyl;
wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 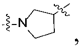 and absent;
A is selected from the group consisting of -CH₂-, -NH-, -O-, and absent, and when Y is 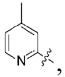 B and A are not simultaneously -NH- or -CH₂-.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, n2 is selected from the group consisting of integers from 2 to 15. In some embodiments, n2 is selected from the group consisting of integers from 2 to 10. In some embodiments, n2 is selected from the group consisting of integers from 2 to 5. In some specific embodiments, n2 is 2, 3, 4, 5, 6, 7, 8, 9, 10, 11, 12, 13, 14, 15, 16, 17, 18, 19, or 20.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

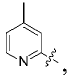

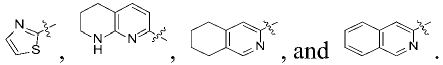

In some specific embodiments, group Y is selected from the group consisting of

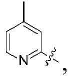

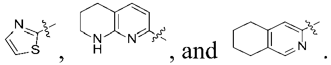

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A are 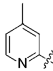 , -NH-, and -NH-, respectively; or groups Y, B, and A are 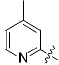 , -NH-, and -O-, respectively; or groups Y, B, and A are 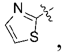 -NH-, and -CH₂-, respectively; or groups Y and A are 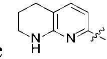 and -CH₂-, respectively, and B is absent; or group Y is 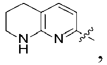 and B and A are absent; or groups Y, B, and A are 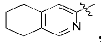 -NH-, and -CH₂-, respectively.

The present disclosure further provides a compound represented by formula (III-e') or a pharmaceutically acceptable salt thereof: 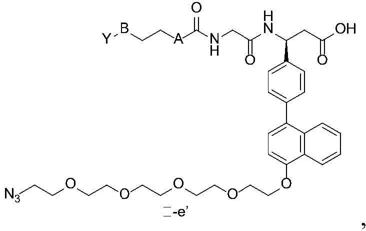 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 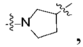 and absent;
A is selected from the group consisting of -CH₂-, -NH-, -O-, and absent, and when Y is 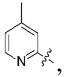 B and A are not simultaneously -NH- or -CH₂-.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

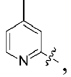

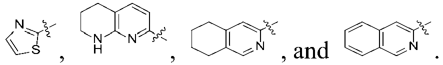

In some specific embodiments, group Y is selected from the group consisting of

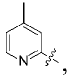

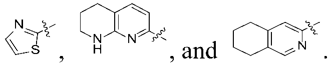

In some embodiments, group B of the targeting group TL is -NH- or absent.

In some embodiments, group B of the targeting group TL is absent.

In some embodiments, group A of the targeting group TL is selected from the group consisting of -CH₂-, -NH-, -O-, and absent.

In some embodiments, group A of the targeting group TL is -CH₂-.

In some embodiments, group A of the targeting group TL is -NH-.

In some embodiments, group A of the targeting group TL is -O-.

In some embodiments, group A of the targeting group TL is absent.

In some specific embodiments, groups Y, B, and A are 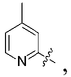 -NH-, and -NH-, respectively; or groups Y, B, and A are 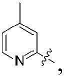 -NH-, and -O-, respectively; or groups Y, B, and A are 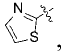 -NH-, and -CH₂-, respectively; or groups Y and A are 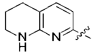 and -CH₂-, respectively, and B is absent; or group Y is 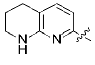 , and B and A are absent; or groups Y, B, and A are 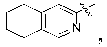 -NH-, and -CH₂-, respectively.

The present disclosure further provides a compound represented by formula (III-e'-1) or a pharmaceutically acceptable salt thereof: 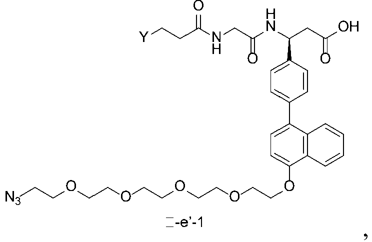 wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

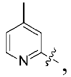

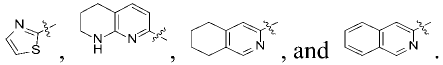

In some specific embodiments, group Y is selected from the group consisting of

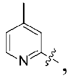

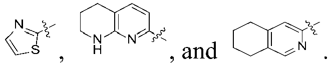

The present disclosure further provides a compound represented by formula (III-e'-2) or a pharmaceutically acceptable salt thereof: 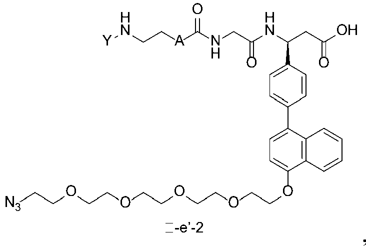 , wherein Y is 5-14 membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano; A is selected from the group consisting of -O- and -CH₂-.

In some embodiments, group Y is 5- to 12-membered heteroaryl, and the heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano.

In some embodiments, group Y is 5- or 6-membered heteroaryl, and the heteroaryl is optionally substituted with one or more groups as follows: halogen, hydroxy, oxo, nitro, cyano, C₁₋₆ alkyl, and C₁₋₆ alkoxy, wherein the C₁₋₆ alkyl and C₁₋₆ alkoxy are optionally substituted with one or more substituents selected from the group consisting of halogen, hydroxy, oxo, nitro, and cyano.

In some embodiments, group Y is pyridyl substituted with one or more methyl groups.

In some specific embodiments, group Y is selected from the group consisting of

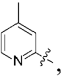

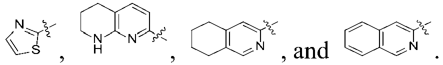

In some specific embodiments, group Y is selected from the group consisting of

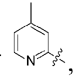

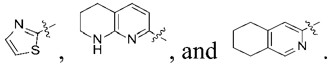

In some embodiments, group A is -O-.

In some embodiments, group A is -CH₂-.

The present disclosure further provides compounds represented by formula (III-f')-formula (III-p') or pharmaceutically acceptable salts thereof: 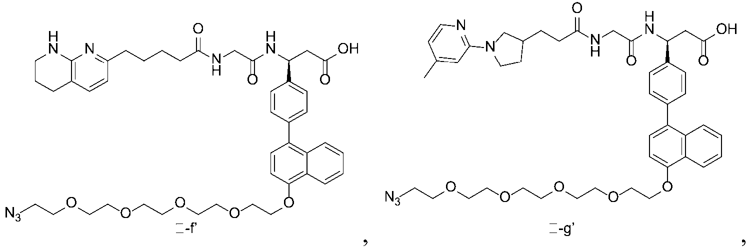 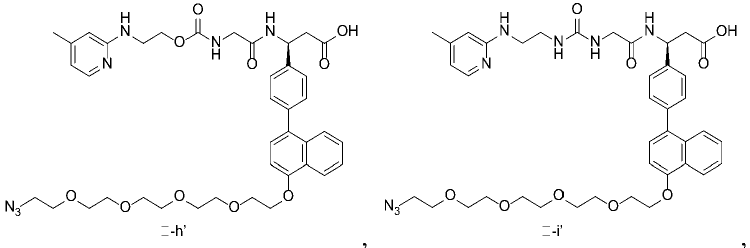 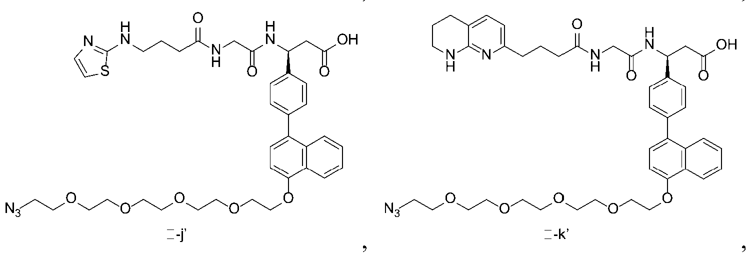 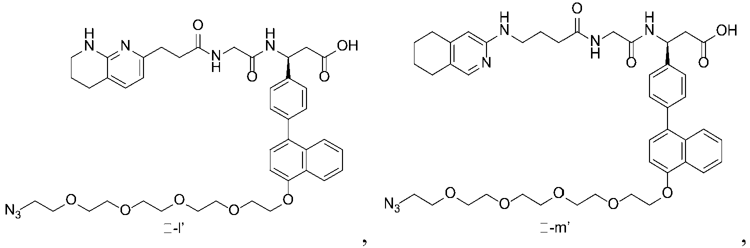 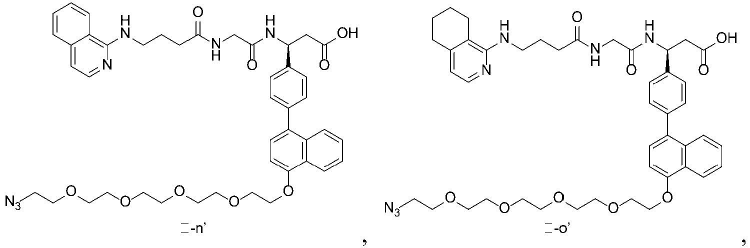 and

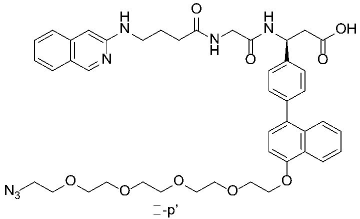

The present disclosure provides a nucleic acid-targeting ligand conjugate or a pharmaceutically acceptable salt thereof, wherein the nucleic acid-targeting ligand conjugate comprises a nucleic acid and one or more targeting ligands conjugated to the nucleic acid; the targeting ligands are as described above, and each of the targeting ligands is identical or different.

In some embodiments, TL in the targeting ligand is selected from the group consisting of formulas (III-a)-(III-p).

In some embodiments, the nucleic acid and the targeting ligands are linked by phosphoester groups, phosphorothioate groups, or phosphonic acid groups.

In some embodiments, the nucleic acid is linked to the alkynyl-containing moieties by phosphoester groups.

In some embodiments, the nucleic acid is linked to the alkynyl-containing moieties by phosphorothioate groups.

In some embodiments, the phosphoester groups are phosphodiester groups.

In some embodiments, the phosphorothioate groups are phosphorothioate diester groups. In some embodiments, the nucleic acid includes, but is not limited to, single-stranded oligonucleotides, single-stranded antisense oligonucleotides, short interfering RNAs (siRNAs, the siRNAs comprising partially complementary sense and antisense strands), double-stranded RNAs (dsRNAs), microRNAs (miRNAs), short hairpin RNAs (shRNAs), and Dicer substrates.

In some embodiments, a 3' end of the nucleic acid is conjugated to a targeting ligand.

In some embodiments, a 5' end of the nucleic acid is conjugated to a targeting ligand.

In some embodiments, a 3' end and a 5' end of the nucleic acid are both conjugated to targeting ligands.

The aforementioned 3' end and/or 5' end of the nucleic acid conjugated to the targeting ligands refer(s) to the 3' end and/or 5' end of a nucleic acid strand conjugated to the targeting ligands.

In some specific embodiments, the nucleic acid is an siRNA in which the 5' end of the sense strand is conjugated to a targeting ligand.

In some embodiments, the nucleic acid is a single-stranded nucleic acid.

In some embodiments, the single-stranded nucleic acid is the sense strand of an siRNA. In some embodiments, the single-stranded nucleic acid is the antisense strand of an siRNA.

In some embodiments, the nucleic acid is a double-stranded nucleic acid.

In some embodiments, the double-stranded nucleic acid comprises at least one duplex region within which a first strand nucleic acid is at least partially complementary to a second strand nucleic acid, wherein:
(1) neither end of the first strand nucleic acid is conjugated to a targeting ligand; and the 5' end of the second strand nucleic acid is conjugated to a targeting ligand, and the 3' end is not conjugated to a targeting ligand; or the 3' end of the second strand nucleic acid is conjugated to a targeting ligand, and the 5' end is not conjugated to a targeting ligand; or both the 3' end and the 5' end of the second strand nucleic acid are conjugated to targeting ligands;
(2) neither end of the second strand nucleic acid is conjugated to a targeting ligand; and the 5' end of the first strand nucleic acid is conjugated to a targeting ligand, and the 3' end is not conjugated to a targeting ligand; or the 3' end of the first strand nucleic acid is conjugated to a targeting ligand, and the 5' end is not conjugated to a targeting ligand; or both the 3' end and the 5' end of the first strand nucleic acid are conjugated to targeting ligands;
(3) both the 5' ends of the first strand nucleic acid and the second strand nucleic acid are conjugated to targeting ligands, and neither 3' end is conjugated to a targeting ligand; or both the 3' ends of the first strand nucleic acid and the second strand nucleic acid are conjugated to targeting ligands, and neither 5' end is conjugated to a targeting ligand; or both the 3' ends and the 5' ends of the first strand nucleic acid and the second strand nucleic acid are conjugated to targeting ligands; or both the 3' end of the first strand nucleic acid and the 5' end of the second strand nucleic acid are conjugated to targeting ligands, and neither the 5' end of the first strand nucleic acid nor the 3' end of the second strand nucleic acid is conjugated to a targeting ligand; or both the 5' end of the first strand nucleic acid and the 3' end of the second strand nucleic acid are conjugated to targeting ligands, and neither the 3' end of the first strand nucleic acid nor the 5' end of the second strand nucleic acid is conjugated to a targeting ligand;
(4) both the 3' end and the 5' end of the first strand nucleic acid are conjugated to targeting ligands, and either the 5' end or the 3' end of the second strand nucleic acid is conjugated to a targeting ligand; or both the 3' end and the 5' end of the second strand nucleic acid are conjugated to targeting ligands, and either the 5' end or the 3' end of the first strand nucleic acid is conjugated to a targeting ligand.

In some embodiments, the nucleic acid is an siRNA.

In some embodiments, the nucleic acid is an siRNA targeting RAGE.

In some embodiments, the nucleic acid is an siRNA targeting MUC5B.

In some embodiments, the nucleic acid is an siRNA targeting APP.

In some embodiments, the nucleic acid comprises one or more modified nucleotides.

In some embodiments, the nucleic acid-targeting ligand conjugate is represented by formula V, 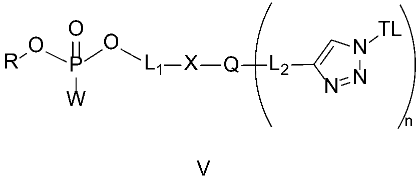 wherein R is the nucleic acid; W is OH, SH, O⁻, or S⁻; L₁, L₂, X, Q, and TL are as defined in formula III.

In some embodiments, W is OH or O⁻.

In some embodiments, W is SH or S⁻.

The present disclosure provides a nucleic acid-targeting ligand conjugate or a pharmaceutically acceptable salt thereof, wherein the nucleic acid-targeting ligand conjugate is selected from the group consisting of the following structures: 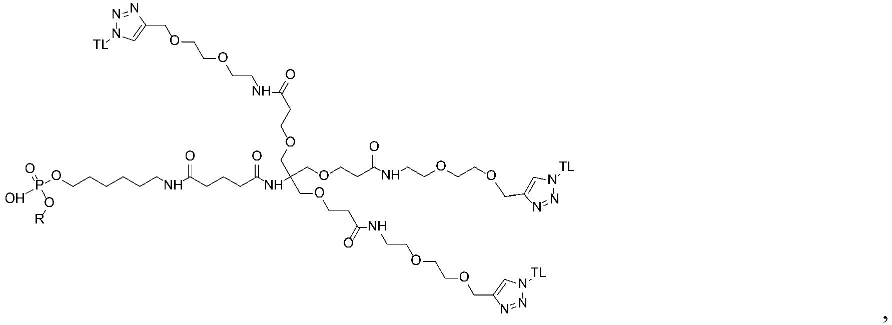 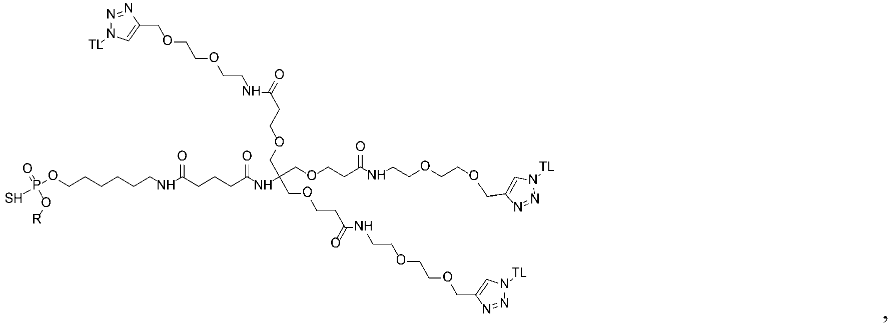 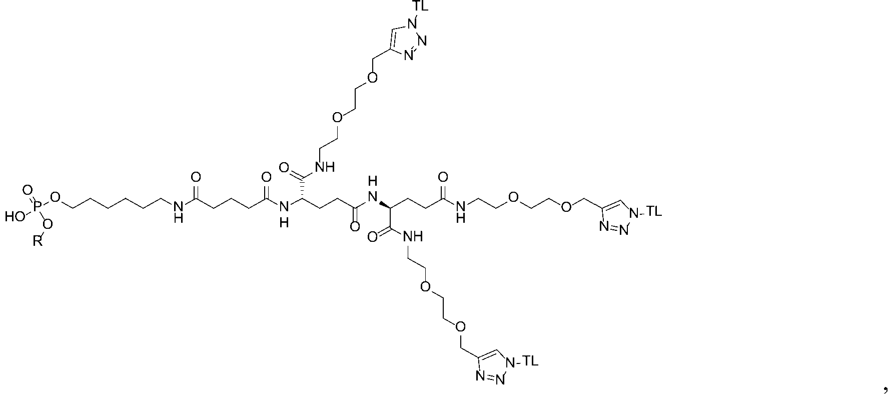 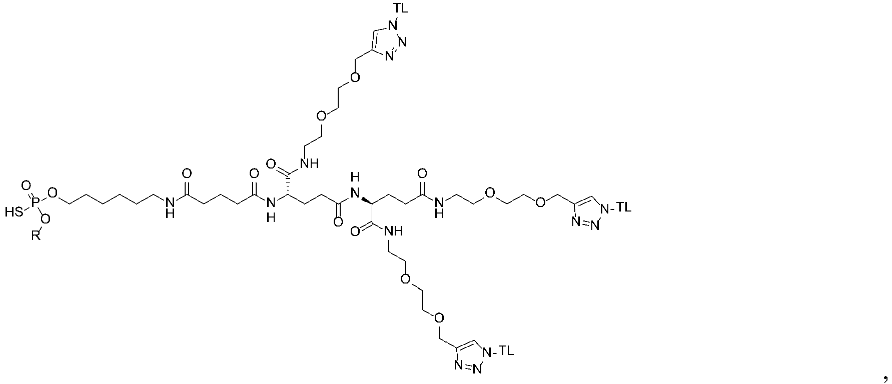 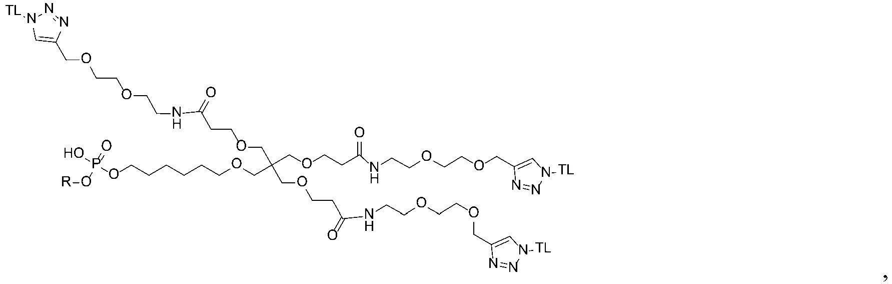 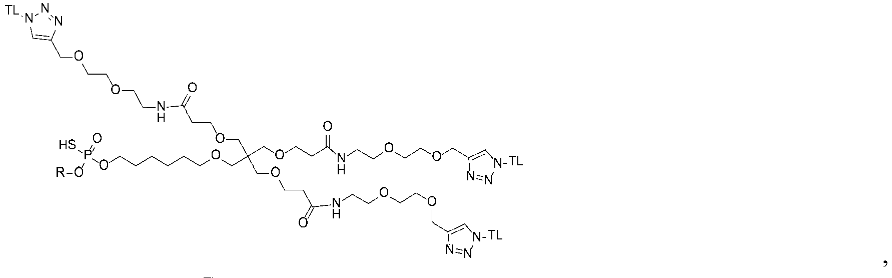 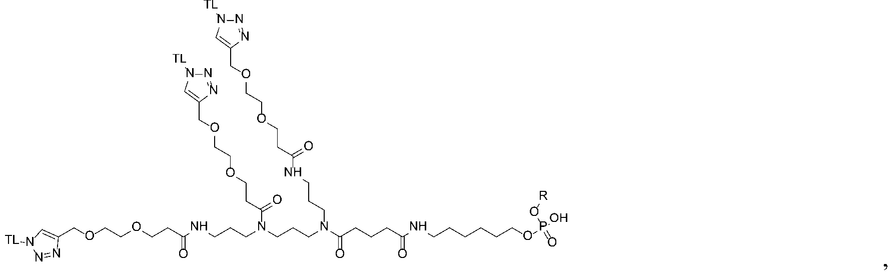 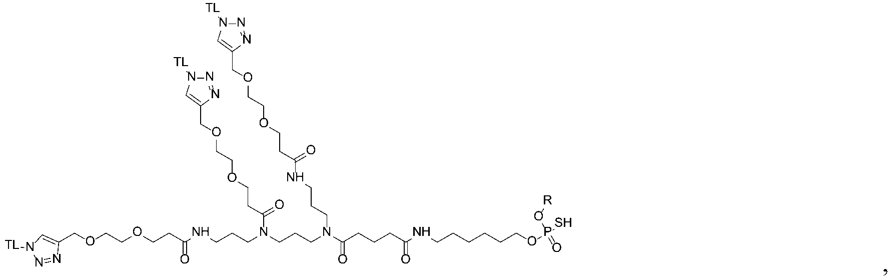 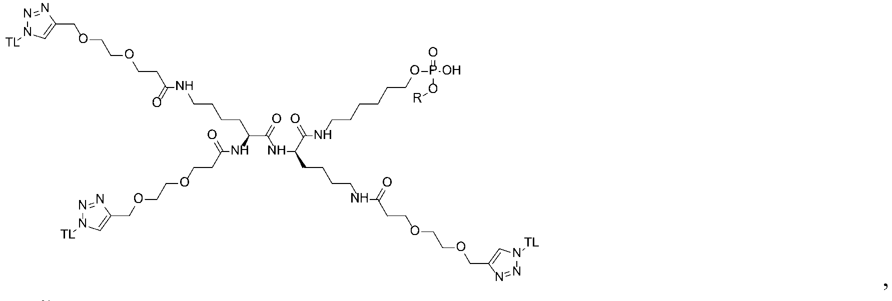 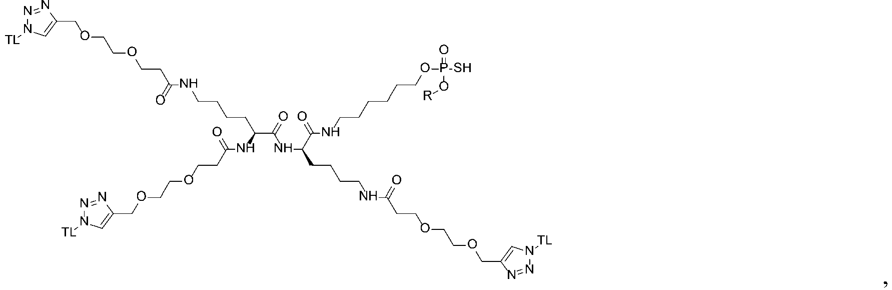 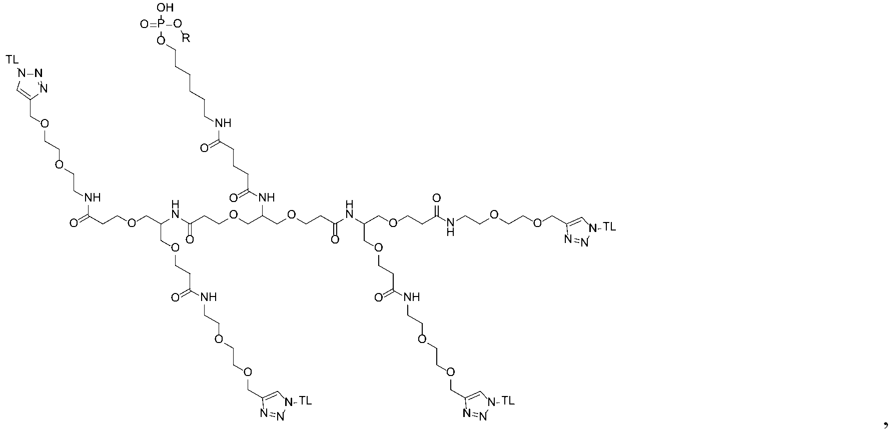 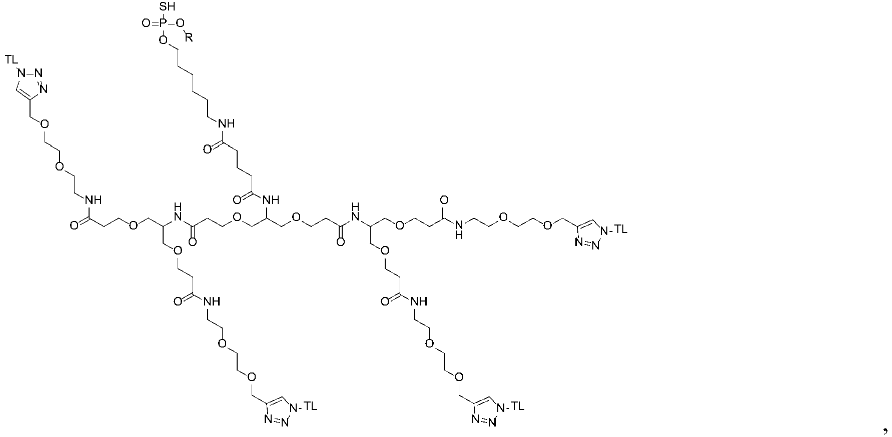 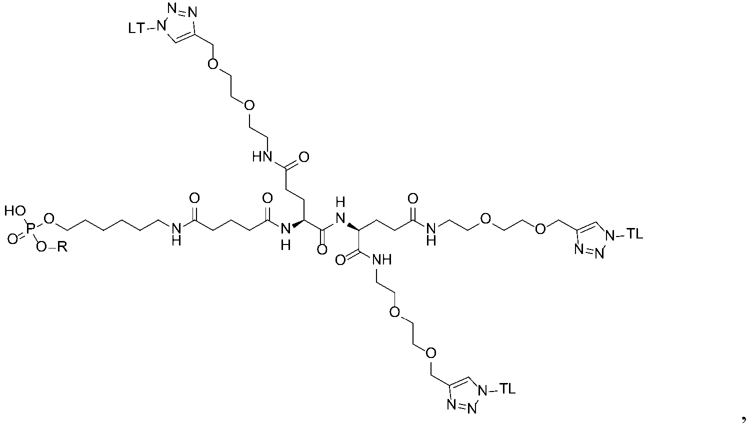 and 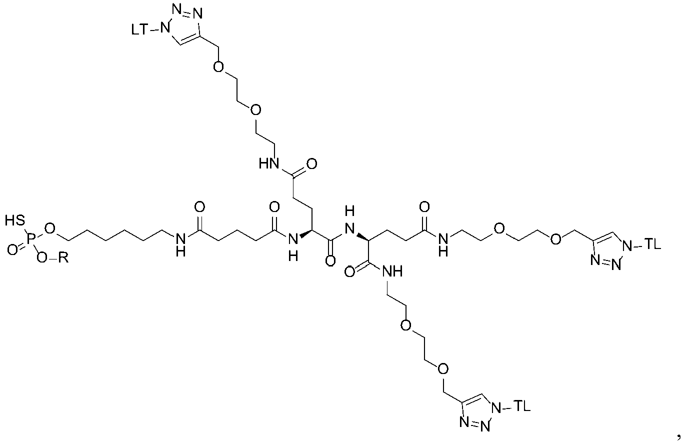 wherein R is a nucleic acid that is the same as that described above, and TL is the same as that described above.

In some embodiments, TL is as described in formula (III-a)-formula (III-p).

In some embodiments, a 5' end of the nucleic acid is conjugated to a targeting ligand. The present disclosure provides a pharmaceutical composition comprising the nucleic acid-targeting ligand conjugate or the pharmaceutically acceptable salt thereof described above, and one or more pharmaceutically acceptable excipients.

In some embodiments, a unit dose of the pharmaceutical composition may be 0.001 mg-1000 mg.

In some embodiments, the nucleic acid-targeting ligand conjugate may be present in an amount of 0.01-99.99%, or 0.1-99.9%, or 0.5%-99.5%, further 1%-99%, and still further 2%-98%, based on the total weight of the pharmaceutical composition.

In some embodiments, the pharmaceutically acceptable excipients may be present in an amount of 0.01-99.99%, or 0.1-99.9%, or 0.5%-99.5%, further 1%-99%, and still further 2%-98%, based on the total weight of the pharmaceutical composition.

In some embodiments, the nucleic acid-targeting ligand conjugate is present in a therapeutically effective amount.

In some embodiments, the pharmaceutically acceptable excipients may be excipients commonly used in the art, such as a carrier, a vehicle, a diluent, and/or a delivery polymer. The present disclosure provides use of the nucleic acid-targeting ligand conjugate or the pharmaceutically acceptable salt thereof described above or the pharmaceutical composition described above in the manufacture of a medicament for preventing and/or treating a disease, wherein the disease is a respiratory disease.

The present disclosure provides use of the nucleic acid-targeting ligand conjugate or the pharmaceutically acceptable salt thereof described above or the pharmaceutical composition described above for inhibiting mRNA or target gene expression in a patient. The present disclosure provides use of the nucleic acid-targeting ligand conjugate or the pharmaceutically acceptable salt thereof described above or the pharmaceutical composition described above in the manufacture of a medicament for preventing and/or treating a disease mediated by RAGE gene expression. In some embodiments, the disease is a respiratory disease.

The present disclosure provides a nucleic acid-targeting ligand conjugate or a pharmaceutically acceptable salt thereof or a pharmaceutical composition for use in the prevention and/or treatment of a disease, wherein the disease is a respiratory disease, and the nucleic acid-targeting ligand conjugate or the pharmaceutically acceptable salt thereof or the pharmaceutical composition is the same as that described above.

The present disclosure provides a nucleic acid-targeting ligand conjugate or a pharmaceutically acceptable salt thereof or a pharmaceutical composition for use in the inhibition of mRNA or target gene expression in a patient, wherein the nucleic acid-targeting ligand conjugate or the pharmaceutically acceptable salt thereof or the pharmaceutical composition is the same as that described above.

The present disclosure provides a nucleic acid-targeting ligand conjugate or a pharmaceutically acceptable salt thereof or a pharmaceutical composition for use in the prevention and/or treatment of a disease mediated by RAGE gene expression, wherein the nucleic acid-targeting ligand conjugate or the pharmaceutically acceptable salt thereof or the pharmaceutical composition is the same as that described above. In some embodiments, the disease is a respiratory disease.

The present disclosure provides a nucleic acid-targeting ligand conjugate or a pharmaceutically acceptable salt thereof or a pharmaceutical composition for use in the delivery of one or more transported molecules to cells, wherein the nucleic acid-targeting ligand conjugate or the pharmaceutically acceptable salt thereof or the pharmaceutical composition is the same as that described above.

The present disclosure provides a method for treating a disease, comprising administering to a patient a therapeutically effective amount of the nucleic acid-targeting ligand conjugate or the pharmaceutically acceptable salt thereof described above or the pharmaceutical composition described above, wherein the disease is a respiratory disease. The present disclosure provides a method for inhibiting mRNA or target gene expression in a patient, comprising administering to the patient a therapeutically effective amount of the nucleic acid-targeting ligand conjugate or the pharmaceutically acceptable salt thereof described above or the pharmaceutical composition described above.

The present disclosure provides a method for preventing and/or treating a disease mediated by RAGE gene expression, comprising administering to a patient a therapeutically effective amount of the nucleic acid-targeting ligand conjugate or the pharmaceutically acceptable salt thereof described above or the pharmaceutical composition described above.

In some embodiments, the respiratory disease described above includes, but is not limited to, cystic fibrosis, chronic bronchitis, non-cystic fibrosis bronchiectasis, idiopathic pulmonary fibrosis (IPF), chronic obstructive pulmonary disease (COPD), asthma, respiratory tract infections, and primary ciliary dyskinesia.

In some embodiments, the cystic fibrosis includes, but is not limited to, lung cancer cystic fibrosis.

The present disclosure provides a method for delivering one or more transported molecules to cells, comprising administering to a patient a therapeutically effective amount of the nucleic acid-targeting ligand conjugate or the pharmaceutically acceptable salt thereof described above or the pharmaceutical composition described above.

In some embodiments, the cells are type I and type II alveolar epithelial cells, goblet cells, secretory epithelial cells, ciliated epithelial cells, corneal and conjunctival epithelial cells, dermal epithelial cells, bile duct epithelial cells, intestinal epithelial cells, ductal epithelial cells, glandular epithelial cells, epithelial tumors (carcinomas), or renal cells.

In some embodiments, delivery may be by local administration (e.g., direct injection or implantation) or systemic administration, or by oral, rectal, or parenteral routes; the parenteral routes include, but are not limited to, subcutaneous injection, intravenous injection, intramuscular injection, intraperitoneal injection, transdermal administration, inhalation administration (e.g., aerosol), mucosal administration (e.g., sublingual or intranasal administration), intracranial administration, etc.

In some embodiments, the nucleic acid-targeting ligand conjugate or pharmaceutical composition provided by the present disclosure may be administered by injection, for example, intravenous, intramuscular, intradermal, subcutaneous, intraduodenal, or intraperitoneal injection.

In some embodiments, the nucleic acid-targeting ligand conjugate or pharmaceutical composition provided by the present disclosure may be packaged in a kit.

The present disclosure further provides a cell comprising the nucleic acid-targeting ligand conjugate described above.

The present disclosure further provides a kit comprising the nucleic acid-targeting ligand conjugate described above or the pharmaceutical composition described above.

The present disclosure provides a preparation method for the nucleic acid-targeting ligand conjugate described above, comprising a step of preparing the nucleic acid-targeting ligand conjugate from a nucleic acid.

The present disclosure provides a preparation method for the nucleic acid-targeting ligand conjugate described above, comprising the following steps: taking a universal CPG support as a start, linking nucleoside monomers one by one in the 3'-5' direction in an order in which nucleotides are arranged, linking the alkynyl-containing compound represented by formula I described above to a synthesized nucleic acid strand, then linking the targeting group described above to an alkynyl-containing moiety, and performing annealing to give the nucleic acid-targeting ligand conjugate.

In some embodiments, the targeting group is as described in formulas (III-a)-(III-p).

In some embodiments, the linking of each monomer involves four reactions: deprotection, coupling, capping, and oxidation or sulfurization; after the last monomer is linked, the nucleic acid sequence linked to the solid-phase support is cleaved, deprotected, purified, and desalted to give the nucleic acid-targeting ligand conjugate.

### Definitions of Terms

In another aspect, where the present disclosure does not define a particular configuration, the compounds of the present disclosure may exist in particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof fall within the scope of the present disclosure.

In addition, the compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization. An example of a lactam-lactim equilibrium is present between A and B as shown below.

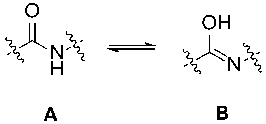

All compounds in the present disclosure can be drawn as form A or form B. All tautomeric forms fall within the scope of the present disclosure. The names of the compounds do not exclude any tautomers.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

The term "alkyl" refers to a saturated aliphatic hydrocarbon group that is a linear or branched group containing 1 to 30 carbon atoms, preferably an alkyl group containing 1 to 12 carbon atoms, more preferably an alkyl group of 1 to 10 carbon atoms, more preferably an alkyl group of 1 to 6 carbon atoms, and further preferably an alkyl group of 1 to 2, 1 to 3, 1 to 4, or 1 to 5 carbon atoms. Alkyl groups containing 1, 2, 3, 4, 5, or 6 carbon atoms are most preferred. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted; when it is substituted, the substituent may be substituted at any available point of attachment, and the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group.

The term "cycloalkyl" or "carbocycle" refers to a saturated or partially unsaturated, monocyclic or polycyclic hydrocarbon substituent, and the cycloalkyl ring contains 3 to 20 carbon atoms, preferably 3 to 7 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be substituted or unsubstituted; when it is substituted, the substituent may be substituted at any available point of attachment, and is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more groups selected from the group consisting of halogen, hydroxy, oxo, nitro, and cyano.

The cycloalkyl ring may be fused to an aryl or heteroaryl ring, wherein the ring attached to the parent structure is cycloalkyl; non-limiting examples include indanyl, tetrahydronaphthyl, benzocycloheptyl, etc. Cycloalkyl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5-to 6-membered heteroaryl are optionally substituted with one or more groups selected from the group consisting of halogen, hydroxy, oxo, nitro, and cyano.

The term "heterocycloalkyl" or "heterocycle" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, and S(O)ₘ (where m is an integer from 0 to 2), but a ring moiety of -O-O-, -O-S-, or -S-S- is excluded, and the other ring atoms are carbon atoms. Preferably, it contains 3 to 12 ring atoms, 1-4 of which are heteroatoms; more preferably, it contains 3 to 7 ring atoms. Non-limiting examples of monocyclic heterocycloalkyl groups include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc. Polycyclic heterocycloalkyl groups include spiro-ring, fused-ring, and bridged-ring heterocycloalkyl groups. Non-limiting examples of "heterocycloalkyl" groups include: 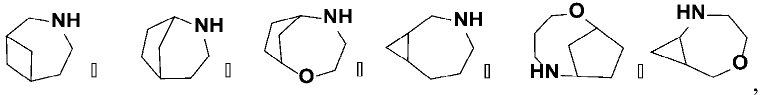 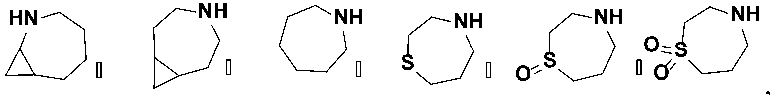 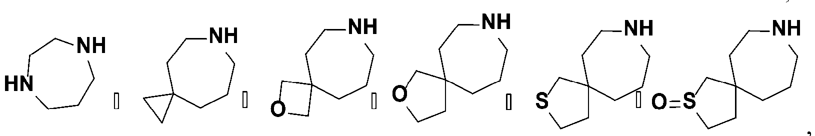 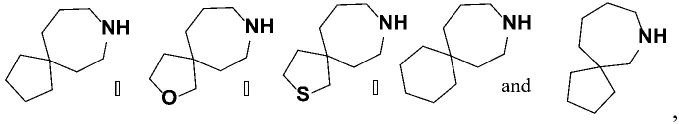 etc.

The heterocycloalkyl ring may be fused to an aryl or heteroaryl ring, wherein the ring attached to the parent structure is heterocycloalkyl; its non-limiting examples include: 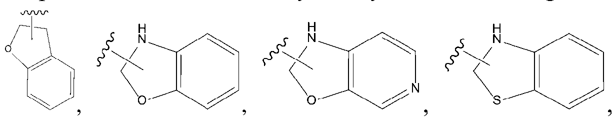 etc.

Heterocycloalkyl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more groups selected from the group consisting of halogen, hydroxy, oxo, nitro, and cyano.

The term "aryl" refers to a 6- to 14-membered, preferably 6- to 12-membered, all-carbon monocyclic or fused polycyclic (i.e., rings sharing a pair of adjacent carbon atoms) group having a conjugated π-electron system, such as phenyl and naphthyl. The aryl ring may be fused to a heteroaryl, heterocycloalkyl, or cycloalkyl ring, wherein the ring attached to the parent structure is the aryl ring; its non-limiting examples include:

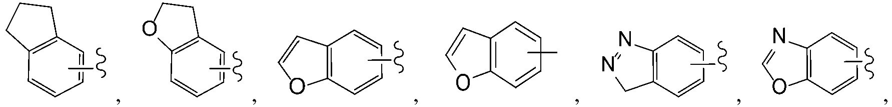

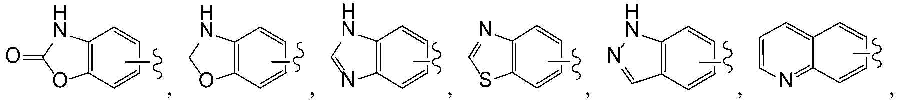

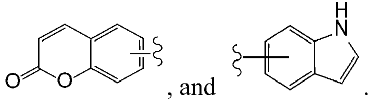

Aryl may be substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more groups selected from the group consisting of halogen, hydroxy, oxo, nitro, and cyano.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 12-membered, and is more preferably 5- or 6-membered. For example, its non-limiting examples include imidazolyl, furanyl, thienyl, thiazolyl, pyrazolyl, oxazolyl, isoxazolyl, pyrrolyl, tetrazolyl, pyridyl, pyrimidinyl, thiadiazole, pyrazinyl, triazolyl, indazolyl, benzimidazolyl, 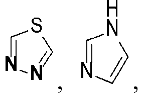 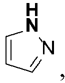 etc.

The heteroaryl ring may be fused to an aryl, heterocycloalkyl, or cycloalkyl ring, wherein the ring attached to the parent structure is the heteroaryl ring; its non-limiting examples include:

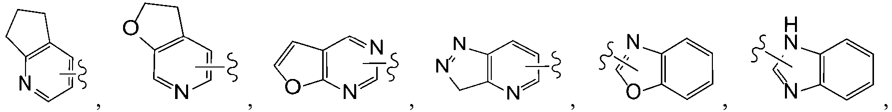

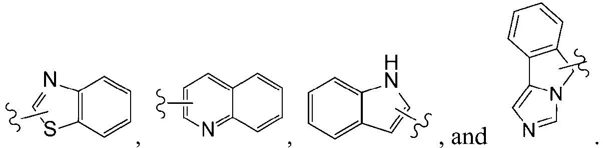

Heteroaryl may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more groups selected from the group consisting of halogen, hydroxy, oxo, nitro, and cyano.

The cycloalkyl, heterocyclyl, aryl, and heteroaryl described above include residues derived by removal of one hydrogen atom from a ring atom of the parent structure, or residues derived by removal of two hydrogen atoms from the same ring atom or two different ring atoms of the parent structure, i.e., "cycloalkylene", "heterocyclylene", "arylene", and "heteroarylene".

The term "spiro-ring" refers to compounds in which two rings share one atom.

The term "spirocycloalkyl" refers to a 5- to 20-membered polycyclic group in which one carbon atom (referred to as a spiro atom) is shared between monocyclic rings; it may contain one or more double bonds, but no ring has a fully conjugated π-electron system. It is preferably 6- to 14-membered, and is more preferably 7- to 10-membered. According to the number of spiro atoms shared between rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl. "Spirocarbocycle" refers to the ring system in spirocycloalkyl. Non-limiting examples of spirocycloalkyl groups include:

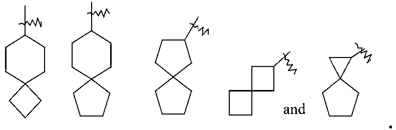

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from the group consisting of nitrogen, oxygen, and S(O)ₘ (where m is an integer from 0 to 2), and the other ring atoms are carbon atoms. It may contain one or more double bonds, but no ring has a fully conjugated π-electron system. It is preferably 6- to 14-membered, and is more preferably 7- to 10-membered. According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl. "Spiroheterocycle" refers to the ring system in spiroheterocyclyl. Non-limiting examples of spiroheterocyclyl groups include:

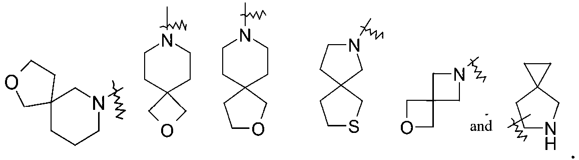

The term "fused-ring" refers to compounds formed by fusing two or more rings by sharing two adjacent atoms.

The term "fused cycloalkyl" refers to a 5- to 20-membered all-carbon polycyclic group in which each ring in the system shares a pair of adjacent carbon atoms with other rings in the system, wherein one or more rings may contain one or more double bonds, but no ring has a fully conjugated π-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl. Non-limiting examples of fused cycloalkyl groups include:

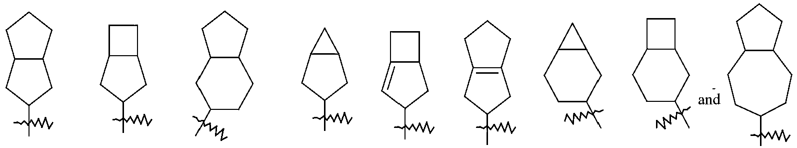

The term "fused heterocyclyl" refers to a 5- to 20-membered polycyclic heterocyclyl group in which each ring in the system shares a pair of adjacent atoms with other rings in the system, wherein one or more rings may contain one or more double bonds, but no ring has a fully conjugated π-electron system; one or more ring atoms are heteroatoms selected from the group consisting of nitrogen, oxygen, and S(O)ₘ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl. "Fused heterocycle" refers to the ring system in fused heterocyclyl. Non-limiting examples of fused heterocyclyl groups include:

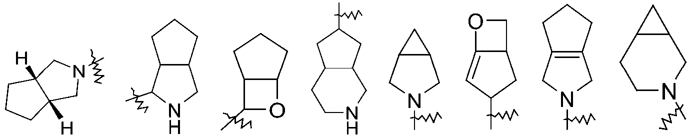

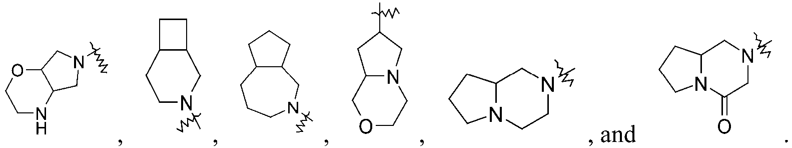

The term "fused heteroaryl" may be an unsaturated aromatic fused ring structure containing 5-14 ring atoms (including at least one heteroatom) formed by connecting two or more cyclic structures that share two adjacent atoms, including the case in which a carbon atom, a nitrogen atom, and a sulfur atom may be substituted with oxo, preferably "5-12 membered fused heteroaryl", "7-12 membered fused heteroaryl", "9-12 membered fused heteroaryl", etc., for example, benzofuranyl, benzoisofuranyl, benzothienyl, indolyl, isoindolyl, benzoxazolyl, benzimidazolyl, indazolyl, benzotriazolyl, quinolyl, 2-quinolinonyl, 4-quinolinonyl, 1-isoquinolinonyl, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazinyl, and phenothiazinyl. "Fused heteroaromatic ring" refers to the ring system in fused heteroaryl.

Fused heteroaryl may be optionally substituted or unsubstituted; when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, and a carboxylate group.

The term "bridged-ring" refers to a structure formed by two or more cyclic structures sharing two non-adjacent ring atoms.

The term "bridged cycloalkyl" refers to a 5- to 20-membered all-carbon polycyclic group in which any two rings share two carbon atoms that are not directly connected; it may contain one or more double bonds, but no ring has a fully conjugated π-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl groups include:

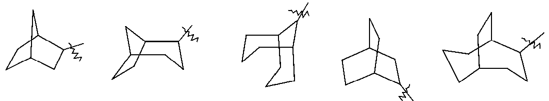

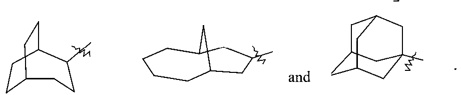

The term "bridged heterocyclyl" refers to a 5- to 14-membered polycyclic heterocyclyl group in which any two rings share two atoms that are not directly connected; it may contain one or more double bonds, but no ring has a fully conjugated π-electron system, wherein one or more ring atoms are heteroatoms selected from the group consisting of nitrogen, oxygen, and S(O)ₘ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl groups include:

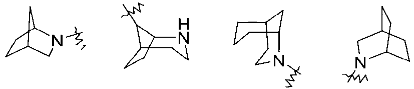

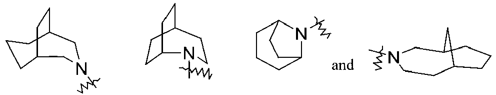

The term "alkoxy" refers to -O-(alkyl) and -O-(unsubstituted cycloalkyl), wherein the alkyl is as defined above. Non-limiting examples of alkoxy groups include methoxy, ethoxy, propoxy, butoxy, cyclopropyloxy, cyclobutoxy, cyclopentyloxy, and cyclohexyloxy. Alkoxy may be optionally substituted or unsubstituted; when it is substituted, the substituent is preferably one or more groups independently selected from the group consisting of halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, and 5- to 6-membered heteroaryl are optionally substituted with one or more groups selected from the group consisting of halogen, hydroxy, oxo, nitro, and cyano. Likewise, the definitions of "alkynyloxy", "alkenyloxy", "cycloalkoxy", "heterocycloalkoxy", and "cycloalkenyloxy" are similar to the above definition of "alkoxy".

The term "hydroxy" refers to -OH.

The term "halogen" refers to fluorine, chlorine, bromine, or iodine.

The term "haloalkyl" refers to an alkyl group substituted with halogen, wherein the alkyl group is as defined above.

The term "cyano" refers to -CN.

The term "nitro" refers to -NO₂.

The term "oxo" refers to the =O group. For example, a carbon atom and an oxygen atom are connected by a double bond to form a ketone or aldehyde group.

The term "amino" refers to -NH₂.

The term "carboxyl" refers to -C(O)OH.

The term "aldehyde group" refers to -CHO.

The term "benzoyl" refers to -Bz.

The term "methoxyphenyldiphenylmethyl" refers to -MMTr.

The term "dimethoxytrityl" refers to -DMTr.

"Optionally" or "optional" means that the event or circumstance subsequently described may, but does not necessarily, occur; this description includes an instance where the event or circumstance occurs or does not occur. For example, "C₁₋₆ alkyl optionally substituted with halogen or cyano" means that the halogen or cyano may, but does not necessarily, exist; this description includes an instance where the alkyl is substituted with halogen or cyano and an instance where the alkyl is not substituted with halogen or cyano.

The term "substituted" means that one or more, preferably up to 5, and more preferably 1 to 3, hydrogen atoms in the group are independently substituted with a corresponding number of substituents. It goes without saying that a substituent is only in its possible chemical position, and those skilled in the art can determine (experimentally or theoretically) possible or impossible substitution without undue effort.

"Substituted with one or more..." refers to substitution with a single substituent or multiple substituents. In the case of substitution with multiple substituents, there may be a plurality of identical substituents, or one or a group of a plurality of different substituents.

The term "link", when used to refer to a relationship between two molecules, means that the two molecules are linked by a covalent bond or that the two molecules are associated via a non-covalent bond (e.g., a hydrogen bond or an ionic bond).

In the chemical structures of the compounds of the present disclosure, the bond " 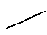 " indicates an unspecified configuration; that is, if chiral isomers exist in the chemical structures, the bond " 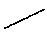 " may be " 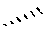 " or " 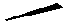 ", or includes both the configurations " 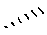 " and " 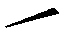 " simultaneously. Although all of the structural formulas described above are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, the bond " 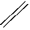 " does not specify a configuration; that is, the configuration of the bond " 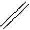 " may be an E configuration or a Z configuration, or includes both the E configuration and the Z configuration simultaneously.

Unless otherwise indicated, the symbol 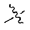 as used herein means that it may be linked to any group or groups according to the scope of the disclosure described herein.

Unless otherwise indicated, the variable may be oriented such that any one point of attachment on the variable is linked to any one point of attachment on the compound of formula I. For example, the variable L₁ has two points of attachment to the compound of formula I. Although L₁ is shown as -(CH₂)₆-O-CH₂- in one embodiment, this embodiment should also be understood as a compound in which L₁ is -CH₂-O-(CH₂)₆-.

In the present disclosure, the terms "comprise" and "include" can be replaced with "consist of".

The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. The term "pharmaceutically acceptable excipient" includes, but is not limited to, any auxiliary, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

The term "pharmaceutically acceptable salt" includes pharmaceutically acceptable acid addition salts and pharmaceutically acceptable base addition salts. As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level may be any type of control level used in the art, such as a pre-dose baseline level or a level determined from a similar untreated or control (e.g., buffer-only control or inert agent control) treated subject, cell, or sample. For example, the remaining mRNA expression level can be used to characterize the degree of inhibition of target gene expression by siRNA; for example, the remaining mRNA expression level is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo^{®} Luciferase Assay System: the Firefly chemiluminescence value and the Renilla chemiluminescence value are each read, and the relative value Ratio = Ren/Fir is calculated; in the present disclosure, the ratio of the remaining mRNA expression level (or residual activity%) = Ratio (siRNA-treated group)/Ratio (siRNA-free control group), and the inhibition rate (%) = 100% - remaining mRNA expression level (%).

"Effective amount", "effective dose", "effective therapeutic amount", or "therapeutically effective amount" refers to the amount of a drug, a compound, or a pharmaceutical composition necessary to obtain any one or more beneficial or desired therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen to avoid significant side effects or toxic effects.

As used herein, "object", "patient", "subject", and "individual" are used interchangeably and include human or non-human animals, e.g., mammals, e.g., humans or monkeys.

In the present disclosure, the term "nucleic acid" refers to a polymeric form of nucleotides of any length. They may comprise one or more ribonucleotides or deoxyribonucleotides. Thus, the term includes, but is not limited to, single-stranded, double-stranded, or multi-stranded DNA or RNA, genomic DNA, cDNA, DNA-RNA hybrids, or polymers comprising purine and pyrimidine bases or other natural, chemically or biochemically modified, non-natural, or derivatized nucleotide bases, e.g., locked nucleic acid (LNA) and peptide nucleic acid (PNA). Preferably, the nucleic acid includes, but is not limited to, single-stranded oligonucleotides, single-stranded antisense oligonucleotides, short interfering RNAs (siRNAs, the siRNAs comprising partially complementary sense and antisense strands), double-stranded RNAs (dsRNAs), microRNAs (miRNAs), short hairpin RNAs (shRNAs), and Dicer substrates.

In the present disclosure, "phosphoester group" and "phosphate linkage" are used interchangeably and include phosphomonoesters, phosphodiesters, or phosphotriesters. The "phosphoester group" in the "phosphorothioate group" also has the same meaning. Unless otherwise specified, the natural internucleotide phosphoester group is a phosphodiester group.

As used herein, in the context of RNA-mediated gene silencing, the sense strand (also referred to as SS or SS strand) of an siRNA refers to a strand that comprises a sequence that is identical or substantially identical to a target mRNA sequence; the antisense strand (also referred to as AS or AS strand) of an siRNA refers to a strand having a sequence complementary to a target mRNA sequence.

As used herein, the first strand may be referred to as the antisense strand, and the second strand may be referred to as the sense strand. The terms first strand and antisense strand or second strand and sense strand should be considered interchangeable.

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

As used herein, the term "base" encompasses any known DNA and RNA bases and base analogs such as purines or pyrimidines, and also includes the natural compounds adenine, thymine, guanine, cytosine, uracil, inosine, and natural analogs.

Unless otherwise specified, in the context of the present disclosure, "G", "C", "A", "T", and "U" represent nucleotides, and comprise the bases of guanine, cytosine, adenine, thymidine, and uracil, respectively. It is well-known to those skilled in the art that the mutual replacement between bases T and U does not significantly affect the properties of the nucleic acid sequence. In the sequences of the present disclosure, U can be arbitrarily replaced with T, and the sequences after the replacement also fall within the protection scope of the present disclosure. In the sequences of the present disclosure, for the same nucleic acid strand, the direction from the 5' end to the 3' end is the direction from the left to the right, and the lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; the lowercase letter s means that the two nucleosides adjacent to the letter s are linked by a phosphorothioate diester group; unless otherwise specified, two nucleosides are linked by a phosphodiester group; VP means that the 5' end of the nucleoside adjacent to the letters on the left/right side is a 5'-vinylphosphodiester group, and the 5'-vinylphosphodiester group may be in a cis or trans configuration, which means that the group may be a 5'-E-VP isomer (i.e., a trans-vinylphosphodiester group), a 5'-Z-VP isomer (i.e., a cis-vinylphosphodiester group), or a mixture thereof; IB represents a reverse abasic nucleoside; (-)hmpNA means that a nucleoside in parentheses adjacent thereto is a nucleoside having an hmpNA modification. Unless otherwise specified, the "nucleotide", "compound", "chemical modification", "oligonucleotide", "siRNA", "dsRNA", and "nucleic acid" of the present disclosure can each independently be present in the form of a salt or mixed salt or in a non-salt form (e.g., a free acid or free base). When present in the form of a salt or mixed salt, it may be a pharmaceutically acceptable salt. The term "pharmaceutically acceptable salt" includes pharmaceutically acceptable acid addition salts and pharmaceutically acceptable base addition salts. When it is present in the form of a salt, part of the groups may be ionized to form anions/cations; for example, phosphodiester groups and phosphorothioate diester groups may be present in the form of anions, and the structures in the form of a salt corresponding to the following structures also fall within the protection scope of the present disclosure.

The modifications and linking groups described above separately have the structures shown in the table below, where Base represents a base:

| Structure | Name |
|---|---|
| 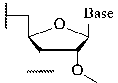 | 2'-Methoxy-modified nucleoside |
| 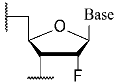 | 2'-Fluoro-modified nucleoside |
| 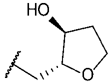 A bond facing the 3' end of the oligonucleotide ; | Reverse nucleoside without base modification when located at the 5' end of the sequence |
| A bond facing the 5' end of the oligonucleotide 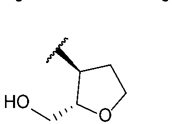 | Reverse nucleoside without base modification when located at the 3' end of the sequence |
| 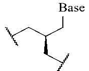 wherein Base represents a base; in the direction from the 5' end to the 3' end of the sequence, "*" represents a site where the nucleoside is linked to a nucleoside adjacent to its 5' end, and "#" represents a site where the nucleoside is linked to a nucleoside adjacent to its 3' end | hmpNA-modified nucleoside |
| 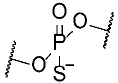 | Anion form of phosphorothioate diester group |
| 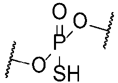 | Phosphorothioate diester group |
| 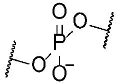 | Anion form of phosphodiester group |
| 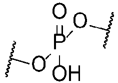 | Phosphodiester group |
| 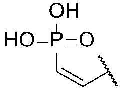 | Z-Vinylphosphoric acid group |
| 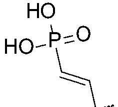 | E-Vinylphosphoric acid group |

The term "pharmaceutically acceptable salt" includes pharmaceutically acceptable acid addition salts and pharmaceutically acceptable base addition salts.

"Pharmaceutically acceptable acid addition salt" refers to salts formed with inorganic or organic acids, capable of retaining the biological effectiveness of free bases without having any other side effect, which can be prepared by methods known in the art. "Pharmaceutically acceptable base addition salt" refers to salts formed with inorganic bases or organic bases, capable of retaining the biological effectiveness of free acids without having any other side effect, which can be prepared by methods known in the art. As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of another strand, and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

The term "partially complementary" means that in the siRNA, a portion of a contiguous sequence in the sequence of the sense strand is complementary to a portion of a contiguous sequence in the sequence of the antisense strand through base pairing, such that the sense strand and the antisense strand can bind complementarily. In one embodiment, the complementary region may be 10 or more (e.g., 19, 20, 21, 30, 40, 50, or 100) contiguous nucleotides, and the non-complementary region may be 1 contiguous nucleotide, 2 contiguous nucleotides, 3 contiguous nucleotides, 4 contiguous nucleotides, 5 contiguous nucleotides, or the like, or 10 contiguous nucleotides or contiguous nucleotides of a longer fragment (20, 30, 40, 50, or the like).

As used herein, "chemical modification" or "modification" means a structure that has chemical differences when compared with a naturally occurring counterpart, including all changes made by chemical means, such as addition or removal of chemical moieties, or substitution of one chemical moiety for another.

As used herein, the term "2'-fluoro-modified nucleotide" refers to a nucleotide formed by substituting the hydroxy group at the 2' position of the ribosyl group of the nucleotide with fluorine; the term "methoxy-modified nucleotide" or "2'-methoxy-modified nucleotide" refers to a nucleotide formed by substituting the 2'-hydroxy group of the ribosyl group with a methoxy group.

### BRIEF DESCRIPTION OF THE DRAWINGS

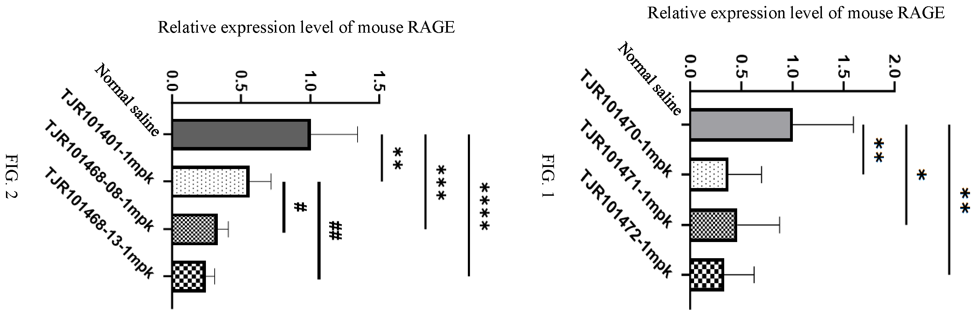

FIG. 1 shows the inhibitory activity of TJR101470, TJR101471, and TJR101472 against mouse lung RAGE 14 days after administration, wherein the data results are expressed as mean ± SD. In the figure, ** represents p < 0.01, and * represents p < 0.05 compared to the normal saline group.
FIG. 2 shows the inhibitory activity of TJR101401, TJR101468-08, and TJR101468-13 against mouse lung RAGE 14 days after administration, wherein the data results are expressed as mean ± SD. In the figure, **** represents p < 0.0001, *** represents p < 0.001, and ** represents p < 0.01 compared to the normal saline group. In the drawing, # represents p < 0.05, and ## represents p < 0.01 compared to the TJR101401 group.

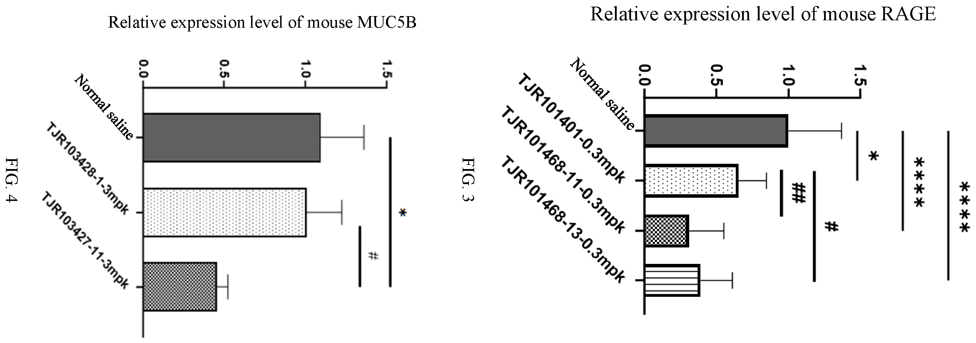

FIG. 3 shows the inhibitory activity of TJR101401, TJR101468-11, and TJR101468-13 against mouse lung RAGE 28 days after administration, wherein the data results are expressed as mean ± SD. In the figure, **** represents p < 0.0001, and * represents p < 0.05 compared to the normal saline group. In the drawing, # represents p < 0.05, and ## represents p < 0.01 compared to the TJR101401 group.
FIG. 4 shows the inhibitory activity of TJR103427-11 and TJR103428-1 against mouse lung MUC5B 28 days after administration, wherein the data results are expressed as mean ± SD. In the figure, * represents p < 0.05 compared to the normal saline group. In the drawing, # represents p < 0.05 compared to the TJR103428-1 group.

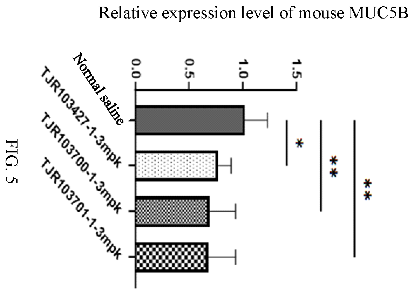

FIG. 5 shows the inhibitory activity of TJR103427-1, TJR103700-1, and TJR103701-1 against mouse lung MUC5B 14 days after administration, wherein the data results are expressed as mean ± SD. In the figure, ** represents p < 0.01, and * represents p < 0.05 compared to the normal saline group.

### DETAILED DESCRIPTION

The present disclosure is further described below with reference to examples; however, these examples are not intended to limit the scope of the present disclosure. Experimental methods without specific conditions indicated in the examples of the present disclosure were generally conducted under conventional conditions, or conditions recommended by the manufacturers of the starting materials or commercial products. Where the specific source of a reagent is not indicated, the reagent may be obtained from any supplier of molecular biology reagents at a quality/purity level for molecular biology applications.

### List of abbreviations

FmocCl: 9-fluorenylmethyl chloroformate
DCM: dichloromethane
DIPEA, DIEA: N-ethyldiisopropylamine
TBTU: O-(benzotriazol-1-yl)-N,N,N',N'-tetramethyluronium tetrafluoroborate
TEA, Et3N: triethylamine
DMF: N,N-dimethylformamide
EDCI: 1-ethyl-(3-dimethylaminopropyl)carbodiimide hydrochloride
MeOH: methanol
EtOH: ethanol
TFA: trifluoroacetic acid
DMSO: dimethyl sulfoxide
NMM: N-methylmorpholine
TDBSPCl: tert-butyl(chloro)diphenylsilane
DMAP: 4-(dimethylamino)pyridine
THF: tetrahydrofuran
PE: petroleum ether
EA: ethyl acetate
N₃-PEG₅-Tos: azide-PEG5-Tos
NaHMDS: sodium bis(trimethylsilyl)amide
HATU: O-(7-azabenzotriazol-1-yl)-N,N,N',N'-tetramethyluronium hexafluorophosphate
LCMS: liquid chromatography mass spectrometry
NMR: nuclear magnetic resonance

### Example 1: Synthesis of Compound I-1

Unless otherwise specified, all reagents used in the following examples were commercially available. The synthesis scheme for compound **I-1** is as follows:

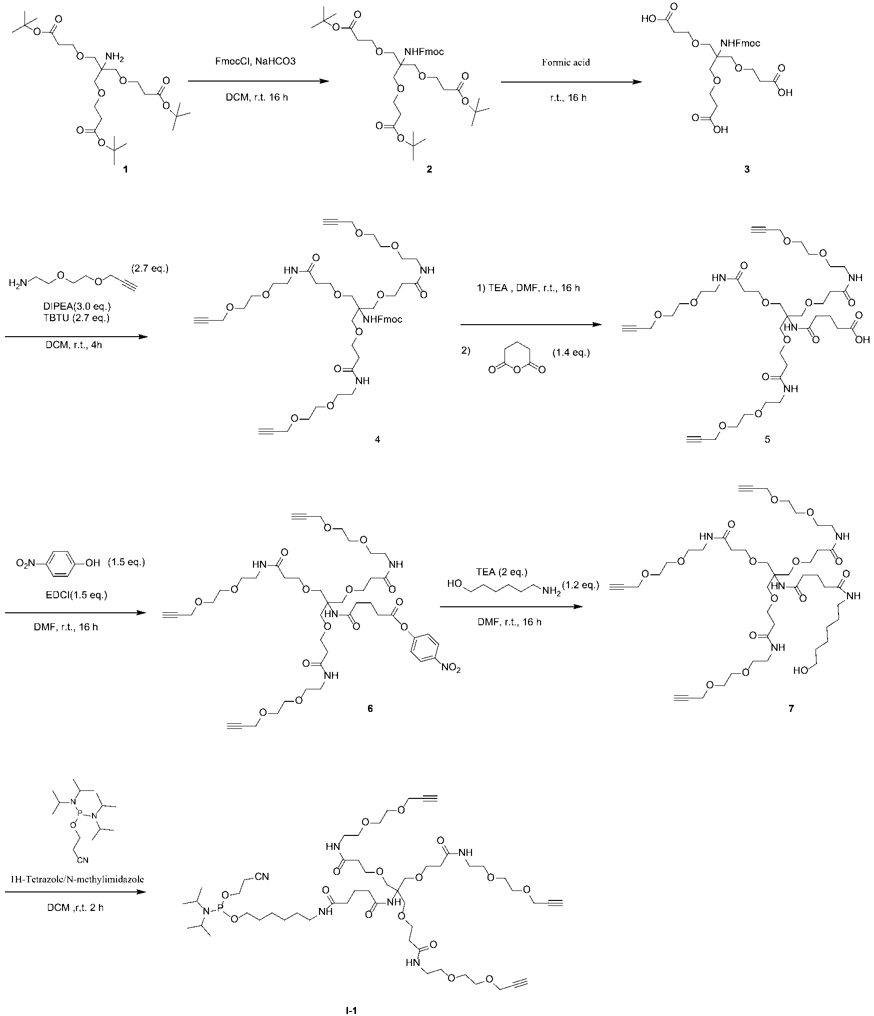

The synthesis and identification of specific intermediates and final products involved in the above scheme are as follows:

### Compound 2:

A solution of compound **1** (3.00 g, 5.93 mmol) in DCM (70 mL) was added to a 20% solution of Na₂CO₃ (30 mL), and a solution of 9-fluorenylmethyl chloroformate (4.60 g, 17.8 mmol) in DCM (20 mL) was slowly added. The mixture was stirred at room temperature overnight, and LCMS analysis showed that the reaction was complete. The reaction mixture was left to stand. After layers formed, the organic phase was separated, washed with saturated brine, dried over Na₂SO₄, and concentrated, and the resulting crude product was purified by normal-phase silica gel column chromatography (eluent PE:EA = 5:1, product peak at 20%) and concentrated to dryness by rotary evaporation to give compound **2** (3.20 g, yield: 74.07%).

**LCMS:** tR = 3.62 min, MS (ESI) m/z = 750.8 [M+Na]⁺.

### Compound 3:

Compound **2** (3.2 g, 4.40 mmol) was dissolved in formic acid (10 mL). The solution was stirred overnight, and LCMS monitoring showed that the reaction was complete. The reaction mixture was concentrated to dryness by rotary evaporation to give a crude product of compound **3** (2.50 g), and the crude product was directly used in the next step. **LCMS:** tR = 0.99 min, MS (ESI) m/z = 560.5 [M+H]⁺.

### Compound 4:

At 0 °C, compound **3** (2.50 g, 4.46 mmol) was dissolved in a solution of DCM (20 mL). In a nitrogen atmosphere, TBTU (5.74 g, 17.87 mmol) and propynyl-tripolyethylene glycol-amino (2.56 g, 17.87 mmol) were sequentially added, and DIPEA (4.4 mL, 26.8 mmol) was slowly added dropwise over 5 min. After the reaction mixture was stirred at room temperature for 2 h, LCMS analysis showed that the starting materials disappeared. After the reaction mixture was quenched with water, the organic phase was separated, washed with saturated brine, dried over Na₂SO₄, and concentrated, and the resulting crude product was purified by reversed-phase column chromatography to give compound **4** (3.89 g, yield: 93.11%).

**LCMS:** tR = 2.37 min, MS (ESI) m/z = 935.9 [M+H]⁺.

### Compound 5:

At 0 °C, triethylamine (7.5 mL) was added to a solution of compound **4** (3.89 g, 4.16 mmol) in DMF (20 mL). After the mixture was stirred at room temperature overnight, glutaric anhydride (0.62 g, 5.40 mmol) was added. The mixture was left to react for another 2 h, and LCMS analysis showed that the starting materials disappeared, indicating that the reaction was complete. The reaction mixture was concentrated and then directly purified by reversed-phase column chromatography to give compound **5** (3.20 g, yield: 93.02%).

**LCMS:** tR = 1.22 min, MS (ESI) m/z = 827.8 [M+H]⁺.

### Compound 6:

At 0 °C, 4-nitrophenol (0.81 g, 5.80 mmol) and EDCI (1.11 g, 5.80 mmol) were added to a solution of compound 5 (3.20 g, 3.87 mmol) in DMF. The reaction was stirred at room temperature overnight, and LCMS analysis showed that the reaction was complete. The reaction mixture was quenched with water and then extracted three times with ethyl acetate (40 mL). The combined organic phases were washed with brine, dried over Na₂SO₄, and concentrated, and the resulting crude product was purified by reversed-phase column chromatography to give compound **6** (3.00 g, yield: 81.74%).

**LCMS:** tR = 1.67 min MS (ESI) m/z = 948.8 [M+H]⁺.

### Compound 7:

The compounds 6-amino-1-hexanol (0.50 g, 4.28 mmol) and Et₃N (0.94 mL, 6.73 mmol) were added to a solution of compound **6** (2.90 g, 3.06 mmol) in DCM (20 mL). The reaction was stirred overnight, and LCMS analysis showed that the reaction was complete. After the reaction mixture was quenched with brine, the organic phase was dried over Na₂SO₄ and concentrated to dryness by rotary evaporation, and the resulting crude product was purified by reversed-phase column chromatography to give compound **7** (2.31 g, yield: 81.62%).

**LCMS:** tR = 1.42 min, MS (ESI) m/z = 926.9 [M+H]⁺.

### Compound I-1:

At 0 °C, in a nitrogen atmosphere, a 3A molecular sieve was added to a solution of compound **7** (2.20 g, 2.37 mmol) in dry acetonitrile (15 mL). After 5 min of stirring, bis(diisopropylamino)(2-cyanoethoxy)phosphine (1.58 g, 5.22 mmol) and a pre-formulated solution of 1H-tetrazole (0.45 M) and N-methylimidazole (0.18 M) in dry acetonitrile (5.28 mL) were added. Then, the mixture was warmed to room temperature and left to react for 2 h, and LCMS analysis showed that the reaction was complete. A 5% aqueous sodium bicarbonate solution (40 mL) was added to the reaction mixture, and the mixture was shaken for 1 min to quench the reaction. Then, extraction was performed twice with dichloromethane (50 mL). The combined organic phases were washed with saturated brine, dried over sodium sulfate, filtered, and concentrated to give a crude product of compound **I-1** (2.45 g), and the crude product was directly used in the subsequent solid-phase synthesis.

**LCMS:** tR = 1.84 min, MS (ESI) m/z = 1149.2 [M+Na]⁺. ³¹PNMR (400 MHz, DMSO-d6) δ 146.25.

### Example 2: Synthesis of Compound I-2

Unless otherwise specified, all reagents used in the following examples were commercially available. The synthesis scheme for compound I-2 is as follows:

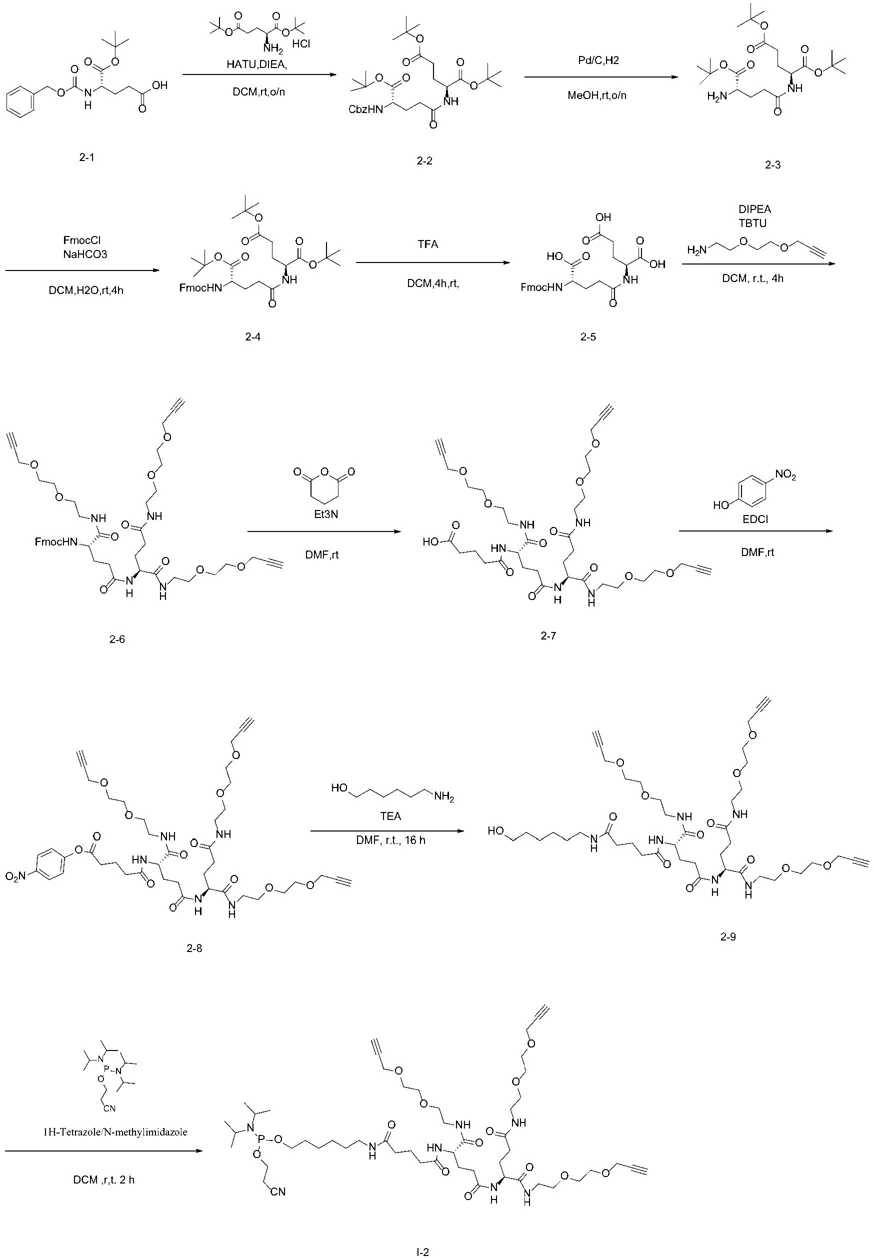

The synthesis and identification of specific intermediates and final products involved in the above scheme are as follows:

### Compound 2-2:

Compound **2-1** (5.03 g, 14.92 mmol) was added to dichloromethane (40 mL), and HATU (6.48 g, 17.04 mmol) and DIEA (5.87 mL, 35.5 mmol) were added at 10 °C. The mixture was naturally warmed to room temperature and left to react for 30 min, and di-tert-butyl L-glutamate hydrochloride (4.20 g, 14.21 mmol) was then added. The mixture was left to react at room temperature overnight, and LCMS analysis showed that the reaction was complete. The reaction mixture was washed with 30 mL of water and dried over Na₂SO₄. After the organic phase was concentrated, the resulting crude product was purified by normal-phase column chromatography to give compound **2-2** (7.20 g, yield: 87.59%). **LCMS:** MS (ESI) m/z = 579.8 [M+H]⁺.

### Compound 2-3:

Compound **2-2** (7.20 g, 12.44 mmol) was added to methanol (50 mL), and Pd/C (700 mg) was then added. The mixture was purged twice using a hydrogen balloon and then left to react at room temperature overnight. After LCMS monitoring showed that the reaction was complete, the reaction mixture was filtered through diatomaceous earth, and the filtrate was concentrated under reduced pressure to give compound **2-3** (4.80 g, yield: 86.8%). **LCMS:** MS (ESI) m/z = 445.8 [M+H]⁺.

### Compound 2-4:

Water (50 mL) and sodium bicarbonate (1.07 g, 12.79 mmol) were separately added to a solution of compound **2-3** (4.74 g, 10.66 mmol) in dichloromethane (120 mL), and a solution of Fmoc-Cl (3.03 g, 11.73 mmol) in DCM (5 mL) was slowly added dropwise. The mixture was stirred at room temperature overnight, and LCMS analysis showed that the starting materials disappeared. After the reaction mixture was left to stand, the organic phase was separated, dried over Na₂SO₄, and concentrated, and the resulting crude product was purified by normal-phase column chromatography to give compound **2-4** (7.00 g, yield: 98.45%). **LCMS:** MS (ESI) m/z = 667.9 [M+H]⁺.

### Compound 2-5:

Trifluoroacetic acid (20 mL) was added to a solution of compound **2-4** (6.92 g, 10.39 mmol) in dichloromethane (10 mL). The mixture was stirred at room temperature for 4 h, and LCMS analysis showed that the reaction was complete. The reaction mixture was directly concentrated to dryness by rotary evaporation to give compound **2-5** (5.61 g), and the compound was directly used in the next step. **LCMS:** MS (ESI) m/z = 499.7 [M+H]⁺.

### Compound 2-6:

At 0 °C, TBTU (10.99 g, 34.23 mmol) and propynyl-tripolyethylene glycol-amino (4.9 g, 34.23 mmol) were sequentially added to a solution of compound **2-5** (6.92 g, 10.38 mmol) in dichloromethane (50 mL), and DIPEA (10.3 mL, 62.23 mmol) was slowly added dropwise. The reaction mixture was naturally warmed to room temperature and stirred for 4 h, and LCMS analysis showed that the reaction was complete. After the reaction mixture was quenched with water, the organic phase was separated, washed with saturated brine, dried over Na₂SO₄, and concentrated, and the resulting crude product was purified by normal-phase column chromatography to give compound **2-6** (8.20 g, yield: 90.51%). **LCMS:** MS (ESI) m/z = 875.1 [M+H]⁺.

### Compound 2-7:

Triethylamine (7.73 mL, 55.61 mmol) was added to a solution of compound **2-6** (8.11 g, 9.27 mmol) in DMF (10 mL), and the mixture was left to react at room temperature for 4 h. Then, glutaric anhydride (1.16 g, 10.20 mmol) was added. The reaction mixture was stirred at room temperature for 1 h, and LCMS analysis showed that the reaction was complete. The reaction mixture was concentrated and then directly purified by reversed-phase column chromatography to give compound **2-7** (3.30 g, yield: 46.48%). **LCMS:** MS (ESI) m/z = 767.0 [M+H]⁺.

### Compound 2-8:

At 0 °C, p-nitrophenol (0.67 g, 4.8 mmol) and EDC-HCl (0.92 g, 4.8 mmol) were separately added to a solution of compound **2-7** (3.34 g, 4.36 mmol) in DMF (30 mL). After the reaction was naturally warmed to room temperature and stirred for 2 h, LCMS analysis showed that the reaction was complete. The reaction mixture was quenched with water (100 mL), and a large amount of solid precipitated. After filtration, the filter cake was washed with water (50 mL × 2) to give compound **2-8** (2.4 g, yield: 62.02%). **LCMS:** MS (ESI) m/z = 888.2 [M+H]⁺.

### Compound 2-9:

6-Amino-1-hexanol (0.35 g, 2.98 mmol) was added to a solution of compound **2-8** (2.40 g, 2.71 mmol) in DCM (10 mL), and triethylamine (0.75 mL, 5.41 mmol) was added dropwise. After the reaction was stirred at room temperature for 1 h, LCMS analysis showed that the reaction was complete. The reaction mixture was concentrated and then directly purified by reversed-phase column chromatography to give compound **2-9** (1.96 g, yield: 83.74%). **LCMS:** MS (ESI) m/z = 866.3 [M+H]⁺.

### Compound I-2:

At 0 °C, in a nitrogen atmosphere, a 3A molecular sieve was added to a solution of compound **2-9** (1.60 g, 1.85 mmol) in dry dichloromethane (10 mL) and acetonitrile (10 mL). After 5 min of stirring, bis(diisopropylamino)(2-cyanoethoxy)phosphine (1.39 g, 4.62 mmol) and a pre-formulated solution of 1H-tetrazole (0.45 M) and N-methylimidazole (0.18 M) in dry acetonitrile (5 mL) were added. Then, the mixture was warmed to room temperature and left to react for 2 h, and LCMS analysis showed that the reaction was complete. A saturated aqueous sodium bicarbonate solution (30 mL) was added to the reaction mixture, and the mixture was shaken for 1 min to quench the reaction. Then, extraction was performed twice with dichloromethane (40 mL). The combined organic phases were washed with saturated brine, dried over sodium sulfate, filtered, and concentrated to give a crude product of compound **I-2** (2.50 g), and the crude product was directly used in the subsequent solid-phase synthesis. **LCMS:** MS (ESI) m/z = 1088.12 [M+Na]⁺. ³¹PNMR (400 MHz, DMSO-d6) δ 146.32.

### Example 3: Synthesis of Compound I-3

Unless otherwise specified, all reagents used in the following examples were commercially available. The synthesis scheme for compound I-3 is as follows:

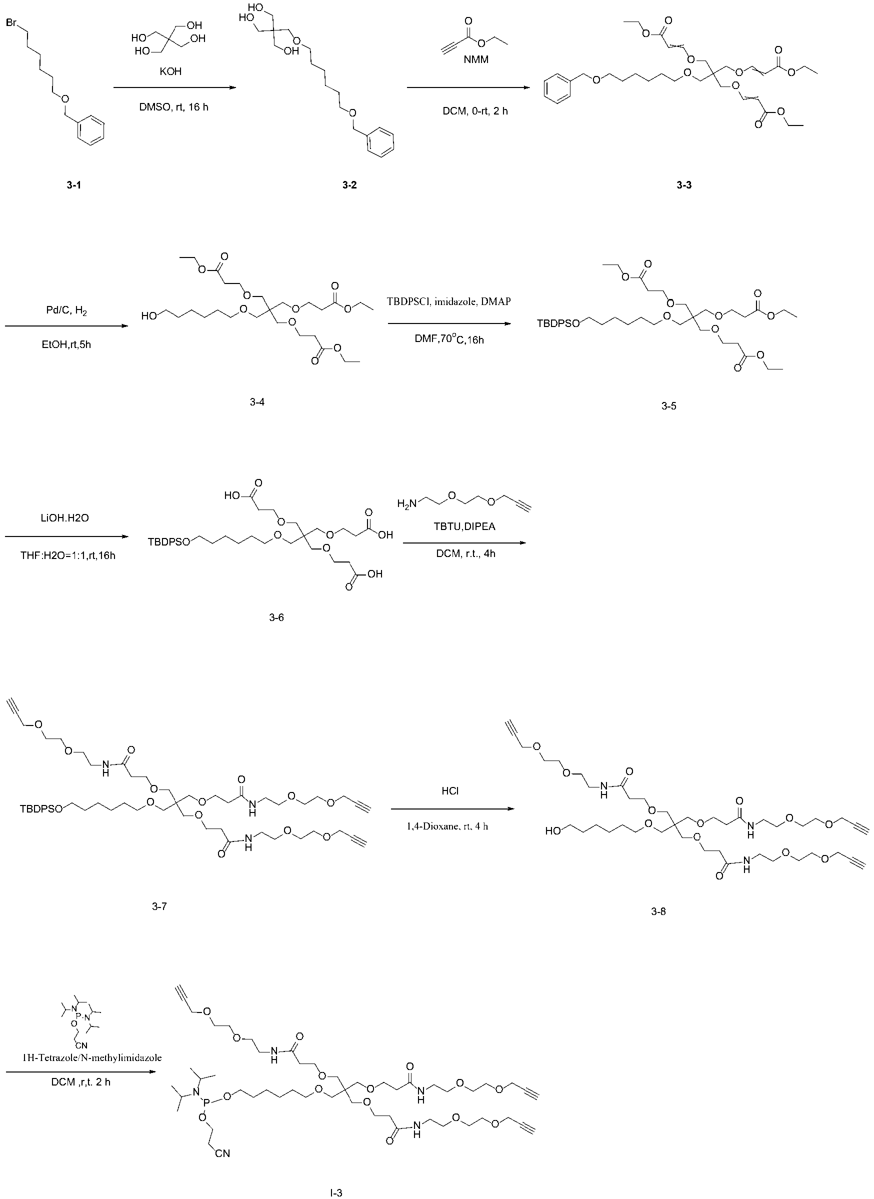

The synthesis and identification of specific intermediates and final products involved in the above scheme are as follows:

### Compound 3-2:

Potassium hydroxide (74.2 g, 1322.1 mmol) was added to a solution of pentaerythritol (180.0 g, 1322.07 mmol) in DMSO (8000 mL). After the reaction was stirred at room temperature for 15 min, a solution of compound **3-1** (29.6 mL, 132.21 mmol) in DMSO (100 mL) was slowly added dropwise. The reaction mixture was stirred at room temperature overnight. After LCMS monitoring showed that the reaction was complete, the pH of the reaction mixture was adjusted to 1 with 3 M HCl, and extraction was performed with DCM (2000 mL × 4). The combined organic phases were washed with saturated brine, dried over Na₂SO₄, and concentrated, and the resulting crude product was purified by normal-phase column chromatography to give compound **3-2** (20 g, yield: 46.3%). **LCMS:** MS (ESI) m/z = 327.2 [M+H]⁺.

### Compound 3-3:

At 0 °C, in a nitrogen atmosphere, N-methylmorpholine (30.3 mL, 275.71 mmol) was added to a solution of compound **3-2** in dichloromethane (200 mL), and ethyl propiolate (24.83 mL, 245.07 mmol) was slowly added dropwise. The mixture was warmed to room temperature and left to react for 2 h. After LCMS monitoring showed that the reaction was complete, the reaction mixture was concentrated and purified by normal-phase column chromatography to give compound **3-3** (25 g, yield: 65.7%). **LCMS:** MS (ESI) m/z = 643.4 [M+Na]⁺.

### Compound 3-4:

Pd/C (300 mg) was added to a solution of compound **3-3** (3.00 g, 4.79 mmol) in ethanol (30 mL). The mixture was purged twice using a hydrogen balloon and stirred at room temperature for 4 h. After LCMS monitoring showed that the reaction was complete, the reaction mixture was filtered through diatomaceous earth, and the filtrate was concentrated under reduced pressure to give compound **3-4** (2.50 g, 97.33%). **LCMS:** MS (ESI) m/z = 537.8 [M+H]⁺.

### Compound 3-5:

Imidazole (0.48 g, 6.99 mmol), TBDPSC1 (1.41 g, 5.12 mmol), and DMAP (0.57 g, 4.66 mmol) were sequentially added to a solution of compound **3-4** (2.50 g, 4.66 mmol) in DMF (20 mL). The reaction was heated to 70 °C and stirred overnight, and LCMS monitoring showed that the reaction was substantially complete. The reaction mixture was quenched with water (100 mL) and extracted with ethyl acetate (50 mL × 2). The combined organic phases were washed with saturated brine, dried over Na₂SO₄, and concentrated, and the resulting crude product was purified by normal-phase column chromatography to give compound **3-5** (3.10 g, yield: 85.86%). **LCMS:** MS (ESI) m/z = 776.1 [M+H]⁺.

### Compound 3-6:

Lithium hydroxide monohydrate (0.49 g, 11.61 mmol) was added to a mixed solution of compound **3-5** (3.10 g, 3.87 mmol) in THF (15 mL) and water (15 mL). The mixture was stirred at room temperature overnight, and LCMS analysis showed that the starting materials disappeared. After the reaction mixture was concentrated, the pH of the crude product was adjusted to 1-2 with 1 N HCl, and extraction was performed with ethyl acetate (20 mL × 2). The combined organic phases were washed with saturated brine (20 mL) and dried over Na₂SO₄. The filtrate was concentrated to give compound **3-6** (3.00 g, yield: 112.18%). **LCMS:** MS (ESI) m/z = 692.0 [M+H]⁺.

### Compound 3-7:

At 0 °C, compound **3-6** (3.00 g, 4.34 mmol) was dissolved in a solution of DCM (30 mL). In a nitrogen atmosphere, TBTU (4.59 g, 14.33 mmol) and propynyl-tripolyethylene glycol-amino (2.05 g, 14.33 mmol) were sequentially added, and DIPEA (2.5 mL, 15.20 mmol) was slowly added dropwise over 5 min. After the reaction mixture was stirred at room temperature for 2 h, LCMS analysis showed that the starting materials disappeared. After the reaction mixture was quenched with water, the organic phase was separated, washed with saturated brine, dried over Na₂SO₄, and concentrated, and the resulting crude product was purified by normal-phase column chromatography to give compound **3-7** (4.30 g, yield: 92.86%). **LCMS:** MS (ESI) m/z = 1067.4 [M+H]⁺.

### Compound 3-8:

Compound **3-7** (4.00 g, 3.75 mmol) was added to a solution of hydrochloric acid in dioxane (20 mL). The mixture was left to react at room temperature for 4 h, and LCMS analysis showed that the reaction was complete. The reaction mixture was concentrated and then directly purified by reversed-phase column chromatography to give compound **3-8** (2.20 g, yield: 70.84%). **LCMS:** MS (ESI) m/z = 829.3 [M+H]⁺.

### Compound I-3:

At 0 °C, in a nitrogen atmosphere, a 3A molecular sieve was added to a solution of compound **3-8** (1.48 g, 1.79 mmol) in dry dichloromethane (10 mL). After 5 min of stirring, bis(diisopropylamino)(2-cyanoethoxy)phosphine (1.19 g, 3.93 mmol) and a pre-formulated solution of 1H-tetrazole (0.45 M) and N-methylimidazole (0.18 M) in dry acetonitrile (4 mL) were added. Then, the mixture was warmed to room temperature and left to react for 2 h, and LCMS analysis showed that the reaction was complete. A saturated aqueous sodium bicarbonate solution (30 mL) was added to the reaction mixture, and the mixture was shaken for 1 min to quench the reaction. Then, extraction was performed twice with dichloromethane (40 mL). The combined organic phases were washed with saturated brine, dried over Na₂SO₄, filtered, and concentrated to give a crude product of compound **I-3** (2.30 g), and the crude product was directly used in the subsequent solid-phase synthesis. **LCMS:** MS (ESI) m/z = 1051.04 [M+Na]⁺, ³¹PNMR (400 MHz, DMSO) δ 146.29.

### Example 4: Synthesis of Compound I-4

Unless otherwise specified, all reagents used in the following examples were commercially available. The synthesis scheme for compound I-4 is as follows:

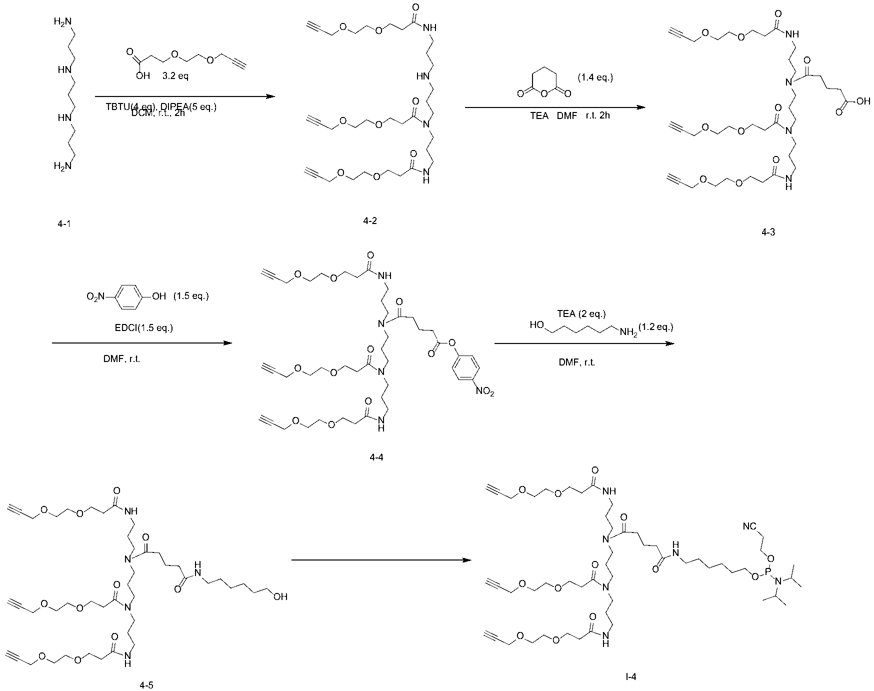

The synthesis and identification of specific intermediates and final products involved in the above scheme are as follows:

### Compound 4-2:

3-{[2-(Prop-2-ynyloxy)ethyl]oxy}propanoic acid (6.64 g, 38.55 mmol), TBTU (16.88 g, 52.57 mmol), and DIEA (14.48 mL, 87.62 mmol) were sequentially added to a solution of compound 4-1 (3.30 g, 17.52 mmol) in DCM (50 mL). After the mixture was left to react at room temperature for 1 h, the reaction was monitored by LCMS, and 3-{[2-(prop-2-ynyloxy)ethyl]oxy}propanoic acid (3.02 g, 17.523 mmol) and TBTU (5.63 g, 17.523 mmol) were added to the reaction system. After 1 h of stirring at room temperature, LCMS monitoring showed that the reaction was complete. The reaction mixture was directly concentrated, and the resulting crude product was purified by reversed-phase column chromatography to give compound 4-**2** (4.00 g, yield: 35.07%). **LCMS:** MS (ESI) m/z = 652.0 [M+H]⁺.

### Compound 4-3:

In a nitrogen atmosphere, TEA (10 mL) and glutaric anhydride (0.98 g, 8.605 mmol) were added to a solution of compound **4-2** (4.00 g, 6.15 mmol) in DMF (50 mL). After the mixture was left to react at room temperature for 2 h, LCMS monitoring showed that the reaction was complete. After the reaction mixture was filtered, the filtrate was purified by reversed-phase column chromatography to give compound **4-3** (3.80 g, yield: 80.83%). **LCMS:** MS (ESI) m/z = 766.3 [M+H]⁺.

### Compound 4-4:

At room temperature, 4-nitrophenol (1.04 g, 7.45 mmol) and EDCI (1.43 g, 7.45 mmol) were added to a solution of compound **4-3** (3.80 g, 4.97 mmol) in DMF. The reaction was stirred at room temperature overnight, and LCMS analysis showed that the reaction was complete. The reaction mixture was quenched with water and then extracted three times with dichloromethane (50 mL). The combined organic phases were washed with brine, dried over Na₂SO₄, and concentrated, and the resulting crude product was purified by reversed-phase column chromatography to give compound **4-4** (2.50 g, yield: 56.80%). **LCMS:** MS (ESI) m/z = 887.3 [M+H]⁺.

### Compound 4-5:

6-Amino-1-hexanol (0.50 g, 4.23 mmol) and Et₃N (0.78 mL, 5.64 mmol) were added to a solution of compound **4-4** (2.50 g, 2.82 mmol) in DMF (20 mL). The reaction was stirred overnight, and LCMS analysis showed that the reaction was complete. The reaction mixture was quenched with brine and then extracted three times with dichloromethane (50 mL). The combined organic phases were washed with brine, dried over Na₂SO₄, and concentrated, and the resulting crude product was purified by reversed-phase column chromatography to give compound **4-5** (2.00 g, yield: 82.03%). **LCMS:** MS (ESI) m/z = 865.4 [M+H]⁺.

### Compound I-4

At 0 °C, in a nitrogen atmosphere, a 3A molecular sieve was added to a solution of compound **4-5** (2.00 g, 2.32 mmol) in dry acetonitrile (15 mL). After 5 min of stirring, bis(diisopropylamino)(2-cyanoethoxy)phosphine (1.88 g, 6.25 mmol) and a pre-formulated solution of 1H-tetrazole (0.45 M) and N-methylimidazole (0.18 M) in dry acetonitrile (5.14 mL) were added. Then, the mixture was warmed to room temperature and left to react for 2 h, and LCMS analysis showed that the reaction was complete. A 5% aqueous sodium bicarbonate solution (40 mL) was added to the reaction mixture, and the mixture was shaken for 1 min to quench the reaction. Then, extraction was performed twice with dichloromethane (50 mL). The combined organic phases were washed with saturated brine, dried over sodium sulfate, filtered, and concentrated to give a crude product of compound **I-4** (3.03 g), and the crude product was directly used in the subsequent solid-phase synthesis. LCMS: MS (ESI) m/z = 1109.1 [M+46]⁺. ³¹P-NMR (162 MHz, DMSO) δ 146.28.

### Example 5: Synthesis of Compound I-5

Unless otherwise specified, all reagents used in the following examples were commercially available. The synthesis scheme for compound **I-5** is as follows:

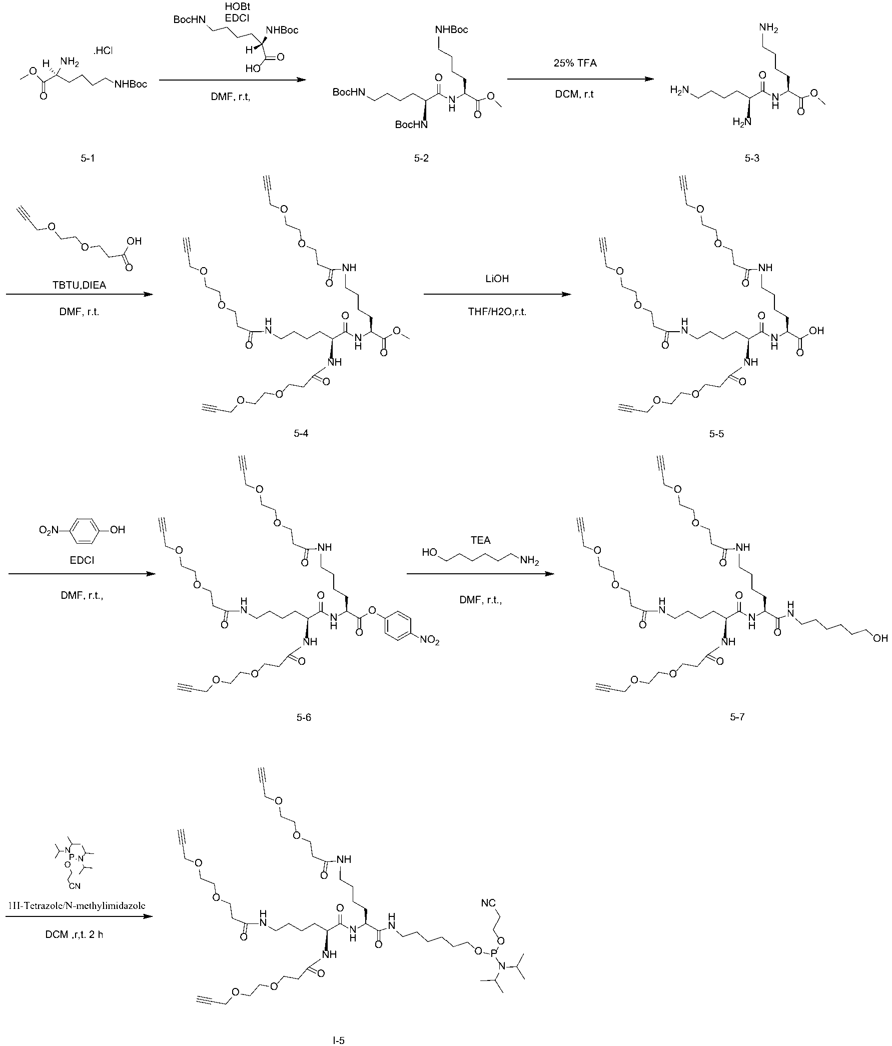

The synthesis and identification of specific intermediates and final products involved in the above scheme are as follows:

### Compound 5-2:

Compound **5-1** (3.00 g, 8.23 mmol) was dissolved in anhydrous DMF (30 mL), and the compounds *N*-Boc-L-lysine methyl ester hydrochloride (2.36 g, 9.05 mmol), DIEA (4.1 mL, 24.68 mmol), EDC· HCl (1.89 g, 9.87 mmol), and HOBt (1.33 g, 9.87 mmol) were sequentially added to the solution while the solution was stirred. The mixture was stirred at room temperature for 3 h, and LCMS analysis showed that the reaction was complete. The reaction mixture was diluted with ethyl acetate, washed twice with a 5% aqueous citric acid solution, washed twice with a saturated sodium bicarbonate solution, and washed with saturated brine. The organic phase was dried over Na₂SO₄, filtered, and concentrated to give a crude product of compound **5-2** (4.50 g, yield: 92.91%), and the crude product was directly used in the next step. LCMS: MS (ESI) m/z = 589.9 [M+H]⁺.

### Compound 5-3:

Compound **5-2** (4.5 g, 7.64 mmol) was dissolved in DCM (18 mL), and trifluoroacetic acid (9 mL) was added to the solution. The mixture was stirred at room temperature overnight, and LCMS monitoring showed that the reaction was complete. The reaction mixture was concentrated to dryness by rotary evaporation to give a crude product of the trifluoroacetate of compound **5-3** (8 g), and the crude product was directly used in the next step. LCMS: MS (ESI) m/z = 289.5 [M+H]⁺.

### Compound 5-4:

Compound **5-3** (7.70 g, 7.92 mmol) was dissolved in a solution of anhydrous DCM (25 mL), and DIPEA (15.7 mL, 95 mmol) and TBTU (10.17 g, 31.67 mmol) were sequentially added to the solution under ice-bath conditions in a nitrogen atmosphere. After the mixture was slowly warmed to room temperature and left to react for 30 min, the compound propargyl-PEG2-acid (5.45 g, 31.67 mmol) was added. The reaction mixture was stirred at room temperature for 1 h, and LCMS analysis showed that the starting materials disappeared. After the reaction mixture was quenched with water, the organic phase was separated, washed with saturated brine, dried over Na₂SO₄, and concentrated, and the resulting crude product was purified by reversed-phase column chromatography to give compound **5-4** (4.00 g, yield: 67.28%). LCMS: MS (ESI) m/z = 752.0 [M+H]⁺.

### Compound 5-5:

Compound **5-4** (3.72 g, 4.93 mmol) was dissolved in a solution of THF/H₂O (4:1, 35 mL), and lithium hydroxide monohydrate (0.39 g, 9.36 mmol) was added to the solution. The mixture was left to react at room temperature for 30 min, and LCMS analysis showed that the starting materials disappeared. After the reaction was complete, 1 M hydrochloric acid was added to adjust the pH to 5-6, and extraction was performed with ethyl acetate. The organic phases were combined, dried over anhydrous sodium sulfate, filtered, and concentrated to give compound **5-5** (3.59 g, yield: 99.15%). LCMS: MS (ESI) m/z = 738.0 [M+H]⁺.

### Compound 5-6:

At 0 °C, 4-nitrophenol (1.02 g, 7.33 mmol) and EDCI (1.4 g, 7.33 mmol) were added to a solution of compound **5-5** (3.55 g, 4.89 mmol) in DMF. The mixture was warmed to room temperature and then left to react for another hour, and LCMS analysis showed that the reaction was complete. The reaction mixture was quenched with water and then extracted three times with ethyl acetate (40 mL). The combined organic phases were washed with brine, dried over Na₂SO₄, and concentrated, and the resulting crude product was purified by reversed-phase column chromatography to give compound **5-6** (3.68 g, yield: 88.27%). LCMS: MS (ESI) m/z = 859.3 [M+H]⁺.

### Compound 5-7:

The compounds 6-amino-1-hexanol (0.61 g, 5.18 mmol) and Et₃N (1.2 mL, 8.63 mmol) were added to a solution of compound **5-6** (3.65 g, 4.31 mmol) in DCM (10 mL). The mixture was left to react at room temperature for 10 min, and LCMS analysis showed that the reaction was complete. After the reaction mixture was quenched with brine, the organic phase was dried over Na₂SO₄ and concentrated to dryness by rotary evaporation, and the resulting crude product was purified by reversed-phase column chromatography to give compound **5-7** (2.00 g, yield: 55.4%). LCMS: MS (ESI) m/z = 837.3 [M+H]⁺.

### Compound I-5:

At 0 °C, in a nitrogen atmosphere, a 3A molecular sieve was added to a solution of compound **5-7** (1.35 g, 1.62 mmol) in dry acetonitrile (10 mL). After 5 min of stirring, bis(diisopropylamino)(2-cyanoethoxy)phosphine (1.07 g, 3.55 mmol) and a pre-formulated solution of 1H-tetrazole (0.45 M) and N-methylimidazole (0.18 M) in dry acetonitrile (4.3 mL) were added. Then, the mixture was warmed to room temperature and left to react for 2 h, and LCMS analysis showed that the reaction was complete. A 5% aqueous sodium bicarbonate solution (40 mL) was added to the reaction mixture, and the mixture was shaken for 1 min to quench the reaction. Then, extraction was performed twice with dichloromethane (50 mL). The combined organic phases were washed with saturated brine, dried over sodium sulfate, filtered, and concentrated to give a crude product of compound **I-5** (2.10 g), and the crude product was directly used in the subsequent solid-phase synthesis. LCMS: MS (ESI) m/z = 1058.98 [M+Na]⁺. ³¹PNMR (400 MHz, DMSO) δ 146.28.

### Example 6: Synthesis of Compound III-f'

The synthesis scheme for compound **III-f'** is as follows:

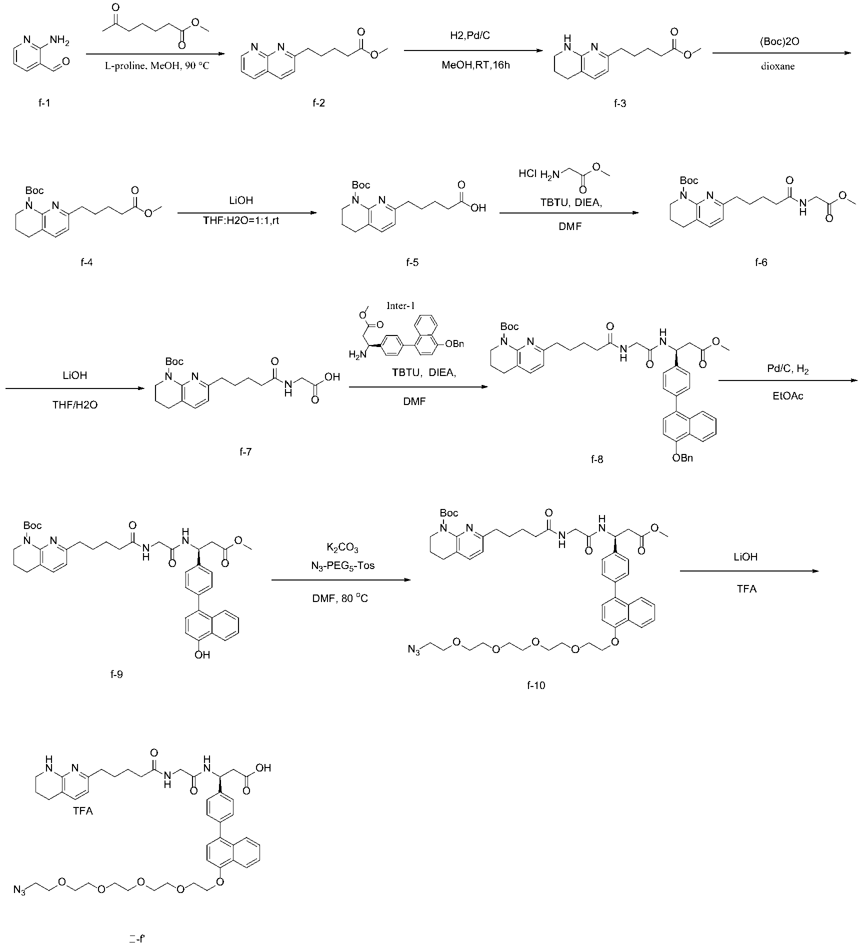

The synthesis and identification of specific intermediates and final products involved in the above scheme are as follows:

### Compound f-2:

Methyl 6-oxoheptanoate (1.00 g, 6.32 mmol) and L-proline (0.36 g, 3.16 mmol) were sequentially added to a solution of compound **f-1** (0.77 g, 6.32 mmol) in methanol (10 mL). The mixture was heated at reflux overnight, and LCMS analysis showed that the reaction was complete. The reaction mixture was cooled to room temperature and then concentrated to dryness by rotary evaporation, and the resulting crude product was purified by normal-phase silica gel column chromatography to give compound **f-2** (720 mg, yield: 46.6%). **LCMS:** MS (ESI) m/z = 245.1 [M+Na]⁺.

### Compound f-3:

Palladium on carbon (42 mg) was added to a solution of compound **f-2** (412 mg, 1.69 mmol) in methanol (10 mL). The mixture was stirred at room temperature overnight in a hydrogen atmosphere, and LCMS monitoring showed that the reaction was complete. The reaction mixture was filtered through diatomaceous earth, and the filtrate was concentrated to dryness by rotary evaporation to give compound **f-3** (410 mg, yield: 97.9%). **LCMS:** MS (ESI) m/z = 249.2 [M+H]⁺.

### Compound f-4:

Di-tert-butyl dicarbonate (395 mg, 1.81 mmol) was added to a solution of compound **f-3** (300 mg, 1.21 mmol) in 1,4-dioxane (3 mL). The reaction was heated to 90 °C and stirred for 2 h, and LCMS analysis showed that the reaction was complete. The reaction mixture was cooled to room temperature and then concentrated to dryness by rotary evaporation, and the resulting crude product was purified by normal-phase silica gel column chromatography to give compound **f-4** (380 mg, yield: 90.2%). **LCMS:** MS (ESI) m/z = 349.2 [M+H]⁺.

### Compound f-5:

Lithium hydroxide monohydrate (54.9 mg, 1.31 mmol) was added to a mixed solution of compound **f-4** (380 mg, 1.09 mmol) in water (2 mL) and tetrahydrofuran (2 mL). The mixture was left to react at room temperature for 30 min, and LCMS analysis showed that the reaction was complete. The reaction mixture was directly concentrated to dryness by rotary evaporation to give a crude product of compound **f-5,** and the crude product was directly used in the next step. **LCMS:** MS (ESI) m/z = 335.3 [M+H]⁺.

### Compound f-6:

Glycine methyl ester hemihydrochloride (180 mg, 1.44 mmol), O-(benzotriazol-1-yl)-N,N,N',N'-tetramethyluronium tetrafluoroborate (461 mg, 1.44 mmol), and N,N-diisopropylethylamine (464 mg, 3.59 mmol) were sequentially added to a solution of compound **f-5** (400 mg, 1.2 mmol) in N,N-dimethylformamide (4 mL). The mixture was left to react at room temperature for 30 min, and LCMS analysis showed that the reaction was complete. The reaction mixture was quenched with 100 mL of water and extracted with ethyl acetate (40 mL × 3). The extract was washed with saturated brine, dried over anhydrous sodium sulfate, and concentrated, and the resulting crude product was purified by normal-phase silica gel column chromatography to give compound **f-6** (340 mg, yield: 70.1%). **LCMS:** MS (ESI) m/z = 406.3 [M+H]⁺.

### Compound f-7:

Lithium hydroxide monohydrate (42 mg, 1.00 mmol) was added to a mixed solution of compound **f-6** (340 mg, 0.84 mmol) in water (2 mL) and tetrahydrofuran (2 mL). The mixture was left to react at room temperature for 30 min, and LCMS analysis showed that the reaction was complete. The reaction mixture was directly concentrated to dryness by rotary evaporation to give a crude product of compound **f-7,** and the crude product was directly used in the next step. **LCMS:** MS (ESI) m/z = 392.2 [M+H]⁺.

### Compound f-8:

**Inter-1** (180 mg, 0.4 mmol), O-(benzotriazol-1-yl)-N,N,N',N'-tetramethyluronium tetrafluoroborate (142 mg, 0.44 mmol), and N,N-diisopropylethylamine (156 mg, 1.21 mmol) were sequentially added to a solution of compound **f-7** (173 mg, 0.44 mmol) in N,N-dimethylformamide (2 mL). The mixture was left to react at room temperature for 30 min, and LCMS analysis showed that the reaction was complete. The reaction mixture was quenched with 10 mL of water and extracted with ethyl acetate (40 mL × 3). The extract was washed with saturated brine, dried over anhydrous sodium sulfate, and concentrated, and the resulting crude product was purified by normal-phase silica gel column chromatography to give compound **f-8** (120 mg, yield: 38.1%). **LCMS:** MS (ESI) m/z = 785.4 [M+Na]⁺.

### Compound f-9:

10% palladium on carbon (120 mg) was added to a solution of compound **f-8** (240 mg, 0.31 mmol) in tetrahydrofuran (3 mL) and ethyl acetate (6 mL). The mixture was purged with hydrogen three times and then stirred at room temperature overnight. LCMS analysis showed that the reaction was complete. The reaction mixture was filtered, and the organic phase was concentrated to give compound **f-9** (205 mg, yield: 96.5%) as a white solid product. LCMS: MS (ESI) m/z = 695.4 [M+H]⁺.

### Compound f-10:

At 0 °C, azide-PEG5-Tos (148 mg, 0.35 mmol) and potassium carbonate (82 mg, 0.59 mmol) were added to a solution of compound **f-9** (205 mg, 0.29 mmol) in N,N-dimethylformamide (4 mL). The reaction was stirred at 80 °C for 6 h. After LCMS monitoring showed that the reaction was complete, the reaction mixture was quenched with a saturated sodium bicarbonate solution and extracted with ethyl acetate (40 mL × 3). The extract was washed with saturated brine, dried over anhydrous sodium sulfate, and concentrated, and the resulting crude product was purified by normal-phase silica gel column chromatography (dichloromethane:methanol = 10/1) to give compound **f-10** (240 mg, yield: 86.5%) as a pale yellow oily product. LCMS: MS (ESI) m/z = 962.2 [M+H]⁺.

### Compound III-f':

Lithium hydroxide monohydrate (0.021 mL, 0.76 mmol) was added to a mixed solution of compound **f-10** (240 mg, 0.25 mmol) in water (2 mL) and tetrahydrofuran (2 mL), and the mixture was stirred at room temperature for 2 h. LCMS analysis showed that the reaction was complete. After the reaction mixture was concentrated, dichloromethane (1.5 mL) and trifluoroacetic acid (1.5 mL) were added to the resulting crude product. The mixture was stirred at room temperature overnight, and LCMS analysis showed that the reaction was complete. After concentration, the resulting crude product was purified by preparative high performance liquid chromatography to give compound **III-f'** (140 mg, a yellow solid) as an off-white solid product. LCMS: MS (ESI) m/z = 826.3 [M+H]⁺. ¹H NMR (400 MHz, DMSO) δ 13.15 (s, 1H), 8.49 (d, *J =* 0.8 Hz, 1H), 8.27 (d, *J =* 0.8 Hz, 1H), 8.11 (t, *J =* 0.8 Hz, 1H), 7.77 (1H, s), 7.76 (d, *J =* 0.8 Hz, 1H), 7.53-7.49 (m, 2H), 7.49-7.31 (m, 6H), 7.06 (d, *J =* 1.2 Hz, 1H), 5.35-5.27 (m, 1H), 4.33 (t, *J =* 0.4 Hz, 2H), 3.92 (t, *J* = 0.4 Hz, 2H), 3.76-3.65 (m, 4H), 3.63-3.49 (m, 10H), 3.38-3.29 (m, 6H), 2.78 (d, *J* = 0.8 Hz, 2H), 2.69-2.64 (m, 2H), 2.62-2.57 (m, 2H), 2.17 (t, *J* = 0.8 Hz, 2H), 1.81-1.75 (m, 2H), 1.62-1.50 (m, 4H).

### Example 7: Synthesis of Compound III-g'

The synthesis scheme for compound **III-g'** is as follows:

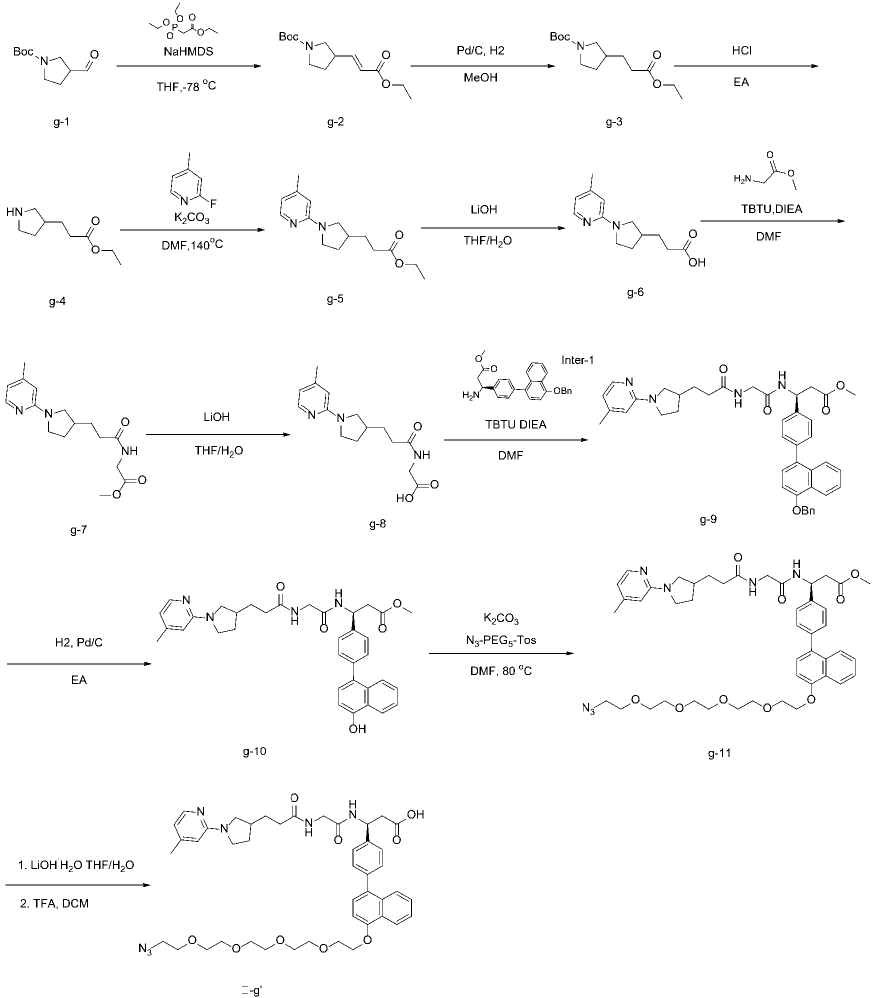

The synthesis and identification of specific intermediates and final products involved in the above scheme are as follows:

### Compound g-2:

At -78 °C, in a nitrogen atmosphere, sodium bis(trimethylsilyl)amide (13.55 mL, 27.11 mmol) was slowly added dropwise to a solution of triethyl phosphorylacetate (6.75 g, 30.11 mmol) in tetrahydrofuran (30 mL). After 30 min of stirring at this temperature, a solution of compound **g-1** (3.00 g, 15.06 mmol) in tetrahydrofuran (10 mL) was added. The mixture was left to react for another 30 min, and LCMS monitoring showed that the reaction was complete. The reaction mixture was quenched with water (100 mL) and extracted with ethyl acetate (40 mL × 3). The extract was washed with saturated brine, dried over anhydrous sodium sulfate, and concentrated, and the resulting crude product was purified by normal-phase silica gel column chromatography to give compound **g-2** (1.85 g, yield: 45.6%). **LCMS:** MS (ESI) m/z = 170.1 [M-100+H]⁺.

### Compound g-3:

10% palladium on carbon (100 mg) was added to a solution of compound **g-2** (1.00 g, 3.71 mmol) in methanol (10 mL), and the mixture was stirred at room temperature overnight in a hydrogen atmosphere. LCMS monitoring showed that the reaction was complete. The reaction mixture was filtered through diatomaceous earth, and the filtrate was concentrated to dryness by rotary evaporation to give compound **g-3** (1.00 g, yield: 99.2%). **LCMS:** MS (ESI) m/z = 172.1 [M-100+H]⁺.

### Compound g-4:

Compound **g-3** (1.00 g, 3.69 mmol) was added to a 4 N solution of hydrogen chloride in ethyl acetate (20 mL). After 1 h of stirring at room temperature, LCMS monitoring showed that the reaction was complete. The reaction mixture was concentrated to dryness by rotary evaporation to give a crude product of compound **g-4** (0.81 g), and the crude product was directly used in the next step. **LCMS:** MS (ESI) m/z = 172.1 [M+H]⁺.

### Compound g-5:

2-Fluoro-4-methylpyridine (401 mg, 3.61 mmol) and potassium carbonate (665 mg, 4.82 mmol) were sequentially added to a solution of compound **g-4** (500 mg, 2.41 mmol) in dimethyl sulfoxide (5 mL). The mixture was left to react in a microwave reactor at 140 °C for 1.5 h, and LCMS monitoring showed that the reaction was complete. The reaction mixture was quenched with water and extracted with ethyl acetate (40 mL × 3). The extract was washed with saturated brine, dried over anhydrous sodium sulfate, and concentrated, and the resulting crude product was purified by normal-phase silica gel column chromatography to give compound **g-5** (240 mg, yield: 38.0%). **LCMS:** MS (ESI) m/z = 263.2 [M+H]⁺.

### Compound g-6:

Lithium hydroxide monohydrate (61.4 mg, 1.46 mmol) was added to a mixed solution of compound **g-5** (320 mg, 1.22 mmol) in water (2 mL) and tetrahydrofuran (2 mL). The mixture was left to react at room temperature for 30 min, and LCMS analysis showed that the reaction was complete. The reaction mixture was directly concentrated to dryness by rotary evaporation to give a crude product of compound **g-6** (350 mg), and the crude product was used in the next step. **LCMS:** MS (ESI) m/z = 235.1 [M+H]⁺.

### Compound g-7:

Glycine methyl ester hemihydrochloride (167 mg, 1.33 mmol), O-(benzotriazol-1-yl)-N,N,N',N'-tetramethyluronium tetrafluoroborate (428 mg, 1.33 mmol), and N,N-diisopropylethylamine (430 mg, 3.33 mmol) were sequentially added to a solution of compound **g-6** (260 mg, 1.11 mmol) in N,N-dimethylformamide (3 mL). After the mixture was left to react at room temperature for 30 min, LCMS analysis showed that the reaction was complete. The reaction mixture was quenched with water and extracted with ethyl acetate (40 mL × 3). The extract was washed with saturated brine, dried over anhydrous sodium sulfate, and concentrated, and the resulting crude product was purified by normal-phase silica gel column chromatography to give compound **g-7** (240 mg, yield: 70.8%). **LCMS:** MS (ESI) m/z = 306.2 [M+H]⁺.

### Compound g-8:

Lithium hydroxide monohydrate (40 mg, 0.94 mmol) was added to a mixed solution of compound **g-7** (240 mg, 0.79 mmol) in water (1.5 mL) and tetrahydrofuran (1.5 mL). The mixture was left to react at room temperature for 30 min, and LCMS analysis showed that the reaction was complete. The reaction mixture was directly purified by reversed-phase column chromatography to give compound **g-8** (140 mg, yield: 61.1%). **LCMS:** MS (ESI) m/z = 292.2 [M+H]⁺.

### Compound g-9:

At room temperature, N,N-diisopropylethylamine (0.39 mL, 2.36 mmol), O-(7-azabenzotriazol-1-yl)-N,N,N',N'-tetramethyluronium hexafluorophosphate (300 mg, 0.94 mmol), and compound **Inter-1** (370 mg, 0.91 mmol) were added to a solution of compound **g-8** (220 mg, 0.79 mmol) in N,N-dimethylformamide (5 mL). The reaction mixture was stirred for 2 h, and LCMS showed that the reaction was complete. Water was added dropwise to the reaction, and extraction was performed with ethyl acetate (40 mL × 3). The extract was washed with saturated brine, dried over anhydrous sodium sulfate, and concentrated, and the resulting crude product was purified by normal-phase silica gel column chromatography (dichloromethane:methanol = 20/1) to give compound **g-9** (120 mg, yield: 22.2%). LCMS (ESI) found: 685.4 [M+H]⁺.

### Compound g-10:

Palladium on carbon (100 mg) was added to a solution of compound **g-9** (120 mg, 0.17 mmol) in ethyl acetate (3 mL). The system was purged three times with hydrogen and stirred at room temperature overnight. LCMS showed that the reaction was complete. The reaction mixture was filtered through diatomaceous earth and concentrated to give compound **g-10** (70 mg, yield: 61.7%) as a yellowish-white solid. LCMS (ESI) found: 595.4 [M+H]⁺.

### Compound g-11:

Azide-PEG5-Tos (64 mg, 0.15 mmol) and potassium carbonate (33 mg, 0.24 mmol) were added to a solution of compound **g-10** (70 mg, 0.12 mmol) in N,N-dimethylformamide (2 mL), and the reaction was stirred at 80 °C overnight. After LCMS showed that the reaction was complete, a saturated sodium bicarbonate solution was added to quench the reaction. The reaction mixture was extracted with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 1/1) to give compound **g-11** (46 mg, yield: 46.5%) as a yellow solid. LCMS (ESI) found: 840.3 [M+H]⁺.

### Compound III-g':

Compound **g-11** (45 mg, 0.05 mmol) was dissolved in a mixed solution of tetrahydrofuran (1 mL) and water (1 mL), and lithium hydroxide monohydrate (7 mg, 0.16 mmol) was added. The mixture was stirred at room temperature for 2 h. LCMS analysis showed that the reaction was complete. The pH of the reaction mixture was adjusted to 2 with 1 N hydrochloric acid, and extraction was performed with ethyl acetate (40 mL × 3). The extract was washed with saturated brine, dried over anhydrous sodium sulfate, and concentrated, and the resulting crude product was purified by preparative high performance liquid chromatography to give compound **III-g'** (35 mg, a yellow solid) as a white solid. LCMS: MS (ESI) m/z = 826.4 [M+H]⁺. ¹H NMR (400 MHz, DMSO) δ 8.72 (d, *J =* 0.8 Hz, 1H), 8.37 (s, 1H), 8.26 (d, *J =* 0.8 Hz, 1H), 8.17 (t, *J =* 0.8 Hz, 1H), 7.82 (d, *J* = 0.8 Hz, 1H), 7.77 (d, *J* = 0.8 Hz, 1H), 7.53-7.49 (m, 2H), 7.49-7.45 (m, 2H), 7.45-7.37 (m, 2H), 7.31-7.28 (m, 1H), 7.03 (d, *J* = 0.8 Hz, 1H), 6.34 (s, 1H), 6.21 (s, 1H), 5.26 (dd, *J* = 1.2, 0.8 Hz, 1H), 4.35-4.31 (m, 2H), 3.93-3.85 (m, 2H), 3.76-3.73 (m, 2H), 3.69-3.67 (m, 2H), 3.61-3.52 (m, 10H), 3.49-3.41 (m, 2H), 3.36 (d, *J* = 0.8 Hz, 2H), 3.28-3.23 (m, 2H), 2.91 (dd, *J =* 0.8, 0.4 Hz, 1H), 2.76-2.69 (m, 2H), 2.28-2.21 (m, 2H), 2.17 (s, 3H), 2.14-2.08 (m, 1H), 1.69-1.63 (m, 2H), 1.61-1.53 (m, 1H).

### Example 8: Synthesis of Compound III-h'

The synthesis scheme for compound **III-h'** is as follows:
1) 4-nitrophenyl chloroformate,

### DIEA, DCM

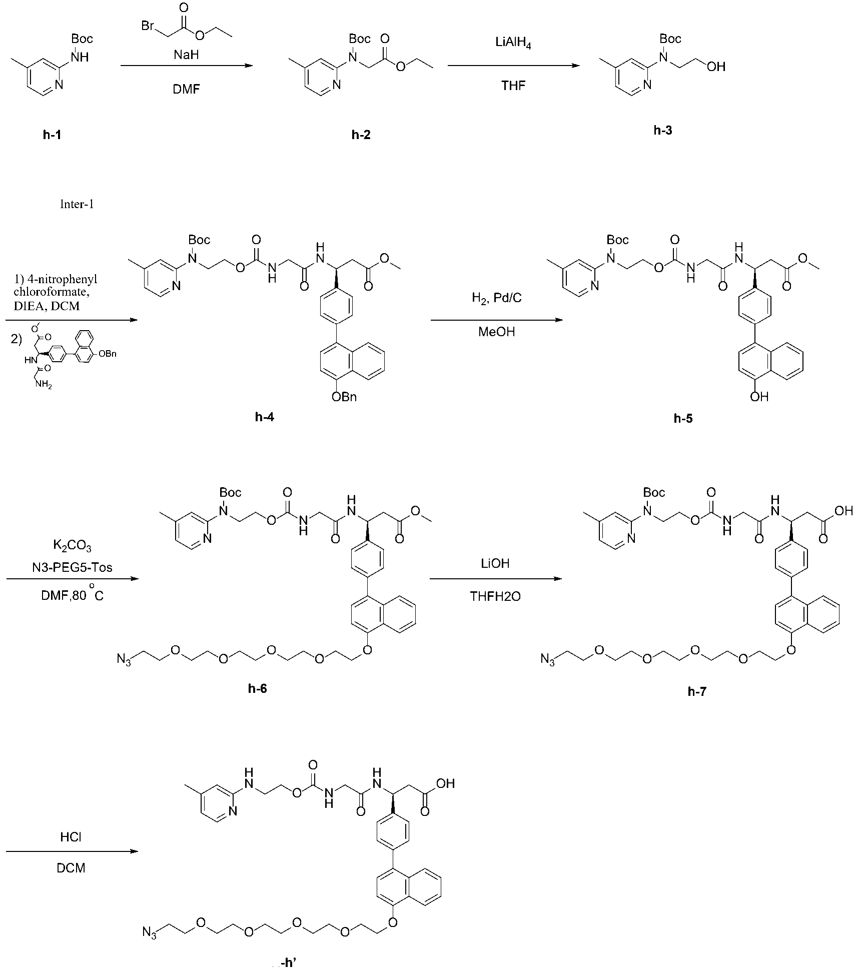

The synthesis and identification of specific intermediates and final products involved in the above scheme are as follows:

### Compound h-2:

Compound **h-1** (1.30 g, 6.24 mmol) was added to N,N-dimethylformamide (10 mL), and sodium hydride (324.59 mg, 8.11 mmol) was slowly added at 0 °C in a nitrogen atmosphere. After 30 min of stirring, ethyl bromoacetate (0.83 mL, 7.49 mmol) was added with the temperature maintained at 0 °C, and the mixture was stirred at room temperature overnight. LCMS analysis showed the reaction had been complete. After water was added dropwise to quench the reaction, extraction was performed with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 5/1) to give compound **h-2** (1.50 g, yield: 81.64%) as a colorless oily liquid.LCMS (ESI) found: 295.4 [M+H]⁺. ¹H NMR (400 MHz, CDCl₃) δ 8.17 (d, *J =* 5.0 Hz, 1H), 7.70 (s, 1H), 6.84 (d, *J =* 5.0 Hz, 1H), 4.71 (s, 2H), 4.22 (q, *J =* 7.0 Hz, 2H), 2.36 (s, 3H), 1.54 (s, 9H), 1.28 (t, *J =* 7.0 Hz, 3H).

### Compound h-3:

In a N₂ atmosphere, 1 N lithium aluminum hydride (10.2 mL, 10.20 mmol) was added to a solution of compound **h-2** (1.50 g, 5.10 mmol) in tetrahydrofuran (20 mL). The reaction mixture was stirred at room temperature for 1 h, and LCMS showed that the reaction was complete. Water was added dropwise to the reaction, and extraction was performed with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 5:1) to give compound **h-3** (400 mg, yield: 31.1%) as a colorless oily liquid.LCMS (ESI) found: 253.4 [M+H]⁺.

### Compound h-4:

Compound **h-3** (200 mg, 0.80 mmol) was dissolved in dichloromethane (5 mL), and N,N-diisopropylethylamine (0.33 mL, 1.98 mmol) and 4-nitrophenyl chloroformate (159.77 mg, 0.80 mmol) were added at room temperature. The reaction mixture was stirred at room temperature for 3 h. LCMS analysis showed the reaction had been complete. The reaction mixture was washed with saturated brine, dried over anhydrous sodium sulfate, and concentrated. The resulting intermediate was redissolved in dichloromethane (4 mL), and triethylamine (0.5 mL, 3.57 mmol) and compound **Inter-1** (371.41 mg, 0.80 mmol) were sequentially added at room temperature. The reaction mixture was stirred at room temperature for another 2 h, and LCMS showed that the reaction was complete. The reaction mixture was washed with saturated brine, dried over anhydrous sodium sulfate, and concentrated, and the resulting crude product was purified by normal-phase silica gel column chromatography (petroleum ether:ethyl acetate = 1/1) to give compound **h-4** (180 mg, yield: 30.4%) as a yellow solid. LCMS (ESI) found: 747.6 [M+H]⁺.

### Compound h-5:

At room temperature, 10% palladium on carbon (50 mg) was added to a solution of compound **h-4** (150 mg, 0.20 mmol) in methanol (30 mL). The system was purged three times with hydrogen and stirred at room temperature overnight. LCMS analysis showed the reaction had been complete. The reaction mixture was filtered through diatomaceous earth and concentrated to give compound **h-5** (130 mg, yield: 98.5%) as a yellowish-white solid. LCMS (ESI) found: 657.2 [M+H]⁺.

### Compound h-6:

Azide-PEG5-Tos (152.56 mg, 0.37 mmol) and potassium carbonate (50.51 mg, 0.37 mmol) were added to a solution of compound **h-5** (120 mg, 0.183 mmol) in N,N-dimethylformamide (5 mL), and the reaction was stirred at 80 °C overnight. LCMS analysis showed the reaction had been complete. The reaction mixture was quenched with a saturated sodium bicarbonate solution and extracted with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 1:1) to give compound h-6 (113 mg, yield: 68.5%) as a yellow solid. LCMS (ESI) found: 902.2 [M+H]⁺.

### Compound h-7:

At room temperature, 1 N lithium hydroxide monohydrate (5 mL, 5.0 mmol) was added to a solution of compound **h-6** (100 mg, 0.11 mmol) in tetrahydrofuran (5 mL). After the reaction was stirred for 2 h, LCMS showed that the reaction was complete. The pH of the reaction mixture was adjusted to 2 with 1 N hydrochloric acid, and extraction was performed with ethyl acetate (40 mL × 3). The extract was washed with saturated brine, dried over anhydrous sodium sulfate, and concentrated to give compound **h-7** (95 mg, yield: 96.5%) as a yellow solid. LCMS (ESI) found: 888.1 [M+H]⁺.

### Compound III-h':

At room temperature, a 4 M solution of hydrochloric acid in 1,4-dioxane (1 mL) was added to a solution of compound **h-7** (100 mg, 0.11 mmol) in dichloromethane (2 mL). The reaction mixture was stirred at room temperature for 3 h. LCMS analysis showed the reaction had been complete. The reaction mixture was concentrated, and the resulting crude product was purified by preparative high performance liquid chromatography to give compound **III-h'** (53.6 mg, yield: 60.4%) as a yellow solid. LCMS (ESI) found: 788.5 [M+H]⁺. ¹H NMR (400 MHz, DMSO) δ 8.52 (d, *J* = 8.2 Hz, 1H), 8.36 - 8.16 (m, 1H), 7.79 (dd, *J* = 18.4, 6.7 Hz, 2H), 7.56-7.29 (m, 7H), 7.05 (d, *J =* 8.0 Hz, 1H), 6.63 (s, 1H), 6.56 (d, *J* = 5.9 Hz, 1H), 5.29 (dd, *J* = 15.0, 7.3 Hz, 1H), 4.40-4.30 (m, 2H), 4.12 (t, *J* = 5.6 Hz, 2H), 3.96-3.90 (m, 2H), 3.72-3.64 (m, 9H), 3.59 (dd, *J* = 11.3, 6.1 Hz, 8H), 3.40-3.35 (m, 6H), 2.79 (d, *J* = 7.1 Hz, 2H), 2.22 (d, *J =* 14.6 Hz, 3H).

### Example 9: Synthesis of Compound III-i'

The synthesis scheme for compound **III-i'** is as follows:

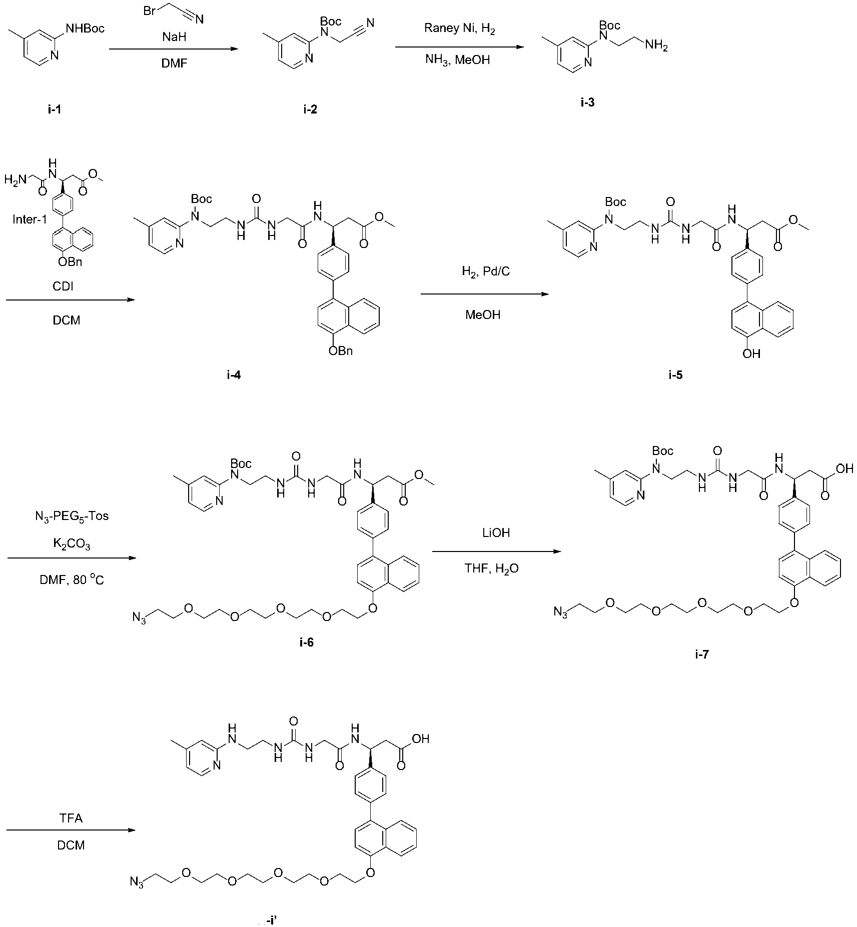

The synthesis and identification of specific intermediates and final products involved in the above scheme are as follows:

### Compound i-2:

At 0 °C, sodium hydride (0.29 g, 7.20 mmol) was added to a solution of compound **i-1** (1.00 g, 4.802 mmol) in N,N-dimethylformamide (20 mL). After the reaction mixture was stirred at 0 °C for 30 min, bromoacetonitrile (0.40 mL, 5.762 mmol) was added. The mixture was stirred at room temperature for another 3 h. After LCMS monitoring showed that the reaction was complete, the reaction mixture was quenched with water and extracted with ethyl acetate (40 mL × 3). The combined organic phases were washed with saturated brine, dried over sodium sulfate, and concentrated, and the resulting crude product was purified by normal-phase silica gel column chromatography (petroleum ether:ethyl acetate = 5/1) to give compound **i-2** (932 mg, yield: 78.5%) as a yellow oil. LCMS (ESI) found: 231.1 [M+H]⁺. ¹H NMR (400 MHz, CDCl₃) *δ* 8.25 (d, *J =* 5.1 Hz, 1H), 7.67 (s, 1H), 6.92 (d, *J* = 5.1 Hz, 1H), 4.91 (s, 2H), 2.39 (s, 3H), 1.60 (s, 9H).

### Compound i-3:

Ammonia water (3 mL) and Raney nickel were sequentially added to a solution of compound **i-2** (932 mg, 3.77 mmol) in ethanol (30 mL), and the mixture was stirred at room temperature overnight in a hydrogen atmosphere. After LCMS monitoring showed that the reaction was complete, the reaction mixture was filtered and then concentrated to give compound **i-3** (837 mg, yield: 88.4%). LCMS (ESI) found: 252.4 [M+H]⁺.

### Compound i-4:

In a N₂ atmosphere, N,N-carbonyldiimidazole (260 mg, 1.60 mmol) was added to a solution of compound **i-3** (500 mg, 1.07 mmol) in dichloromethane (20 mL). After the reaction was stirred at room temperature for 2.5 h, thin layer chromatography analysis showed that the reaction was complete. A solution of compound **Inter-1** (322 mg, 1.28 mmol) in dichloromethane was then added to the previous reaction mixture, and the mixture was stirred at room temperature overnight. After LCMS monitoring showed that the reaction was complete, the reaction mixture was diluted with saturated brine. The separated organic layer was dried over sodium sulfate and concentrated, and the resulting crude product was purified by normal-phase silica gel column chromatography (petroleum ether:ethyl acetate = 2/3) to give compound **i-4** (471 mg, yield: 59.2%). LCMS (ESI) found: 746.5 [M+H]⁺.

### Compound i-5:

10% palladium on carbon (50 mg, 0.134 mmol) was added to a solution of compound **i-4** (100 mg, 0.13 mmol) in methanol (25 mL), and the reaction was stirred at room temperature overnight in a hydrogen atmosphere. After LCMS monitoring showed that the reaction was complete, the reaction mixture was filtered and then concentrated to give compound **i-5** (85 mg, yield: 96.7%). LCMS (ESI) found: 656.5 [M+H]⁺.

### Compound i-6:

At 0 °C, azide-PEG5-Tos (108 mg, 0.26 mmol) and potassium carbonate (36 mg, 0.26 mmol) were added to a solution of compound **i-5** (85 mg, 0.12 mmol) in N,N-dimethylformamide (10 mL), and the mixture was stirred at 80 °C overnight. LCMS analysis showed the reaction had been complete. The reaction mixture was quenched with a saturated sodium bicarbonate solution and extracted with ethyl acetate (40 mL × 3). The extract was washed with saturated brine and dried over anhydrous sodium sulfate. The solution was concentrated to give a crude product of compound **i-6** (200 mg). LCMS (ESI) found: 901.7 [M+H]⁺.

### Compound i-7:

Lithium hydroxide monohydrate (17 mg, 0.73 mmol) was added to a solution of compound **i-6** (220 mg, 0.24 mmol) in tetrahydrofuran (5 mL) and water (5 mL). The reaction mixture was stirred at room temperature for 1 h, and LCMS showed that the reaction was complete. The pH of the reaction mixture was adjusted to 2 with 1 N hydrochloric acid, and extraction was performed with ethyl acetate (40 mL × 3). The extract was washed with saturated brine, dried over anhydrous sodium sulfate, and concentrated to give compound **i-7** (167 mg, yield: 77.1%). LCMS (ESI) found: 887.7 [M+H]⁺.

### Compound III-i'

Trifluoroacetic acid (0.8 mL) was added to a solution of compound **i-7** (167 mg, 0.19 mmol) in dichloromethane (5 mL). The reaction mixture was stirred at room temperature overnight. After LCMS showed that the reaction was complete, the reaction mixture was concentrated, and the resulting crude product was purified by reversed-phase preparative chromatography to give compound **III-i'** (31.6 mg, yield: 21.3%) as a yellow oil. LCMS (ESI) found: 787.6 [M+H]⁺. ¹H NMR (400 MHz, DMSO) δ 8.50 (d, *J =* 8.3 Hz, 1H), 8.32- 8.18 (m, 1H), 7.77 (dd, *J =* 13.9, 3.7 Hz, 2H), 7.58-7.48 (m, 2H), 7.45 (d, *J =* 8.2 Hz, 2H), 7.39 (d, *J =* 8.1 Hz, 2H), 7.32 (d, *J =* 7.9 Hz, 1H), 7.05 (d, *J =* 8.1 Hz, 1H), 6.47 (s, 2H), 6.31 (d, *J* = 27.3 Hz, 2H), 5.30 (q, *J =* 7.4 Hz, 1H), 4.37-4.29 (m, 2H), 3.97-3.88 (m, 2H), 3.75-3.63 (m, 11H), 3.63-3.57 (m, 9H), 3.25-3.14 (m, 4H), 2.78 (d, *J =* 7.1 Hz, 2H), 2.20 (s, 3H).

### Example 10: Synthesis of Compound III-j'

The synthesis scheme for compound **III-j'** is as follows:

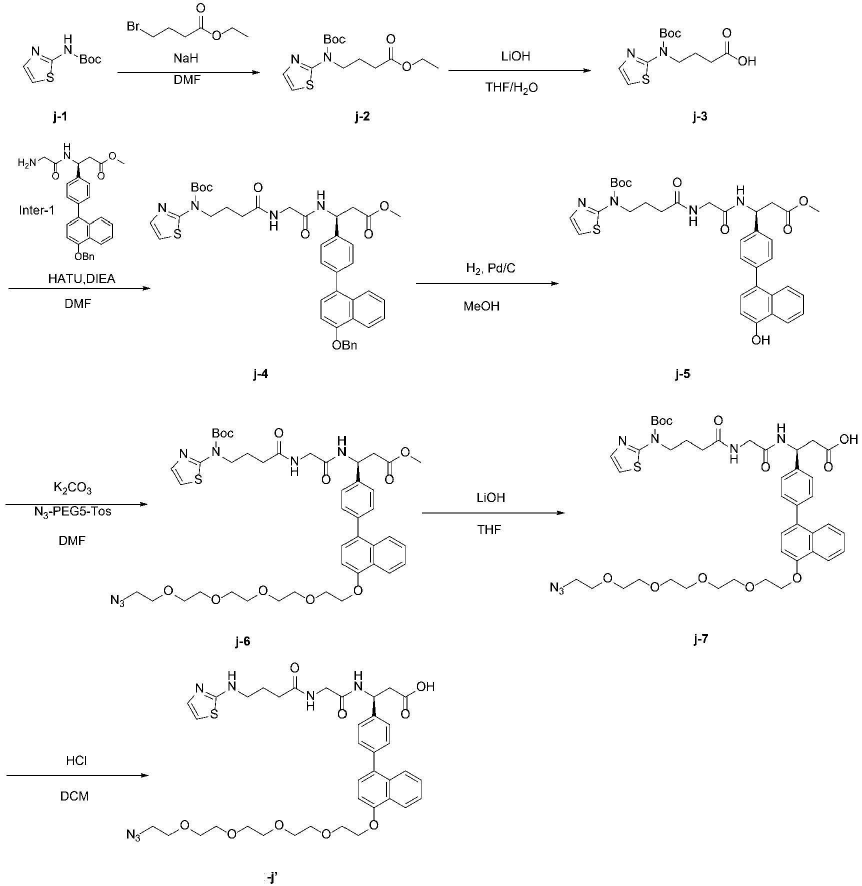

The synthesis and identification of specific intermediates and final products involved in the above scheme are as follows:

### Compound j-2:

At 0 °C, in a nitrogen atmosphere, sodium hydride (0.78 g, 19.48 mmol) was slowly added to a solution of compound **j-1** (1.00 g, 14.98 mmol) in N,N-dimethylformamide (30 mL). After 30 min of stirring, ethyl 4-bromobutyrate (2.92 g, 14.98 mmol) was added, and the mixture was stirred at room temperature overnight. LCMS analysis showed the reaction had been complete. After water was added dropwise to quench the reaction, extraction was performed with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 5/1) to give compound **j-2** (1.50 g, yield: 81.6%) as a colorless oily liquid. LCMS (ESI) found: 315.1 [M+H]⁺. ¹H NMR (400 MHz, CDCl₃) δ 7.39 (d, *J* = 3.6 Hz, 1H), 6.91 (d, *J* = 3.6 Hz, 1H), 4.16 (dd, *J =* 12.5, 5.4 Hz, 2H), 4.10 (t, *J* = 7.1 Hz, 2H), 2.36 (t, *J* = 7.5 Hz, 2H), 2.08- 2.00 (m, 2H), 1.58 (s, 9H), 1.24 (t, *J* = 7.1 Hz, 3H).

### Compound j-3:

At room temperature, 1 N lithium hydroxide monohydrate (5 mL, 5.00 mmol) was added to a solution of compound **j-2** (100 mg, 0.11 mmol) in tetrahydrofuran (5 mL). The mixture was stirred at room temperature for 2 h. After LCMS showed that the reaction was complete, the pH of the reaction system was adjusted to 2 with 1 N HCl, and extraction was performed with ethyl acetate (40 mL × 3). The extract was washed with saturated brine and dried over anhydrous sodium sulfate. The filtrate was concentrated to give compound **j-3** (87 mg, yield: 95.5%) as a yellow solid. LCMS (ESI) found: 287.1 [M+H]⁺.

### Compound j-4:

At room temperature, N,N-diisopropylethylamine (0.32 mL, 1.92 mmol), O-(7-azabenzotriazol-1-yl)-N,N,N',N'-tetramethyluronium hexafluorophosphate (175.28 mg, 0.46 mmol), and compound **Inter-1** (180 mg, 0.38 mmol) were added to a solution of compound **j-3** (110 mg, 0.38 mmol) in N,N-dimethylformamide (5 mL). The reaction mixture was stirred for 2 h, and LCMS showed that the reaction was complete. Water was added dropwise to the reaction, and extraction was performed with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 5/1) to give compound **j-4** (275 mg, yield: 97.1%) as a yellow solid. LCMS (ESI) found: 737.1 [M+H]⁺.

### Compound j-5:

10% palladium on carbon (250 mg) was added to a solution of compound **j-4** (250 mg, 0.34 mmol) in methanol (30 mL). The system was purged three times with hydrogen and stirred at room temperature overnight. LCMS showed that the reaction was complete. The reaction mixture was filtered through diatomaceous earth and concentrated to give compound **j-5** (200 mg, yield: 63.8%) as a yellowish-white solid. LCMS (ESI) found: 647.1 [M+H]⁺.

### Compound j-6:

Azide-PEG5-Tos (193.65 mg, 0.46 mmol) and potassium carbonate (64.11 mg, 0.46 mmol) were added to a solution of compound **j-5** (150 mg, 0.23 mmol) in N,N-dimethylformamide (5 mL), and the reaction was stirred at 80 °C overnight. After LCMS showed that the reaction was complete, a saturated sodium bicarbonate solution was added to quench the reaction. The reaction mixture was extracted with ethyl acetate (40 mL × 3). The extract was washed with saturated brine, dried over anhydrous sodium sulfate, and concentrated, and the resulting crude product was purified by normal-phase silica gel column chromatography (petroleum ether:ethyl acetate = 1/1) to give compound **j-6** (120 mg, yield: 58.0%) as a yellow solid. LCMS (ESI) found: 892.2 [M+H]⁺.

### Compound j-7:

At room temperature, 1 N lithium hydroxide monohydrate (5 mL, 5.00 mmol) was added to a solution of compound **j-6** (70 mg, 0.08 mmol) in tetrahydrofuran (5 mL). After 2 h of stirring at room temperature, LCMS showed that the reaction was complete. The pH of the reaction mixture was adjusted to 2 with 1 N HCl, and extraction was performed with ethyl acetate (40 mL × 3). The extract was washed with saturated brine and dried over anhydrous sodium sulfate. The filtrate was concentrated to give compound **j-7** (65 mg, yield: 94.3%) as a yellow solid. LCMS (ESI) found: 878.1 [M+H]⁺.

### Compound III-j':

A 4 M solution of hydrochloric acid in 1,4-dioxane (1 mL) was added to a solution of compound **j-7** (100 mg, 0.11 mmol) in dichloromethane (2 mL). The reaction mixture was stirred at room temperature for 3 h, and LCMS showed that the reaction was complete. The reaction mixture was concentrated, and the resulting crude product was purified by preparative high performance liquid chromatography to give compound **III-j'** (53.6 mg, yield: 60.4%) as a yellow solid. LCMS (ESI) found: 778.5. ¹H NMR (400 MHz, DMSO) δ 8.97 (s, 1H), 8.50 (t, *J* = 8.8 Hz, 1H), 8.28 (dd, *J* = 8.1, 6.2 Hz, 1H), 8.15 (t, *J* = 5.8 Hz, 1H), 7.81-7.64 (m, 1H), 7.59-7.14 (m, 8H), 7.06 (d, *J =* 8.0 Hz, 1H), 6.81 (d, *J =* 4.0 Hz, 1H), 5.30 (q, *J* = 7.4 Hz, 1H), 4.37-4.27 (m, 2H), 3.96-3.89 (m, 2H), 3.76 (d, *J* = 5.7 Hz, 3H), 3.69 (dd, *J =* 5.7, 3.7 Hz, 3H), 3.60-3.55 (m, 7H), 3.39-3.30 (m, 8H), 2.79 (d, *J* = 7.3 Hz, 2H), 2.24 (t, *J =* 7.3 Hz, 2H), 1.87-1.75 (m, 2H).

### Example 11: Synthesis of Compound III-k'

The synthesis scheme for compound **III-k'** is as follows:

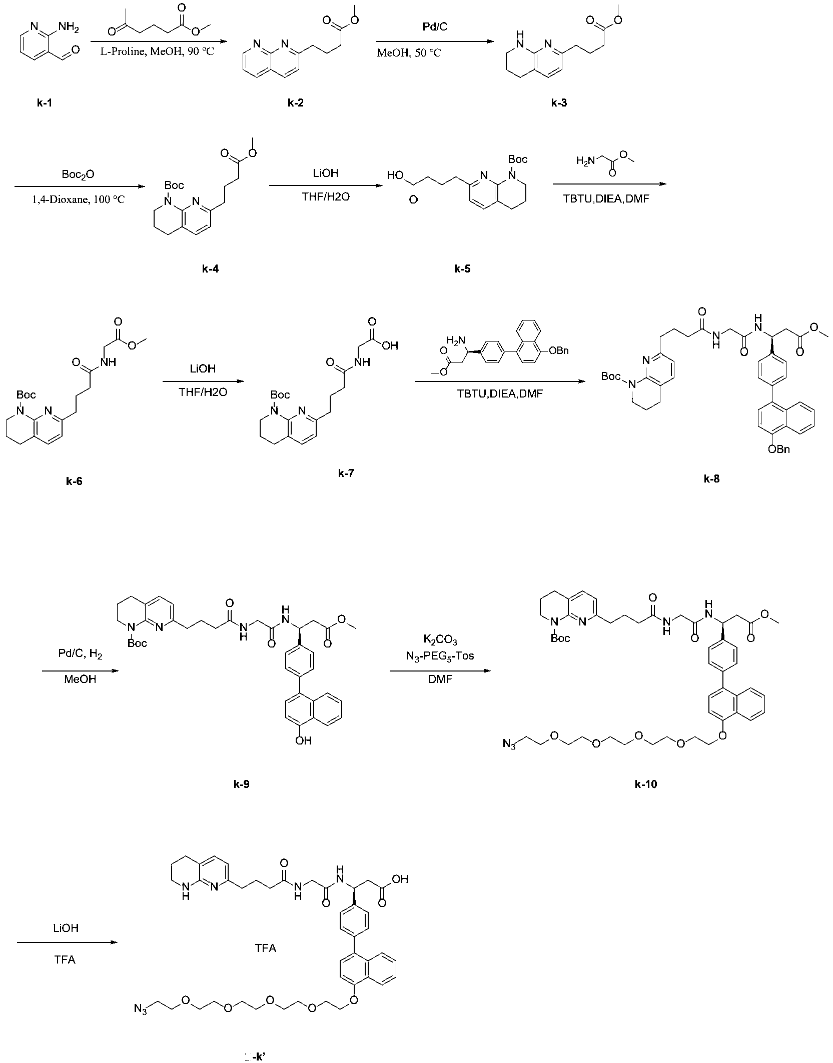

The synthesis and identification of specific intermediates and final products involved in the above scheme are as follows:

### Compound k-2

Methyl 4-acetylbutyrate (2.0 g, 13.89 mmol), L-proline (2.10 g, 18.01 mmol), and concentrated sulfuric acid (0.1 mL) were sequentially added to a solution of compound **k-1** (1.50 g, 12.30 mmol) in methanol (50.0 mL). The reaction mixture was heated at reflux overnight, and LCMS analysis showed that the reaction was complete. After the reaction mixture was concentrated to half of the original volume, water was added to quench the reaction, and extraction was performed with ethyl acetate (50 mL × 3). The combined organic phases were washed with saturated brine, dried over anhydrous sodium sulfate, and concentrated, and the resulting crude product was purified by normal-phase silica gel column chromatography to give compound **k-2** (2.00 g, yield: 71%). LCMS (ESI) found: 231.4 [M+H]⁺.

### Compound k-3

Palladium on carbon (0.20 g) was added to a solution of compound **k-2** (2.00 g, 8.68 mmol) in methanol (30.0 mL) in a hydrogen atmosphere. The reaction was heated to 50 °C and stirred overnight, and LCMS analysis showed that the reaction was complete. The reaction mixture was filtered through diatomaceous earth and concentrated to give compound **k-3** (2.00 g, yield: 98%). LCMS (ESI) found: 235.5 [M+H]⁺.

### Compound k-4

Di-tert-butyl dicarbonate (5.60 g, 25.61 mmol) was added to a solution of compound **k-3** (2.00 g, 8.48 mmol) in 1,4-dioxane (30.0 mL). The reaction was heated at reflux and then stirred overnight, and LCMS analysis showed that the reaction was complete. After the reaction mixture was concentrated to half of the original volume, water was added to quench the reaction, and extraction was performed with ethyl acetate (50 mL × 3). The combined organic phases were washed with saturated brine, dried over anhydrous sodium sulfate, and concentrated, and the resulting crude product was purified by normal-phase silica gel column chromatography to give compound **k-4** (2.00 g, yield: 70%). LCMS (ESI) found: 335.5 [M+H]⁺.

### Compound k-5

Lithium hydroxide monohydrate (0.75 g, 18.02 mmol) was added to a mixed solution of compound **k-4** (2.00 g, 6.01 mmol) in tetrahydrofuran (8.0 mL) and water (2.0 mL), and the mixture was stirred overnight. After LCMS analysis showed that the reaction was complete, the pH of the reaction mixture was adjusted to 5 with 1 N HCl, and extraction was performed with ethyl acetate (50 mL × 3). The combined organic phases were washed with saturated brine, dried over anhydrous sodium sulfate, and concentrated to give compound **k-5** (1.80 g, yield: 94%). LCMS (ESI) found: 321.5 [M+H]⁺.

### Compound k-6

Glycine methyl ester hydrochloride (0.29 g, 2.28 mmol), O-(benzotriazol-1-yl)-N,N,N',N'-tetramethyluronium tetrafluoroborate (0.75 g, 2.31 mmol), and N,N-diisopropylethylamine (1.00 mL, 6.21 mmol) were sequentially added to a solution of compound **k-5** (0.50 g, 1.58 mmol) in N,N-dimethylformamide (10.0 mL). The reaction was stirred at room temperature for 1 h, and LCMS analysis showed that the reaction was complete. After the reaction mixture was concentrated to half of the original volume, water was added to quench the reaction, and extraction was performed with ethyl acetate (50 mL × 3). The combined organic phases were washed with saturated brine, dried over anhydrous sodium sulfate, and concentrated, and the resulting crude product was purified by normal-phase silica gel column chromatography to give compound **k-6** (0.25 mg, yield: 41%). LCMS (ESI) found: 392.5 [M+H]⁺.

### Compound k-7

Lithium hydroxide monohydrate (0.08 g, 1.91 mmol) was added to a mixed solution of compound **k-6** (0.25 g, 0.61 mmol) in tetrahydrofuran (4.0 mL) and water (1.0 mL). The reaction was stirred at room temperature overnight, and LCMS analysis showed that the reaction was complete. The pH of the reaction mixture was adjusted to 5 with 1 N HCl, and extraction was performed with ethyl acetate (50 mL × 3). The combined organic phases were washed with saturated brine, dried over anhydrous sodium sulfate, and concentrated to give compound **k-7** (0.20 g, yield: 83%). LCMS (ESI) found: 378.5 [M+H]⁺.

### Compound k-8

Compound **Inter-1** (0.16 g, 0.41 mmol), N,N-diisopropylethylamine (0.13 mL, 0.81 mmol), and O-(benzotriazol-1-yl)-N,N,N',N'-tetramethyluronium tetrafluoroborate (0.15 g, 0.50 mmol) were sequentially added to a solution of compound **k-7** (0.10 g, 0.27 mmol) in anhydrous N,N-dimethylformamide (2.0 mL). The reaction mixture was stirred at room temperature for 2 h, and LCMS analysis showed that the reaction was complete. The reaction mixture was quenched with a saturated sodium bicarbonate solution and extracted with ethyl acetate (30 mL × 3). The combined organic phases were washed with saturated brine, dried over anhydrous sodium sulfate, and concentrated, and the resulting crude product was purified by normal-phase silica gel column chromatography to give compound **k-8** (0.08 g, yield: 39%). LCMS (ESI) found: 771.6 [M+H]⁺.

### Compound k-9

Palladium on carbon (0.05 g) was added to a mixed solution of compound **k-8** (0.08 g, 0.11 mmol) in tetrahydrofuran (1.5 mL) and ethyl acetate (1.5 mL) in a hydrogen atmosphere. The reaction was stirred at room temperature overnight, and LCMS analysis showed that the reaction was complete. The reaction mixture was filtered through diatomaceous earth and concentrated to give compound **k-9** (0.05 g, yield: 71%). LCMS (ESI) found: 681.6 [M+H]⁺.

### Compound k-10

Azide-PEG5-Tos (0.06 g, 0.11 mmol) and potassium carbonate (0.02 g, 0.11 mmol) were added to a solution of compound **k-9** (0.05 g, 0.10 mmol) in anhydrous N,N-dimethylformamide (3.0 mL). The reaction mixture was heated to 80 °C and stirred for 3 h, and LCMS analysis showed that the reaction was complete. The reaction mixture was quenched with water and extracted with ethyl acetate (20 mL × 3). The combined organic phases were washed with saturated brine, dried over anhydrous sodium sulfate, and concentrated, and the resulting crude product was purified by normal-phase silica gel column chromatography to give compound **k-10** (0.05 g, yield: 73%). LCMS (ESI) found: 926.7 [M+H]⁺.

### Compound III-k'

Lithium hydroxide monohydrate (0.01 g, 0.24 mmol) was added to a mixed solution of compound **k-10** (0.05 g, 0.11 mmol) in tetrahydrofuran (1.6 mL) and water (0.4 mL). The reaction was stirred at room temperature for 1 h, and LCMS analysis showed that the reaction was complete. The pH of the reaction mixture was adjusted to 3 with 1 N HCl, and extraction was performed with ethyl acetate (10 mL × 3). The combined organic phases were washed with saturated brine, dried over anhydrous sodium sulfate, and concentrated. The resulting crude product was redissolved in a solution of dichloromethane (2.0 mL), and trifluoroacetic acid (1 mL) was added. The reaction mixture was stirred at room temperature for 6 h, and LCMS analysis showed that the reaction was complete. The reaction mixture was concentrated, and the resulting crude product was purified by preparative high performance liquid chromatography to give compound **III-k'** (0.02 g, yield: 65%).LCMS (ESI) found: 812.6 [M+H]⁺. ¹H NMR (400 MHz, chloroform-*d*) δ 9.12-.78 (m, 1H), 8.33 (d, *J* = 8.3 Hz, 1H), 8.26 -8.09 (m, 1H), 7.80 (d, *J* = 8.3 Hz, 2H), 7.54-7.32 (m, 6H), 7.23-7.12 (m, 2H), 6.82 (d, *J* = 7.9 Hz, 1H), 6.33 (d, *J* = 7.1 Hz, 1H), 5.59-5.47 (m, 1H), 4.32 (t, *J* = 4.8 Hz, 2H), 4.02 (t, *J* = 4.9 Hz, 2H), 3.84-3.80 (m, 2H), 3.74-3.70 (m, 2H), 3.68-3.60 (m, 10H), 3.35 (t, *J =* 5.1 Hz, 2H), 3.06-2.84 (m, 2H), 2.75-2.59 (m, 4H), 2.11-1.94 (m, 2H), 1.90-1.81 (m, 2H).

### Example 12: Synthesis of Compound III-l'

The synthesis scheme for compound **III-l'** is as follows:

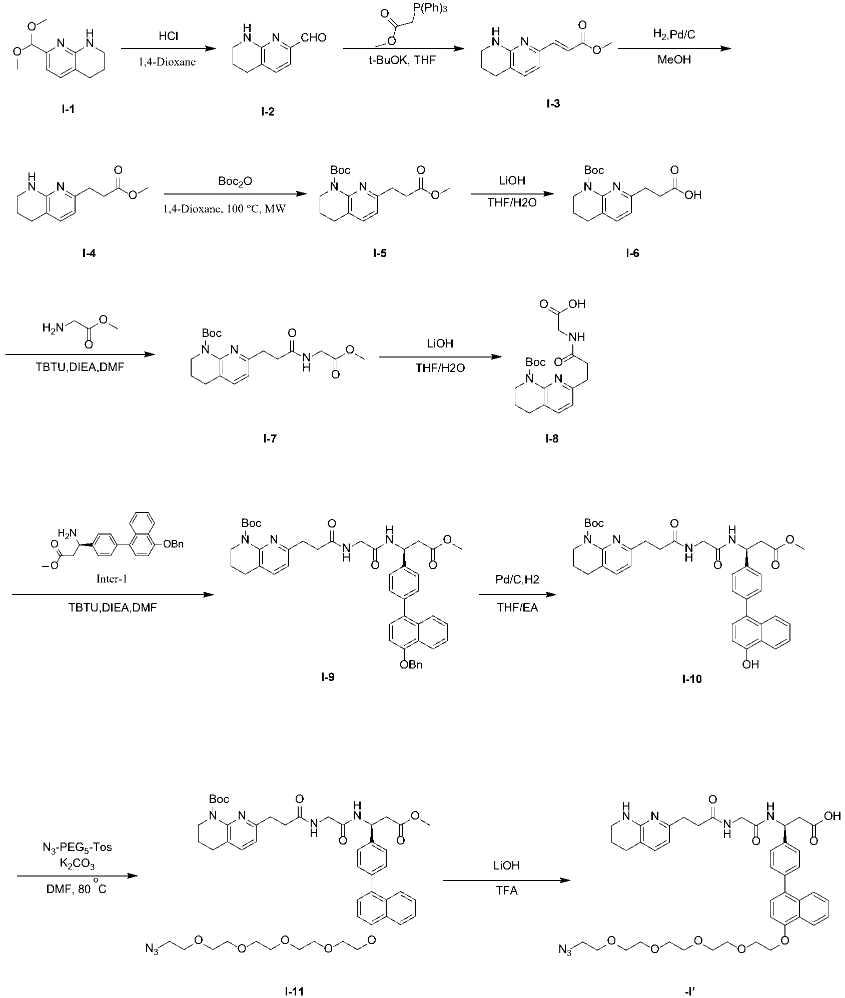

The synthesis and identification of specific intermediates and final products involved in the above scheme are as follows:

### Compound l-2

Compound **l-1** (1.00 g, 4.81 mmol) was dissolved in a solution of hydrochloric acid in 1,4-dioxane (4.0 M, 5.0 mL). The reaction was stirred at room temperature for 2 h, and LCMS monitoring showed that the reaction was complete. The reaction mixture was concentrated to give compound **l-2** (0.70 g, yield: 90%). LCMS (ESI) found: 163.2 [M+H]⁺.

### Compound l-3

(Methoxycarbonylmethyl)triphenylphosphonium bromide (2.20 g, 5.19 mmol) and potassium tert-butoxide (0.70 g, 6.42 mmol) were added to a solution of compound **l-2** (0.70 g, 4.31 mmol) in anhydrous tetrahydrofuran (10.0 mL). The reaction was heated to 50°C and stirred overnight, and LCMS monitoring showed that the reaction was complete.

The reaction mixture was quenched with water and extracted with ethyl acetate (30 mL × 3). The combined organic phases were washed with saturated brine, dried over anhydrous sodium sulfate, and concentrated, and the resulting crude product was purified by normal-phase silica gel column chromatography to give compound **l-3** (0.80 g, yield: 85%). LCMS (ESI) found: 219.4 [M+H]⁺.

### Compound l-4

Palladium on carbon (0.11 g) was added to a solution of compound **l-3** (0.80 g, 3.69 mmol) in tetrahydrofuran (10.0 mL) in a hydrogen atmosphere. The reaction was heated to 50 °C and stirred overnight, and LCMS analysis showed that the reaction was complete. The reaction mixture was filtered through diatomaceous earth and concentrated to give compound **l-4** (0.80 g, yield: 99%). LCMS (ESI) found: 221.4 [M+H]⁺.

### Compound l-5

Di-tert-butyl dicarbonate (1.60 g, 7.32 mmol) was added to a solution of compound **l-4** (0.80 g, 3.59 mmol) in 1,4-dioxane (10.0 mL). The reaction mixture was refluxed and stirred overnight, and LCMS analysis showed that the reaction was complete. After the reaction mixture was concentrated to half of the original volume, water was added to quench the reaction, and extraction was performed with ethyl acetate (50 mL × 3). The combined organic phases were washed with saturated brine, dried over anhydrous sodium sulfate, and concentrated, and the resulting crude product was purified by normal-phase silica gel column chromatography to give compound **l-5** (0.60 g, yield: 52%). LCMS (ESI) found: 321.5 [M+H]⁺.

### Compound l-6

Lithium hydroxide monohydrate (0.19 g, 4.61 mmol) was added to a mixed solution of compound **l-5** (0.50 g, 1.58 mmol) in tetrahydrofuran (4.0 mL) and water (1.0 mL). The reaction was stirred at room temperature overnight, and LCMS analysis showed that the reaction was complete. The pH of the reaction mixture was adjusted to 5 with 1 N HCl, and extraction was performed with ethyl acetate (50 mL × 3). The combined organic phases were washed with saturated brine, dried over anhydrous sodium sulfate, and concentrated to give compound **l-6** (0.40 g, yield: 83%). LCMS (ESI) found: 307.5 [M+H]⁺.

### Compound l-7

Glycine methyl ester hydrochloride (0.12 g, 1.31 mmol), O-(benzotriazol-1-yl)-N,N,N',N'-tetramethyluronium tetrafluoroborate (0.42 g, 1.31 mmol), and N,N-diisopropylethylamine (0.3 mL, 1.91 mmol) were added to a solution of compound **l-6** (0.20 g, 0.61 mmol) in anhydrous N,N-dimethylformamide (3.0 mL). The reaction was stirred at room temperature for 1 h, and LCMS monitoring showed that the reaction was complete. The reaction mixture was quenched with water and extracted with ethyl acetate (30 mL × 3). The combined organic phases were washed with saturated brine, dried over anhydrous sodium sulfate, and concentrated, and the resulting crude product was purified by normal-phase silica gel column chromatography to give compound **l-7** (0.15 g, yield: 61%). LCMS (ESI) found: 378.5 [M+H]⁺.

### Compound l-8

Lithium hydroxide (0.05 g, 1.2 mmol) was added to a mixed solution of compound **l-7** (0.15 g, 0.4 mmol) in tetrahydrofuran (1.6 mL) and water (0.4 mL). The reaction was stirred at room temperature for 1 h, and LCMS analysis showed that the reaction was complete. The pH of the reaction mixture was adjusted to 5 with 1 N HCl, and extraction was performed with ethyl acetate (50 mL × 3). The combined organic phases were washed with saturated brine, dried over anhydrous sodium sulfate, and concentrated to give compound **l-8** (0.10 g, yield: 69%). LCMS (ESI) found: 364.5 [M+H]⁺.

### Compound l-9

Compound **inter-1** (0.14 g, 0.33 mmol), N,N-diisopropylethylamine (0.13 mL, 0.82 mmol), and O-(benzotriazol-1-yl)-N,N,N',N'-tetramethyluronium tetrafluoroborate (0.13 g, 0.41 mmol) were sequentially added to a solution of compound **l-8** (0.10 g, 0.30 mmol) in anhydrous N,N-dimethylformamide (2.0 mL) at 0 °C. The reaction was stirred at room temperature for 2 h, and LCMS analysis showed that the reaction was complete. The reaction mixture was quenched with a saturated aqueous sodium bicarbonate solution and extracted with ethyl acetate (30 mL × 3). The combined organic phases were washed with saturated brine, dried over anhydrous sodium sulfate, and concentrated, and the resulting crude product was purified by normal-phase silica gel column chromatography to give compound **l-9** (0.80 g, yield: 38%). LCMS (ESI) found: 757.6 [M+H]⁺.

### Compound l-10

Palladium on carbon (0.03 g) was added to a solution of compound **l-10** (0.08 g, 0.11 mmol) in tetrahydrofuran (1.5 mL) and ethyl acetate (1.5 mL) in a hydrogen atmosphere. The reaction was heated to 50 °C and stirred overnight, and LCMS analysis showed that the reaction was complete. The reaction mixture was filtered through diatomaceous earth and concentrated to give compound **l-10** (0.05 g, yield: 71%). LCMS (ESI) found: 667.6 [M+H]⁺.

### Compound l-11

Azide-PEG5-Tos (0.05 g, 0.12 mmol) and potassium carbonate (0.02 g, 0.24 mmol) were sequentially added to a solution of compound **l-10** (0.05 g, 0.10 mmol) in anhydrous N,N-dimethylformamide (3.0 mL). The reaction was heated to 80 °C and stirred for 3 h, and LCMS analysis showed that the reaction was complete. The reaction mixture was quenched with water and extracted with ethyl acetate (20 mL × 3). The combined organic phases were washed with saturated brine, dried over anhydrous sodium sulfate, and concentrated, and the resulting crude product was purified by normal-phase silica gel column chromatography to give compound **l-11** (0.05 g, yield: 73%). LCMS (ESI) found: 926.8 [M+H]⁺.

### Compound III-l'

Lithium hydroxide (0.01 g, 0.25 mmol) was added to a mixed solution of compound **l-11** (50 mg, 0.05 mmol) in tetrahydrofuran (1.6 mL) and water (0.4 mL). The reaction was stirred at room temperature for 1 h, and LCMS monitoring showed that the reaction was complete. The pH of the reaction mixture was adjusted to 3 with 1 N HCl, and extraction was performed with ethyl acetate (10 mL × 3). The combined organic phases were washed with saturated brine, dried over anhydrous sodium sulfate, and concentrated. The resulting crude product was redissolved in a solution of dichloromethane (2.0 mL), and trifluoroacetic acid (1.0 mL) was added. The reaction was stirred at room temperature for 6 h, and LCMS analysis showed that the reaction was complete. The reaction mixture was concentrated, and the resulting crude product was purified by preparative high performance liquid chromatography to give compound **III-l'** (20 mg, yield: 45.7%). LCMS (ESI) found: 798.7 [M+H]⁺. ¹H NMR (400 MHz, chloroform-*d*) δ 9.19-8.93 (m, 1H), 8.33 (d, *J =* 8.3 Hz, 1H), 8.26-8.19 (m, 1H), 8.18-8.09 (m, 1H), 7.79 (d, *J =* 8.4 Hz, 1H), 7.52-7.32 (m, 6H), 7.19 (t, *J =* 6.6 Hz, 2H), 6.80 (d, *J* = 7.9 Hz, 1H), 6.36 (d, *J* = 7.2 Hz, 1H), 5.61-5.39 (m, 1H), 4.31 (t, *J* = 4.9 Hz, 2H), 4.01 (t, *J* = 4.8 Hz, 2H), 3.81 (t, *J* = 4.7 Hz, 2H), 3.71 (t, *J =* 4.8 Hz, 2H), 3.69-3.59 (m, 10H), 3.38-3.33 (m, 2H), 3.01-2.92 (m, 2H), 2.70-2.57 (m, 4H), 1.87-1.77 (m, 2H).

### Example 13: Synthesis of Compound III-m'

The synthesis scheme for compound **III-m'** is as follows:

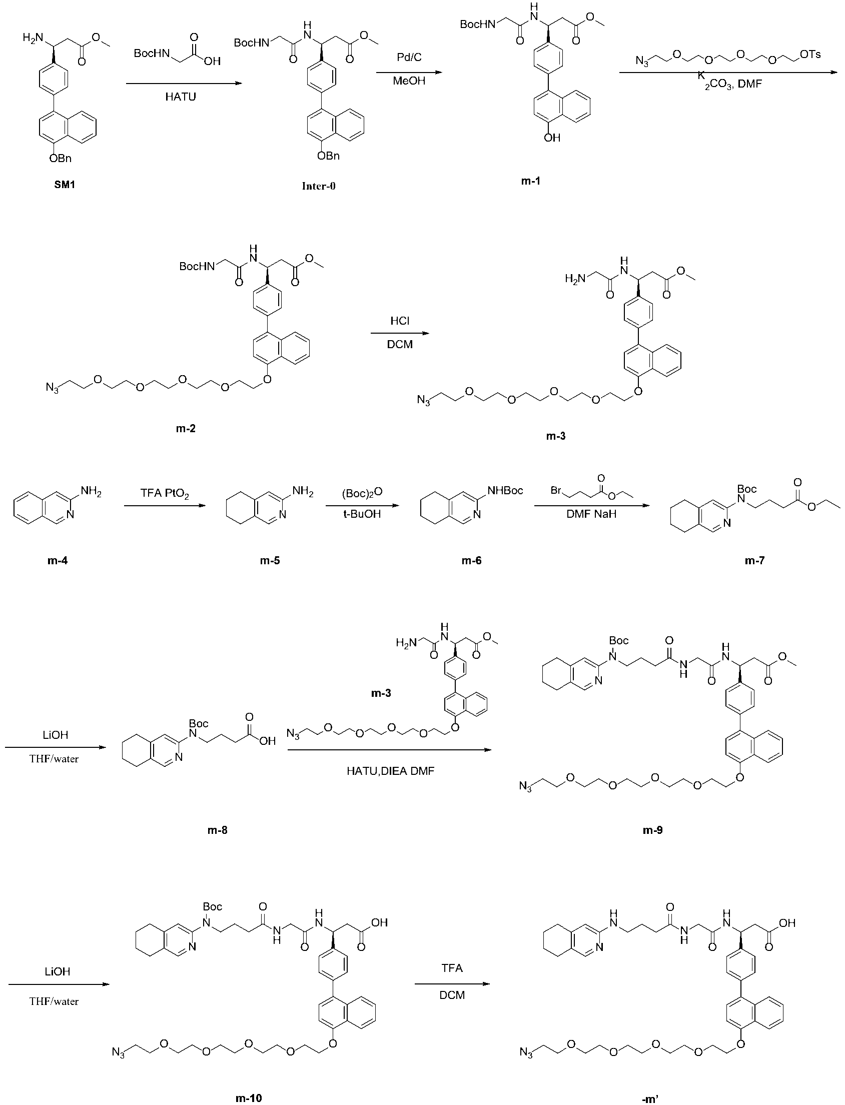

The synthesis and identification of specific intermediates and final products involved in the above scheme are as follows:

### Compound Inter-0

N,N-Diisopropylethylamine (132 mL, 799.2 mmol), O-(7-azabenzotriazol-1-yl)-N,N,N',N'-tetramethyluronium hexafluorophosphate (66.85 g, 175.8 mmol), and compound **SM1** (78.93 g, 191.8 mmol, prepared using the method described in Patent Application No. WO2019089765A1) were added to a solution of the commercially available starting material N-(tert-butoxycarbonyl)glycine (28.00 g, 159.8 mmol) in N,N-dimethylformamide (200 mL), and the mixture was purged 3 times with nitrogen. The reaction was stirred at room temperature for 3 h in a nitrogen atmosphere, and LCMS monitoring showed that the reaction was complete. The reaction mixture was quenched with water and extracted with ethyl acetate. The combined organic phases were washed with saturated brine, dried over anhydrous sodium sulfate, and concentrated, and the resulting crude product was purified by normal-phase silica gel column chromatography to give compound **Inter-0** (60.0 g, yield: 66%). LCMS: MS (ESI) m/z = 513.6 [M+H-56]⁺.

### Compound m-1

10% palladium on carbon (0.23 g) was added to a solution of compound **Inter-0** (2.30 g, 4.05 mmol) in methanol (30.0 mL) in a hydrogen atmosphere. The reaction was stirred at room temperature for 8 h, and LCMS monitoring showed that the reaction was complete. The reaction mixture was filtered and concentrated to give compound **m-1** (1.80 g, yield: 93%). ¹H NMR (400 MHz, DMSO) δ 10.27 (s, 1H), 8.39 (d, *J =* 8.4 Hz, 1H), 8.30-8.09 (m, 1H), 7.75 (dd, *J =* 6.4, 3.2 Hz, 1H), 7.54-7.40 (m, 4H), 7.37 (d, *J =* 8.4 Hz, 2H), 7.23 (d, *J* = 7.6 Hz, 1H), 6.95 (dd, *J* = 14.0, 6.8 Hz, 2H), 5.32 (q, *J* = 7.6 Hz, 1H), 3.60 (s, 3H), 3.57 (d, *J* = 6.0 Hz, 2H), 2.88 (t, *J =* 6.0 Hz, 2H), 1.39 (s, 9H).

### Compound m-2

Azide-PEG5-Tos (1.74 g, 4.18 mmol) and potassium carbonate (0.58 g, 4.18 mmol) were added to a solution of compound **m-1** (1.00 g, 2.09 mmol) in N,N-dimethylformamide (10.0 mL). The reaction was heated to 80 °C and stirred overnight, and LCMS monitoring showed that the reaction was complete. The reaction mixture was quenched with a saturated sodium bicarbonate solution and extracted with ethyl acetate (40 mL × 3). The combined organic phases were washed with saturated brine, dried over anhydrous sodium sulfate, and concentrated, and the resulting crude product was purified by normal-phase silica gel column chromatography to give compound **m-2** (1.30, yield: 85%). LCMS: MS (ESI) m/z = 724.5 [M+H]⁺.

### Compound m-3

A solution of hydrochloric acid in 1,4-dioxane (20.0 mL) was added to a solution of compound **m-2** (1.30 g, 1.79 mmol) in dichloromethane (20.0 mL). The reaction was stirred at room temperature overnight, and LCMS monitoring showed that the reaction was complete. The reaction mixture was concentrated to give compound **m-3** (1.10 g, yield: 98%). LCMS: MS (ESI) m/z = 624.4 [M+H]⁺.

### Compound m-5

Platinum dioxide (50.00 mg) was added to a solution of compound **m-4** (0.50 g, 3.50 mmol) in trifluoroacetic acid (20.0 mL) in a hydrogen atmosphere. The reaction was stirred at room temperature overnight, and LCMS monitoring showed that the reaction was complete. The reaction mixture was filtered through diatomaceous earth and washed with ethyl acetate. After concentration, the resulting solid mixture was dissolved in ethyl acetate. The solution was washed with saturated sodium bicarbonate and saturated brine, dried over anhydrous sodium sulfate, and concentrated. The resulting crude product was redissolved in a mixed solvent of methanol (20.0 mL) and water (20.0 mL), and potassium carbonate was added. The reaction was stirred at 40 °C for 3 h, and LCMS monitoring showed that the reaction was complete. The reaction mixture was diluted with water and extracted with ethyl acetate (50 mL × 3). The combined organic phases were washed with saturated brine, dried over anhydrous sodium sulfate, and concentrated to give compound **m-5** (0.50 g, yield: 97%). LCMS (ESI) found: 149.4 [M+H]⁺. ¹H NMR (400 MHz, DMSO) δ 7.67 (s, 1H), 6.19 (s, 1H), 5.49 (s, 2H), 2.65-2.56 (m, 4H), 1.72 (dt, *J =* 6.4, 3.2 Hz, 4H).

### Compound m-6

Di-tert-butyl dicarbonate (1.10 g, 5.01 mmol) was added to a solution of compound **m-5** (0.50 g, 3.40 mmol) in tert-butanol (20.0 mL). The reaction was stirred at room temperature overnight, and LCMS monitoring showed that the reaction was complete. The reaction mixture was concentrated, and the resulting crude product was purified by normal-phase silica gel column chromatography to give compound **m-6** (0.33 g, yield: 39%). LCMS (ESI) found: 249.4 [M+H]⁺. ¹H NMR (400 MHz, CDCl₃) δ 8.44 (m, 1H), 7.94 (s, 1H), 7.60 (s, 1H), 2.69 (d, *J* = 5.6 Hz, 2H), 2.59 (d, *J* = 5.6 Hz, 2H), 1.75-1.68 (m, 4H), 1.47 (s, 9H).

### Compound m-7

Sodium hydride (0.06 g, 1.61 mmol) was slowly added to a solution of compound **m-6** (0.33 g, 1.31 mmol) in N,N-dimethylformamide (10.0 mL) at 0 °C in a nitrogen atmosphere. After the mixture was stirred for 30 min with the temperature maintained, ethyl 4-bromobutyrate (0.31 g, 1.61 mmol) was added. The reaction was stirred at room temperature overnight, and LCMS monitoring showed that the reaction was complete. The reaction mixture was quenched with water and extracted with ethyl acetate (50 mL × 3). The combined organic phases were washed with saturated brine, dried over anhydrous sodium sulfate, and concentrated, and the resulting crude product was purified by normal-phase silica gel column chromatography to give compound **m-7** (0.35 g, yield: 72%). LCMS (ESI) found: 363.5 [M+H]⁺.

### Compound m-8

A 1 N aqueous lithium hydroxide solution (3.0 mL) was added to a solution of compound **m-7** (0.35 g, 0.91 mmol) in tetrahydrofuran (10.0 mL). The reaction was stirred at room temperature for 1 h, and LCMS analysis showed that the reaction was complete. The pH of the reaction mixture was adjusted to 2 with 1 N hydrochloric acid, and extraction was performed with ethyl acetate (40 mL × 3). The combined organic phases were washed with saturated brine, dried over anhydrous sodium sulfate, and concentrated to give compound **m-8** (0.30 g, yield: 92%). LCMS (ESI) found: 335.4 [M+H]⁺.

### Compound m-9

Compound **m-3** (0.28 g, 0.51 mmol), N,N-diisopropylethylamine (0.29 g, 2.21 mmol), and O-(7-azabenzotriazol-1-yl)-N,N,N',N'-tetramethyluronium hexafluorophosphate (0.20 g, 0.51 mmol) were sequentially added to a solution of compound **m-8** (0.15 g, 0.41 mmol) in N,N-dimethylformamide (5.0 mL). The reaction was stirred at room temperature for 2 h, and LCMS analysis showed that the reaction was complete. The reaction mixture was quenched with water and extracted with ethyl acetate (30 mL × 3). The combined organic phases were washed with saturated brine, dried over anhydrous sodium sulfate, and concentrated, and the resulting crude product was purified by normal-phase silica gel column chromatography to give compound **m-9** (0.40 g, yield: 94%). LCMS (ESI) found: 940.6 [M+H]⁺.

### Compound m-10

A 1 N aqueous lithium hydroxide solution (3.0 mL) was added to a solution of compound **m-9** (0.40 g, 0.38 mmol) in tetrahydrofuran (5.0 mL). The reaction was stirred at room temperature for 2 h, and LCMS analysis showed that the reaction was complete. The pH of the reaction mixture was adjusted to 2 with 1 N hydrochloric acid, and extraction was performed with ethyl acetate (40 mL × 3). The combined organic phases were washed with saturated brine, dried over anhydrous sodium sulfate, and concentrated to give compound **m-10** (0.39 g, yield: 98%). LCMS (ESI) found: 926.6 [M+H]⁺.

### Compound III-m'

Trifluoroacetic acid (3.0 mL) was added to a solution of compound **m-10** (0.39 g, 0.41 mmol) in dichloromethane (5.0 mL). The reaction was stirred at room temperature for 3 h, and LCMS analysis showed that the reaction was complete. The reaction mixture was concentrated, and the resulting crude product was purified by preparative high performance liquid chromatography to give compound **III-m'** (0.20 g, yield: 58%). LCMS (ESI) found: 826.7 [M+H]⁺. ¹H NMR (400 MHz, DMSO) *δ* 13.22 (s, 1H), 8.55 (d, *J =* 8.4 Hz, 1H), 8.35 (s, 1H), 8.30-8.25 (m, 1H), 8.20 (t, *J=* 5.6 Hz, 1H), 7.76 (dd, *J* = 7.6, 1.7 Hz, 1H), 7.66 (s, 1H), 7.56-7.48 (m, 2H), 7.46 (d, *J* = 8.4 Hz, 2H), 7.39 (d, *J* = 8.4 Hz, 2H), 7.32 (d, *J* = 8.0 Hz, 1H), 7.05 (d, *J* = 8.0 Hz, 1H), 6.77 (s, 1H), 5.31 (q, *J* = 7.2 Hz, 1H), 4.34-4.31 (m, 2H), 3.95-3.91 (m, 2H), 3.77 (d, *J* = 5.6 Hz, 2H), 3.69 (dd, *J* = 6.0, 3.6 Hz, 2H), 3.61-3.51 (m, 12H), 3.39-3.35 (m, 2H), 3.28 (dd, *J* = 12.0, 6.4 Hz, 2H), 2.80 (d*, J =* 7.2 Hz, 2H), 2.73 (s, 2H), 2.58 (s, 2H), 2.26 (t, *J =* 7.2 Hz, 2H), 1.81 (dd, *J =* 14.0, 7.2 Hz, 2H), 1.66 (t, *J* = 3.2 Hz, 4H).

### Example 14: Synthesis of Compound III-n'

The synthesis scheme for compound **III-n'** is as follows:

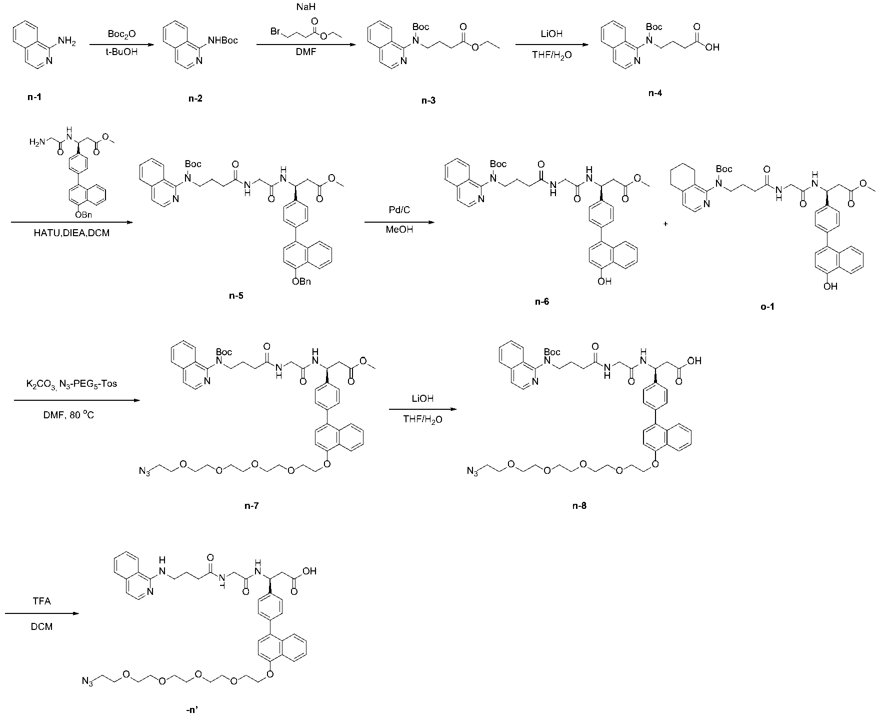

The synthesis and identification of specific intermediates and final products involved in the above scheme are as follows:

### Compound n-2

Di-tert-butyl dicarbonate (7.57 g, 34.68 mmol) was added to a solution of compound **n-1** (4.48 mL, 34.68 mmol) in tert-butanol (40.0 mL). The reaction was stirred at room temperature overnight, and LCMS monitoring showed that the reaction was complete. The reaction mixture was concentrated, and the resulting crude product was purified by trituration to give compound **n-2** (7.59 g, yield: 89%). **LCMS:** MS (ESI) m/z = 245.2 [M+H]⁺. ¹H NMR (400 MHz, DMSO) *δ* 9.74 (s, 1H), 8.27 (d, *J =* 5.6 Hz, 1H), 8.06 (d, *J* = 8.4 Hz, 1H), 7.95 (d, *J* = 8.0 Hz, 1H), 7.76 (t, *J* = 7.2 Hz, 1H), 7.65 (t, *J* = 7.2 Hz, 2H), 1.47 (s, 9H).

### Compound n-3

Sodium hydride (0.10 g, 2.45 mmol) was added to a solution of compound **n-2** (0.50 g, 2.05 mmol) in N,N-dimethylformamide (5.0 mL) at 0 °C. After the mixture was stirred for 30 min with the temperature maintained, ethyl 4-bromobutyrate (0.44 mL, 3.07 mmol) was added. The reaction was stirred at room temperature overnight, and LCMS monitoring showed that the reaction was complete. The reaction mixture was quenched with water and extracted with ethyl acetate (50 mL × 3). The combined organic phases were washed with saturated brine, dried over anhydrous sodium sulfate, and concentrated, and the resulting crude product was purified by normal-phase silica gel column chromatography to give compound **n-3** (0.59 g, yield: 81%). **LCMS:** MS (ESI) m/z = 359.4 [M+H]⁺.

### Compound n-4

Lithium hydroxide monohydrate (0.12 g, 4.94 mmol) was added to a mixed solution of compound **n-3** (0.59 g, 1.64 mmol) in tetrahydrofuran (5.0 mL) and water (2.0 mL). The reaction was stirred at room temperature for 1 h, and LCMS monitoring showed that the reaction was complete. The pH of the reaction mixture was adjusted to 2 with 1 N HCl, and extraction was performed with ethyl acetate (40 mL × 3). The combined organic phases were washed with saturated brine, dried over anhydrous sodium sulfate, and concentrated to give compound **n-4** (0.53 g, yield: 97%). **LCMS:** MS (ESI) m/z = 331.1

### [M+H]⁺.

### Compound n-5

N,N-Diisopropylethylamine (0.85 mL, 4.90 mmol), O-(7-azabenzotriazol-1-yl)-N,N,N',N'-tetramethyluronium hexafluorophosphate (0.37 g, 0.98 mmol), and compound **Inter-1** (0.46 g, 0.98 mmol) were added to a solution of compound **n-4** (0.27 g, 0.82 mmol) in dichloromethane (10.0 mL). The reaction was stirred at room temperature for 2 h, and LCMS monitoring showed that the reaction was complete. The reaction mixture was quenched with water and extracted with ethyl acetate (40 mL × 3). The combined organic phases were washed with saturated brine, dried over anhydrous sodium sulfate, and concentrated, and the resulting crude product was purified by normal-phase silica gel column chromatography to give compound **n-5** (0.46 g, yield: 72%). **LCMS:** MS (ESI) m/z = 781.5 [M+H]⁺.

### Compound n-6

Palladium on carbon (0.12 g) was added to a solution of compound **n-5** (0.15 g, 0.192 mmol) in methanol (30.0 mL) in a hydrogen atmosphere. The reaction mixture was stirred at room temperature for 8 h, and LCMS monitoring showed that the reaction was complete. The reaction mixture was filtered and concentrated to give a mixture of compound **n-6** and compound **o-1** (0.07 g, yield: 56%). Compound **n-6: LCMS:** MS (ESI) m/z = 691.1 [M+H]⁺; compound **o-1: LCMS:** MS (ESI) m/z = 695.5 [M+H]⁺.

### Compound n-7

Azide-PEG5-Tos (0.08 g, 0.20 mmol) and potassium carbonate (0.03 g, 0.20 mmol) were added to a solution of the mixture of compound **n-6** and compound **o-1** (0.07 g, 0.10 mmol) in N,N-dimethylformamide (2 mL). The reaction was heated to 80 °C and stirred for 6 h, and LCMS monitoring showed that the reaction was complete. The reaction mixture was quenched with a saturated sodium bicarbonate solution and extracted with ethyl acetate (40 mL × 3). The combined organic phases were washed with saturated brine, dried over anhydrous sodium sulfate, and concentrated, and the resulting crude product was purified by preparative high performance liquid chromatography to give **compound n-7** (14.0 mg, yield: 15%). Compound **n-7: LCMS:** MS (ESI) m/z = 936.6 [M+H]⁺.

### Compound n-8

Lithium hydroxide monohydrate (2.00 mg, 0.06 mmol) was added to a mixed solution of compound **n-7** (20.00 mg, 0.02 mmol) in tetrahydrofuran (1.0 mL) and water (1.0 mL).

The reaction was stirred at room temperature for 2 h, and LCMS monitoring showed that the reaction was complete. The pH of the reaction mixture was adjusted to 2 with 1 N HCl, and extraction was performed with ethyl acetate (20 mL × 3). The combined organic phases were washed with saturated brine and dried over anhydrous sodium sulfate. The filtrate was concentrated to give compound **n-8** (19.0 mg, yield: 96%). **LCMS:** MS (ESI) m/z = 922.6 [M+H]⁺.

### Compound III-n'

Trifluoroacetic acid (1 mL) was added to a solution of compound **n-8** (19.00 mg, 0.02 mmol) in dichloromethane (1.0 mL). The reaction was stirred at room temperature for 6 h, and LCMS analysis showed that the reaction was complete. The reaction mixture was concentrated, and the resulting crude product was purified by preparative high performance liquid chromatography to give compound **III-n'** (17.60 mg, yield: 73%). **LCMS:** MS (ESI) m/z = 822.8 [M+H]⁺. ¹H NMR (400 MHz, DMSO) δ 8.54 (d, *J* = 8.3 Hz, 1H), 8.46 (d, *J* = 8.4 Hz, 1H), 8.31-8.21 (m, 2H), 7.86 (d, *J* = 6.4 Hz, 2H), 7.78-7.73 (m, 1H), 7.69 (t, *J* = 7.6 Hz, 2H), 7.57-7.48 (m, 2H), 7.47 (dd, *J* = 8.4, 4.8 Hz, 2H), 7.39 (d, *J* = 8.4 Hz, 2H), 7.31 (d, *J* = 8.0 Hz, 1H), 7.13 (d, *J* = 6.4 Hz, 1H), 7.04 (d*, J=* 8.0 Hz, 1H), 5.31 (q, *J* = 7.6 Hz, 1H), 4.36-4.25 (m, 2H), 4.00-3.87 (m, 2H), 3.78 (d, *J =* 5.6 Hz, 2H), 3.69 (dd, *J* = 5.6, 3.7 Hz, 2H), 3.62-3.47 (m, 18H), 2.79 (d, *J* = 7.2 Hz, 2H), 2.35 (t, *J* = 7.2 Hz, 2H), 1.96 (dd, *J* = 14.0, 6.8 Hz, 2H).

### Example 15: Synthesis of Compound III-o'

The synthesis scheme for compound **III-o'** is as follows:

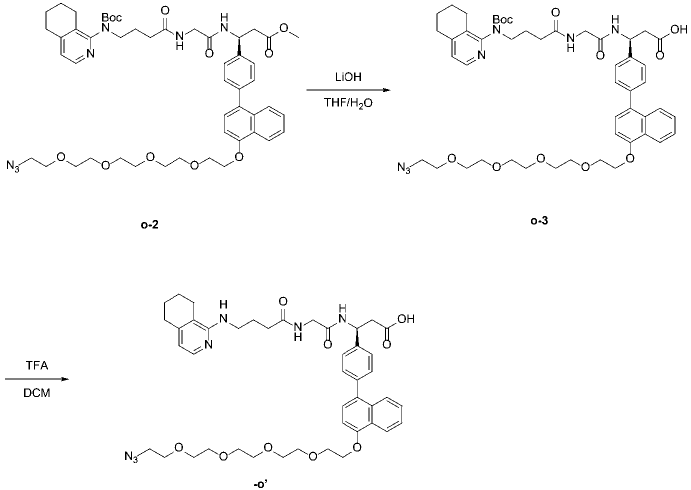

The synthesis and identification of specific intermediates and final products involved in the above scheme are as follows:

### Compound o-3

Lithium hydroxide monohydrate (2.00 mg, 0.08 mmol) was added to a mixed solution of compound **o-2** (24.00 mg, 0.03 mmol) in tetrahydrofuran (1.0 mL) and water (0.2 mL).

The reaction was stirred at room temperature for 2 h, and LCMS monitoring showed that the reaction was complete. The pH of the reaction mixture was adjusted to 2 with 1 N HCl, and extraction was performed with ethyl acetate (20 mL × 3). The combined organic phases were washed with saturated brine and dried over anhydrous sodium sulfate. The filtrate was concentrated to give compound **o-3** (21.00 mg, yield: 89%). **LCMS:** MS (ESI) m/z = 926.7 [M+H]⁺.

### Compound III-o'

Trifluoroacetic acid (1.0 mL) was added to a solution of compound **o-3** (21.00 mg, 0.02 mmol) in dichloromethane (1.0 mL). The reaction was stirred at room temperature for 6 h, and LCMS analysis showed that the reaction was complete. The reaction mixture was concentrated, and the resulting crude product was purified by preparative high performance liquid chromatography to give compound **III-o'** (14.00 mg, yield: 53%). **LCMS:** MS (ESI) m/z = 826.8 [M+H]⁺. ¹H NMR (400 MHz, DMSO) δ 8.54 (d, *J* = 8.4 Hz, 1H), 8.26 (dt, *J* = 11.6, 3.6 Hz, 2H), 7.81-7.71 (m, 1H), 7.65 (d, *J =* 6.4 Hz, 1H), 7.57-7.48 (m, 2H), 7.45 (d, *J* = 8.4 Hz, 2H), 7.39 (d, *J* = 8.4 Hz, 2H), 7.32 (d, *J* = 8.0 Hz, 1H), 7.05 (d, *J* = 8.0 Hz, 1H), 6.58 (d, *J* = 6.4 Hz, 1H), 5.30 (q, *J =* 7.2 Hz, 1H), 4.37-4.28 (m, 2H), 3.98-3.89 (m, 2H), 3.77 (d, *J* = 5.6 Hz, 2H), 3.69 (dd, *J* = 5.6, 3.6 Hz, 2H), 3.61-3.49 (m, 18H), 2.79 (d, *J* = 7.2 Hz, 2H), 2.65 (t, *J* = 5.6 Hz, 2H), 2.32 (dt, *J* = 14.0, 6.4 Hz, 4H), 1.83 (p, *J* = 7.2 Hz, 2H), 1.75 (dd, *J* = 11.6, 5.6 Hz, 2H), 1.66 (dd, *J* = 7.2, 3.6 Hz, 2H).

### Example 16: Synthesis of Compound III-p'

The synthesis scheme for compound **III-p'** is as follows:

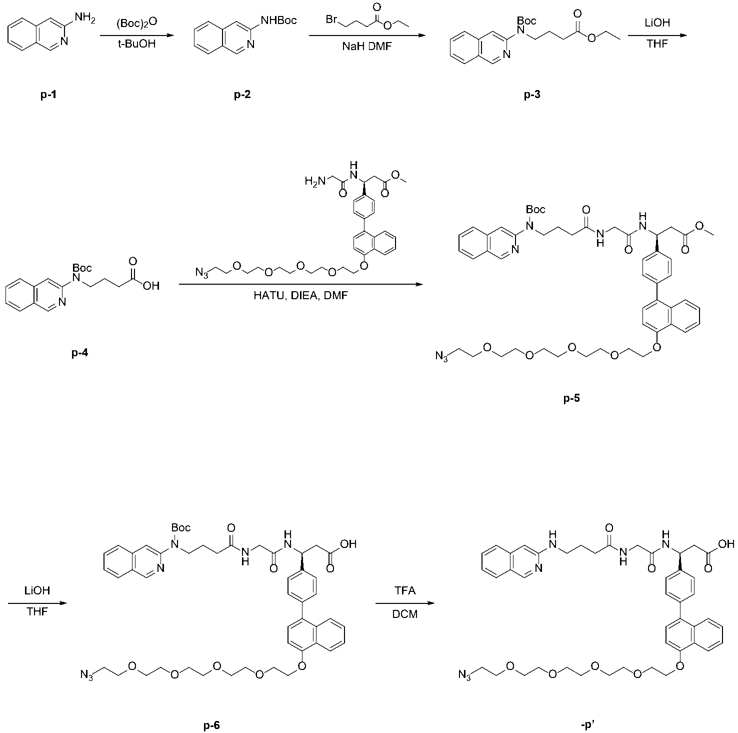

The synthesis and identification of specific intermediates and final products involved in the above scheme are as follows:

### Compound p-2

Di-tert-butyl dicarbonate (6.58 g, 30.17 mmol) was added to a solution of compound **p-1** (2.90 g, 20.11 mmol) in tert-butanol (50.0 mL). The reaction was stirred at room temperature overnight, and LCMS analysis showed that the reaction was complete. The reaction mixture was concentrated, and the resulting crude product was purified by normal-phase silica gel column chromatography to give compound **p-2** (3.90 g, yield: 79%). **LCMS:** MS (ESI) m/z = 245.4 [M+H]⁺.

### Compound p-3

Sodium hydride (0.19 g, 4.80 mmol) was added to a solution of compound **p-2** (0.98 g, 4.00 mmol) in N,N-dimethylformamide (10.0 mL) at 0 °C. After the mixture was stirred for 30 min with the temperature maintained, ethyl 4-bromobutyrate (0.93 mL, 4.81 mmol) was added. The reaction was stirred at room temperature overnight, and LCMS monitoring showed that the reaction was complete. The reaction mixture was quenched with water and extracted with ethyl acetate (50 mL × 3). The combined organic phases were washed with saturated brine, dried over anhydrous sodium sulfate, and concentrated, and the resulting crude product was purified by normal-phase silica gel column chromatography to give compound **p-3** (1.30 g, yield: 91%). **LCMS:** MS (ESI) m/z = 359.5 [M+H]⁺.

### Compound p-4

A 1 N solution of lithium hydroxide (20.0 mL) was added to a solution of compound **p-3** (1.30 g, 3.63 mmol) in tetrahydrofuran (20.0 mL). The reaction was stirred at room temperature for 2 h, and LCMS monitoring showed that the reaction was complete. The pH of the reaction mixture was adjusted to 2 with 1 N HCl, and extraction was performed with ethyl acetate (20 mL × 3). The combined organic phases were washed with saturated brine and dried over anhydrous sodium sulfate. The filtrate was concentrated to give compound **p-4** (1.18 g, yield: 98%). **LCMS:** MS (ESI) m/z = 331.1 [M+H]⁺.

### Compound p-5

N,N-Diisopropylethylamine (0.39 g, 3.03 mmol), O-(7-azabenzotriazol-1-yl)-N,N,N',N'-tetramethyluronium hexafluorophosphate (0.28 g, 0.73 mmol), and compound **m-3** (0.28 g, 0.72 mmol) were added to a solution of compound **p-4** (0.20 g, 0.61 mmol) in N,N-dimethylformamide (5.0 mL). The reaction was stirred at room temperature for 2 h, and LCMS monitoring showed that the reaction was complete. The reaction mixture was quenched with water and extracted with ethyl acetate (40 mL × 3). The combined organic phases were washed with saturated brine, dried over anhydrous sodium sulfate, and concentrated, and the resulting crude product was purified by normal-phase silica gel column chromatography to give compound **p-5** (0.30 g, yield: 53%). **LCMS:** MS (ESI) m/z = 936.7 [M+H]⁺.

### Compound p-6

A 1 N solution of lithium hydroxide (2.0 mL) was added to a solution of compound **p-5** (0.07 g, 0.08 mmol) in tetrahydrofuran (2.0 mL). The reaction was stirred at room temperature for 2 h, and LCMS monitoring showed that the reaction was complete. The pH of the reaction mixture was adjusted to 2 with 1 N HCl, and extraction was performed with ethyl acetate (20 mL × 3). The combined organic phases were washed with saturated brine and dried over anhydrous sodium sulfate. The filtrate was concentrated to give compound **p-6** (0.27 g, yield: 91%). **LCMS:** MS (ESI) m/z = 922.1 [M+H]⁺.

### Compound III-p'

Trifluoroacetic acid (4.0 mL) was added to a solution of compound **p-6** (0.27 g, 0.29 mmol) in dichloromethane (4.0 mL). The reaction was stirred at room temperature for 3 h, and LCMS analysis showed that the reaction was complete. The reaction mixture was concentrated, and the resulting crude product was purified by preparative high performance liquid chromatography to give compound **III-p'** (0.09 g, yield: 37%). **LCMS:** MS (ESI) m/z = 822.1 [M+H]⁺. ¹H NMR (400 MHz, DMSO) δ 8.97 (s, 1H), 8.51 (d, *J* = 8.4 Hz, 1H), 8.33-8.23 (m, 1H), 8.15 (t*, J =* 5.6 Hz, 1H), 7.88 (d, *J =* 8.4 Hz, 1H), 7.79-7.72 (m, 1H), 7.63 (d, *J =* 8.4 Hz, 1H), 7.51 (m, 8H), 7.31 (d, *J =* 8.0 Hz, 1H), 7.26-7.20 (m, 1H), 7.04 (d, *J* = 8.0 Hz, 1H), 6.91 (s, 1H), 5.32 (q, *J* = 7.2 Hz, 1H), 4.34-4.31 (m, 2H), 3.95-3.91 (m, 2H), 3.78 (d, *J* = 5.6 Hz, 2H), 3.69 (dd, *J* = 6.0, 3.6 Hz, 2H), 3.61-3.50 (m, 13H), 3.38-3.34 (m, 2H), 3.29 (t, *J* = 7.2 Hz, 2H), 2.80 (d, *J =* 7.2 Hz, 2H), 2.29 (t, *J =* 7.2 Hz, 2H), 1.86 (p, *J* = 7.2 Hz, 2H).

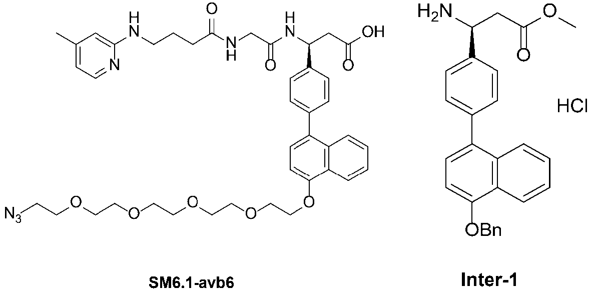

**SM6.1-αvβ6 and Inter-1,** as represented by the above structural formulas, were prepared using the method described in Patent Application No. WO2019089765A1.

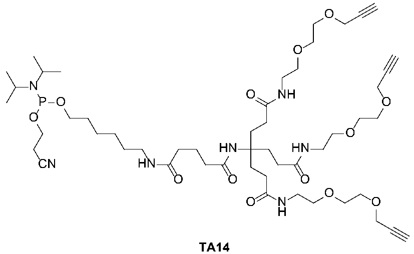

**TA14,** as represented by the above structural formula, was prepared using the method described in Patent Application No. WO2019161213A1.

### Example 17: Conjugation of Targeting Ligands to siRNAs

### A. Synthesis of alkyne-functionalized sense strands

The synthesis of dsRNAs was the same as the conventional phosphoramidite solid-phase synthesis method. The synthesis process is briefly described below: Nucleoside phosphoramidite monomers were linked one by one according to a synthesis program on a Dr. Oligo48 synthesizer (Biolytic), with a universal CPG support as a start. Finally, alkynyl-containing phosphoramidite monomers were linked to the 5' ends of the sense strands. The nucleoside monomer starting materials 2'-F RNA, 2'-O-methyl RNA, and other nucleoside phosphoramidite monomers were purchased from Shanghai Hongene or Suzhou Genepharma. The alkynyl-containing phosphoramidite monomers were prepared using the preparation methods in Examples 1-5. 5-Ethylthio-1H-tetrazole (ETT) was used as an activator (0.6 M in acetonitrile), a 0.22 M solution of PADS in acetonitrile and collidine (1:1 by volume) (Suzhou Kroma) was used as a sulfurizing agent, and an aqueous iodopyridine solution (Kroma) was used as an oxidant.

After solid-phase synthesis was completed, the oligoribonucleotides were cleaved from the solid support by soaking in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by anion exchange chromatography (mobile phase A: 10 mM NaOH in water, mobile phase B: 10 mM NaOH and 1 M NaCl in water). The target oligonucleotides were collected, then lyophilized, identified as the target products by LC-MS, and quantified using NanoDrop with UV (260 nm).

### B. Conjugation to targeting groups

The **5'** alkyne-functionalized sense strands were conjugated to αvβ6 integrin-targeting groups. The conjugation to the αvβ6 integrin-targeting groups is described below:
Stock solutions of 0.5 M tris(3-hydroxypropyltriazolylmethyl)amine (THPTA), 0.5 M Cu(II) sulfate pentahydrate (Cu(II)SO4·5H2O), and 2 M sodium ascorbate solutions were prepared in deionized water. A sense strand containing alkyne functionalization (2.5 µmol) was placed in a centrifuge tube and dissolved in 2 M TEAA, and its concentration was adjusted to 10,000-15,000 ng/µL. An αvβ6 integrin ligand (35-50 µmol) was dissolved in a solution of DMSO (shaking until a clear solution was formed). The solution of the αvβ6 integrin ligand in DMSO was added to the solution of the sense strand containing alkyne functionalization in 2 M TEAA, and the mixture was shaken until it was homogeneous. Then, 75 µL of 0.5 M Cu(II) (Cu(II)SO4·5H2O), 75 µL of 0.5 M THPTA, and 18 µL of 2 M ascorbate were sequentially added to the centrifuge tube. After 1 h of shaking on a thermostatic reactor, LCMS analysis showed that the reaction was complete. After centrifugation, the supernatant was collected and purified by hydrophobic chromatography (A: ammonium sulfate buffer, B: purified water) to give the target product.

### C. Formation of dsRNAs containing conjugates

The single-stranded oligonucleotides obtained were complementarily paired in an equimolar ratio and annealed. The finally obtained dsRNAs were dissolved in 1× PBS, and the concentration was adjusted to the concentration required for the experiment so that they were ready for use.

### Example 18: αvβ6 Binding Ability Test

αvβ6 binding assay using fluorescence polarization (FP) method The analysis was based on competition with a fluorescently labeled RGD peptide for binding to αvβ6. The binding abilities of the test compounds were evaluated and screened according to FP value changes.

The test compounds (3.5 µL) were added to a 384-well black plate. For each test compound, 12 concentration points were set. The highest concentration was 10 µM, and was serially diluted 3-fold. DMSO control wells were used to determine the maximum signal, and 500 nM compound CWHM-12

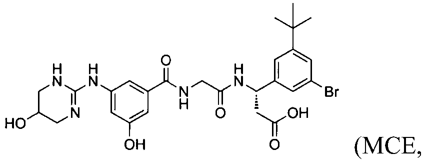

HY-18644) control wells were used as the minimum signal.Then, 3.5 µL of a 4× human recombinant αvβ6 protein working solution (Acrobiosystem) was added. After 15 min of incubation at room temperature, 7 µL of a 2× fluorescent RGD peptide solution (Sangon Biotech) was added to each well. After 1 h of incubation at room temperature, FP signals were read using Envision. GraphPad Prism was used to fit inhibition curves and calculate IC₅₀ values.

The IC₅₀ values (Z factor > 0.5) were calculated using the following formula:
Curve fitting formula: Y = Bottom + (Top - Bottom)/(1 + 10^((LogIC₅₀ - X) × Hill Slope)) X: logarithmic value of inhibitor concentration; Y: inhibition percentage; Bottom: minimum response; Top: maximum response; Hill Slope: curve slope.

**Table 1. Data on the abilities of the compounds (III-h')-(III-m') of the present disclosure to bind to αvβ6**

| **Compound No.** | **αvβ6 IC₅₀ (nM)** |
|---|---|
| SM6.1-αvβ6 | 1.597 |
| III-h' | 0.856 |
| III-i' | 2.469 |
| III-j' | 1.978 |
| III-k' | 2.286 |
| III-l' | 1.672 |
| III-m' | 1.469 |

### Example 19: Design of Mouse RAGE, MUC5B, and APP dsRNAs

With the mouse RAGE gene, the mouse MUC5B gene, and the mouse APP gene as target genes, dsRNAs of 21/21nt or 21/23nt were designed in accordance with the general rules for active dsRNAs. The sequences of unmodified sense and antisense strands are shown in Table 2, and the sequences of sense and antisense strands modified with 2'-fluoro, 2'-methoxy, etc. are detailed in Tables 3 and 4. The preparation method was the same as that in Example 17.

**Table 2. Unmodified sense and antisense strands of dsRNAs**

| | SEQ ID NO: | Unmodified SS strand (5'-3') | SEQ ID NO: | Unmodified AS strand (5'-3') |
|---|---|---|---|---|
| RAGE | 1 | CCACAUGAUCCAUG CUIAGUA | 2 | UACUCAGCAUGGAU CAUGUGG |
| MUC5B | 3 | GUGCUUUAACUACA AUAUACA | 4 | UGUAUAUUGUAGUU AAAGCACAU |
| APP | 5 | CCGGUCCCAGGUUA UGACACA | 6 | UGUGUCAUAACCUG GGACCGGAU |

**Table 3. Alkyne-functionalized sense strand structures**

| No. | SEQ ID NO: | Sense strand (5' to 3') |
|---|---|---|
| S1045 | 7 | II-1- CmsCmAmCmAmUmGmAmUfCfCfAmUmGmC mUmImAmGmUmAmsIB |
| S1112 | 8 | II-5- CmsCmAmCmAmUmGmAmUfCfCfAmUmGmC mUmImAmGmUmAmsIB |
| S1113 | 9 | II-3- CmsCmAmCmAmUmGmAmUfCfCfAmUmGmC mUmImAmGmUmAmsIB |
| S1114 | 10 | II-2- CmsCmAmCmAmUmGmAmUfCfCfAmUmGmC mUmImAmGmUmAmsIB |
| S1044 | 11 | TA14'- CmsCmAmCmAmUmGmAmUfCfCfAmUmGmC mUmImAmGmUmAmsIB |
| S2850 | 12 | II-3- GmsUmsGmCmUmUmUfAmAfCfUfAmCmAmA mUmAmUmAmCmsAmsIB |
| S2851 | 13 | TA14'- GmsUmsGmCmUmUmUfAmAfCfUfAmCmAmA mUmAmUmAmCmsAmsIB |
| S3059 | 14 | II-2- GmsUmsGmCmUmUmUfAmAfCfUfAmCmAmA mUmAmUmAmCmsAmsIB |
| S3060 | 15 | II-5- GmsUmsGmCmUmUmUfAmAfCfUfAmCmAmA mUmAmUmAmCmsAmsIB |
| S3132 | 16 | II-3- CmsCmsGmGmUmCmCfCmAfGfGfUmUmAmU mGmAmCmAmCmsAmsIB |
| S3133 | 17 | TA14'- CmsCmsGmGmUmCmCfCmAfGfGfUmUmAmU mGmAmCmAmCmsAmsIB |

In the table, the structure of II-1 is as follows: 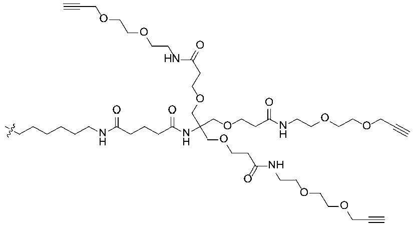
the structure of II-5 is as follows: 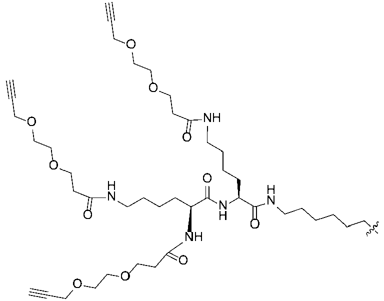
the structure of II-3 is as follows: 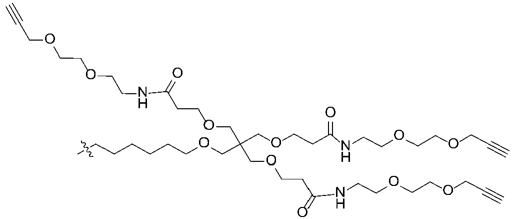
the structure of II-2 is as follows: 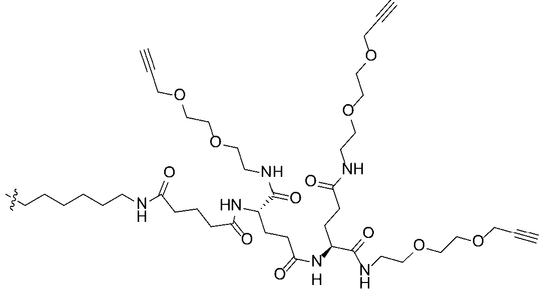
the structure of TA14' is as follows:

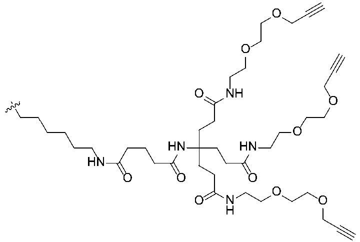

**Table 4. Sense and antisense strands**

| siRNA No. | Nucleobase No. | SEQ ID NO: | Sequence direction 5' to 3' |
|---|---|---|---|
| TJR101402 | Sense strand: S1047 | 18 | III-1- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101470 | Sense strand: S1115 | 20 | III-5- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101471 | Sense strand: S1116 | 21 | III-3- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101472 | Sense strand: S1117 | 22 | III-2- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101468-8 | Sense strand: S1113-8 | 23 | III-S1113-8- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101468-11 | Sense strand: S1113-11 | 24 | III-S1113-11- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101468-13 | Sense strand: S1113-13 | 25 | III-S1113-13- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR101401 | Sense strand: S1046 | 26 | TA14’’- CmsCmAmCmAmUmGmAmUfCfCfAm UmGmCmUmImAmGmUmAmsIB |
| | Antisense strand: A1132 | 19 | VPUmsAfsCmsUfCmAfGmCfAmUfGmG fAmUfCmAfUmGfUmGfsGm |
| TJR103427-8 | Sense strand: S2850-8 | 27 | III-S1113-8- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103427-11 | Sense strand: S2850-11 | 29 | III-S1113-11- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103427-13 | Sense strand: S2850-13 | 30 | III-S1113-13- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103428-1 | Sense strand: S2851-1 | 31 | TA14''- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103427-1 | Sense strand: S2850-1 | 32 | III-3- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103700-1 | Sense strand: S3059-1 | 33 | III-2- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103701-1 | Sense strand: S3060-1 | 34 | III-5- GmsUmsGmCmUmUmUfAmAfCfUfAm CmAmAmUmAmUmAmCmsAmsIB |
| | Antisense strand: A0089 | 28 | VPUmsGfsUmAmUmAfUmUfGfUmAm GmUmUfAmAfAmGmCmAmCmsAmsU m |
| TJR103788-8 | Sense strand: S3132-8 | 35 | III-S1113-8- CmsCmsGmGmUmCmCfCmAfGfGfUm UmAmUmGmAmCmAmCmsAmsIB |
| | Antisense strand: A1318 | 36 | VPUmsGfsUmGmUmCfAmUfAfAmCmC mUmGfGmGfAmCmCmGmGmsAmsUm |
| TJR103788-11 | Sense strand: S3132-11 | 37 | III-S1113-11- CmsCmsGmGmUmCmCfCmAfGfGfUm UmAmUmGmAmCmAmCmsAmsIB |
| | Antisense strand: A1318 | 36 | VPUmsGfsUmGmUmCfAmUfAfAmCmC mUmGfGmGfAmCmCmGmGmsAmsUm |
| TJR103788-13 | Sense strand: S3132-13 | 38 | III-S1113-13- CmsCmsGmGmUmCmCfCmAfGfGfUm UmAmUmGmAmCmAmCmsAmsIB |
| | Antisense strand: A1318 | 36 | VPUmsGfsUmGmUmCfAmUfAfAmCmC mUmGfGmGfAmCmCmGmGmsAmsUm |

In the table, the structure of III-1 is as follows: 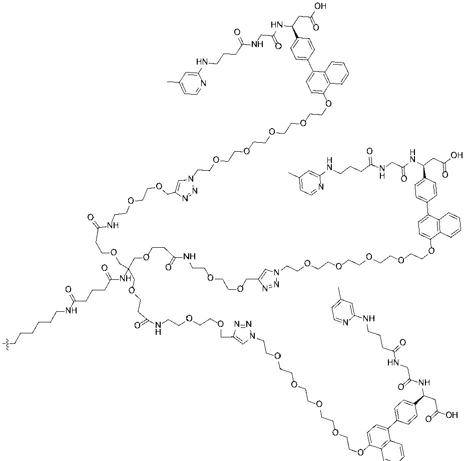
the structure of III-5 is as follows: 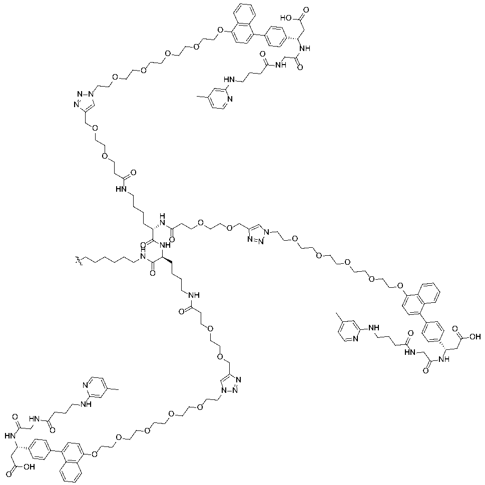
the structure of III-3 is as follows: 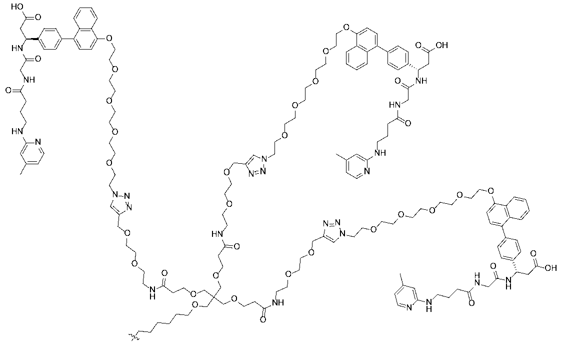
the structure of III-2 is as follows: 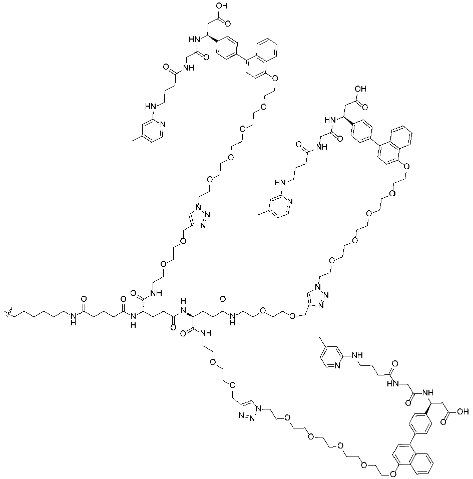
the structure of III-S1113-8 is as follows: 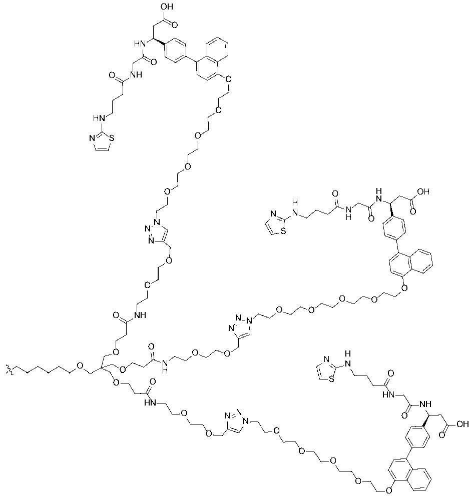
the structure of III-S1113-11 is as follows: 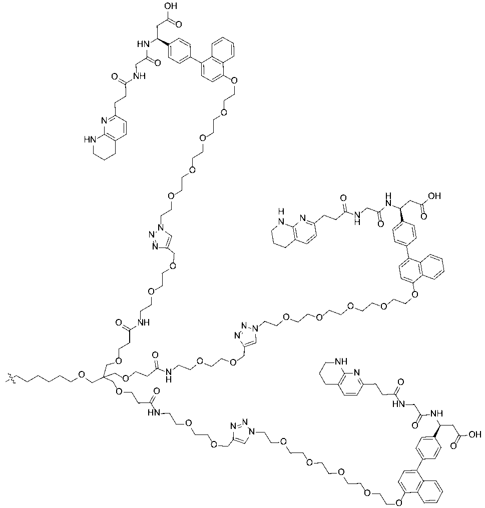
the structure of III-S1113-13 is as follows: 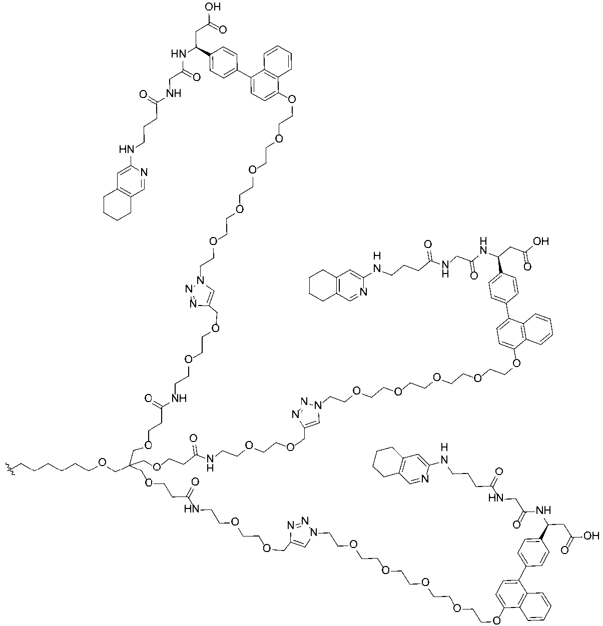
the structure of TA14" is as follows:

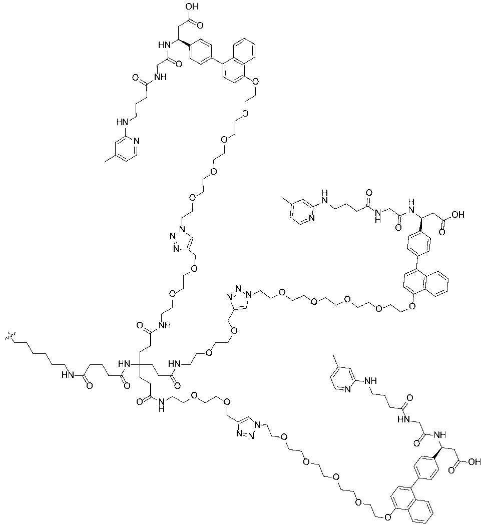

The above III-1, III-2, III-3, III-5, III-S1113-8, III-S1113-11, III-S1113-13, and TA14" are linked to the adjacent nucleotides by phosphodiester linkages.

The lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; the lowercase letter s means that the two nucleosides adjacent to the letter s are linked by a phosphorothioate diester group. Unless otherwise specified, two nucleosides are linked by a phosphodiester group.

The structure of VPUms is as follows:

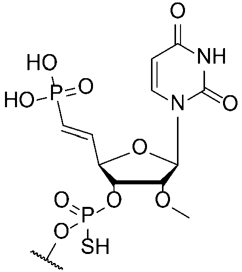

The structural representation of IB is as follows:

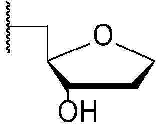

Im = hypoxanthine 2'-OMe ribonucleoside.

### Example 20: In Vitro Activity

### Inhibitory activity against mouse RAGE in MLE 12 cells

An siRNA was subjected to activity screening in MLE 12 cells (Haixing Biosciences) using 2 concentration gradients (100 nM and 10 nM).

MLE 12 cells were cryopreserved in liquid nitrogen. Twenty-four hours prior to transfection, the MLE 12 cells were thawed and then seeded into a 96-well plate at a density of 1.5 × 10⁴ cells per well, with each well containing 100 µL of a culture medium. The cells were transfected with the siRNA using Lipofectamine RNAi MAX (ThermoFisher, 13778150) according to the product instructions. Two concentration points, 100 nM and 10 nM, were set, and two duplicate wells were set for each concentration. After 24 h of treatment, total cell RNA extraction was performed using a high-throughput cell RNA extraction kit, and RNA reverse transcription and quantitative real-time PCR detection were performed to determine the mRNA level of mouse RAGE. The mRNA level of mouse RAGE was corrected based on the GAPDH internal reference gene level.

For the real-time quantitative PCR detection, a probe Q-PCR detection experiment was used, and its primer information is shown in Table 5. The primers were ordered from Sangon Biotech (Shanghai) Co., Ltd.

**Table 5. Taqman primers**

| Primer name | SEQ ID NO: | Primer sequence |
|---|---|---|
| Mouse-RAGE-F | 39 | TTGAGCCTGAAGGTGGAATAG |
| Mouse-RAGE-R | 40 | AGGGTGCACCATCCTTTATC |
| Mouse-RAGE-probe | 41 | CAGAGATGGCACAGGTCAAGGTCAC |
| Mouse-GAPDH-F | 42 | AACAGCAACTCCCACTCTTC |
| Mouse-GAPDH-R | 43 | CCTGTTGCTGTAGCCGTATT |
| Mouse-GAPDH-probe | 44 | AAAGTTGTCATTGAGAGCAATGCCAGC |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-ΔΔCt}, where ΔΔCt = [(Ct experimental group target gene - Ct experimental group internal reference) - (Ct control group target gene - Ct control group internal reference)]. The remaining levels of target gene expression were calculated.

The results are expressed relative to the remaining percentage of mouse RAGE mRNA expression in cells treated with the control siRNA. The results are shown in Table 6.

**Table 6. The activity of an siRNA in MLE 12 cells**

| Double strand No. | 100 nM remaining level (%) | STDEV(%) | 10 nM remaining level (%) | STDEV(%) |
|---|---|---|---|---|
| TJR101402 | 43.74 | 9.18 | 44.43 | 4.23 |

### Example 21: In Vitro Activity

### Inhibitory activity against mouse RAGE in MLE 12 cells

RNAi agents were subjected to a molecular-level simulation of endogenous cell activity screening in MLE 12 cells using 7 concentration gradients.

MLE 12 cells were cryopreserved in liquid nitrogen. For transfection, the MLE 12 cells were thawed and then seeded into a 96-well plate at a density of 2 × 10⁴ cells per well, with each well containing 100 µL of a culture medium. The cells were transfected with the RNAi agents using an RNAi MAX transfection reagent (ThermoFisher, 13778150) according to the instructions, with 0.3 µL of the RNAi MAX transfection reagent used per well. For the RNAi agents, a total of 7 concentration points were set. The highest concentration point final concentration was 100 nM, and was serially diluted 5-fold to obtain 100.0000 nM, 20 nM, 4 nM, 0.8 nM, 0.16 nM, 0.032 nM, and 0.0064 nM, and two duplicate wells were set for each concentration. After 48 h of treatment, total cell RNA extraction was performed using a high-throughput cell RNA extraction kit, and RNA reverse transcription and quantitative real-time PCR detection were performed to determine the mRNA level of mouse RAGE. The mRNA level of mouse RAGE was corrected based on the GAPDH internal reference gene level.

For the real-time quantitative PCR detection, a probe Q-PCR detection experiment was used, and its primer information is shown in Table 5.

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-ΔΔCt}, where ΔΔCt = [(Ct experimental group target gene - Ct experimental group internal reference) - (Ct control group target gene - Ct control group internal reference)]. The remaining levels of target gene expression were calculated.

The results are expressed relative to the remaining percentage of mouse RAGE mRNA expression in cells treated with the control siRNA. The results are shown in Table 7.

**Table 7. The activity of siRNAs in MLE 12 cells**

| Double strand No. | IC₅₀ |
|---|---|
| TJR101468-8 | 0.919 |
| TJR101468-11 | 0.823 |
| TJR101468-13 | 0.568 |

### Example 22: In Vivo Activity of TJR101402

C57BL/6J male mice aged 7 weeks (purchased from Zhejiang Vital River Laboratory Animal Technology Co., Ltd.) were acclimatized for 3-7 days after entering an animal facility. Twenty mice were weighed and divided into 4 groups of 5: G1 was a normal saline group, and G2-4 were TJR101402 0.3 mg/kg, 1 mg/kg, and 3 mg/kg, respectively. On day 1 of the experiment, the mice were weighed, divided into groups, anesthetized with isoflurane, and then dosed transtracheally using a mouse pulmonary drug delivery device (Yuyan Instruments); a single dose of TJR101402 (different concentrations) was administered. On day 8 of the experiment, the mice were euthanized, and after blood was collected from the heart, the whole lungs were collected, snap-frozen in liquid nitrogen, and then stored at -80 °C. The RAGE mRNA levels in the lung tissues of the mice were determined by the real-time quantitative PCR method; the primer information was the same as that in Example 20.

**Table 8. The activity of TJR101402 against mouse lung RAGE**

| Group | Mean relative mouse lung RAGE expression (x̅ ± SD) |
|---|---|
| Normal saline group | 1.000±0.102 |
| TJR101402 0.3 mg/kg | 0.532±0.155 |
| TJR101402 1 mg/kg | 0.424±0.311 |
| TJR101402 3 mg/kg | 0.196±0.116 |

The results are shown above. Seven days after administration, the expression of RAGE in the lungs of the mice in the three dose groups, TJR101402 0.3 mg/kg, 1 mg/kg, and 3 mg/kg, was significantly reduced compared to the normal saline group.

### Example 23: In Vivo Activity of TJR101470, TJR101471, and TJR101472

C57BL/6J male mice aged 7 weeks (purchased from Zhejiang Vital River Laboratory Animal Technology Co., Ltd.) were acclimatized for 3-7 days after entering an animal facility. Forty mice were weighed and divided into 4 groups of 10: G1 was a normal saline group, and G2-4 were TJR101470, TJR101471, and TJR101472, respectively. On day 1 of the experiment, the mice were weighed, divided into groups, anesthetized with isoflurane, and then dosed transtracheally using a mouse pulmonary drug delivery device (Yuyan Instruments); the single-dose injection dose was 1 mg/kg. On day 15 of the experiment, the mice were euthanized, and after blood was collected from the heart, the whole lungs were collected, snap-frozen in liquid nitrogen, and then stored at -80 °C. The RAGE mRNA levels in the lung tissues of the mice were determined by the real-time quantitative PCR method; the primer information was the same as that in Example 20. The experimental results are shown in FIG. 1. Fourteen days after administration, TJR101470, TJR101471, and TJR101472 all exhibited significant mouse lung RAGE expression knockdown activity, and their mouse lung RAGE expression inhibition rates were 62.4%, 54.0%, and 66.6%, respectively.

### Example 24: In Vivo Activity of TJR101401, TJR101468-8, and TJR101468-13

C57BL/6J male mice aged 7 weeks (purchased from Zhejiang Vital River Laboratory Animal Technology Co., Ltd.) were acclimatized for 3-7 days after entering an animal facility. Twenty mice were weighed and divided into 4 groups of 5: G1 was a normal saline group, and G2-4 were TJR101401, TJR101468-08, and TJR101468-13, respectively. On day 1 of the experiment, the mice were weighed, divided into groups, anesthetized with isoflurane, and then dosed transtracheally using a mouse pulmonary drug delivery device (Yuyan Instruments); the single-dose injection dose was 1 mg/kg. On day 15 of the experiment, the mice were euthanized, and after blood was collected from the heart, the whole lungs were collected, snap-frozen in liquid nitrogen, and then stored at -80 °C. The RAGE mRNA levels in the lung tissues of the mice were determined by the real-time quantitative PCR method; the primer information was the same as that in Example 20. The experimental results are shown in FIG. 2. The results show that 14 days after administration, TJR101401, TJR101468-8, and TJR101468-13 all exhibited significant mouse lung RAGE expression knockdown activity, and their mean mouse lung RAGE mRNA expression inhibition rates were 44.2%, 66.8%, and 75.6%, respectively. The data indicate that TJR101468-8 and TJR101468-13 knocked down the expression of RAGE in the lungs of mice more significantly than TJR101401.

### Example 25: In Vivo Activity of TJR101401, TJR101468-11, and TJR101468-13

C57BL/6J male mice aged 7 weeks (purchased from Zhejiang Vital River Laboratory Animal Technology Co., Ltd.) were acclimatized for 3-7 days after entering an animal facility. Forty mice were weighed and divided into 4 groups of 10: G1 was a normal saline group, and G2-4 were TJR101401, TJR101468-11, and TJR101468-13, respectively. On day 1 of the experiment, the mice were weighed, divided into groups, anesthetized with isoflurane, and then dosed transtracheally using a mouse pulmonary drug delivery device (Yuyan Instruments); the single-dose injection dose was 0.3 mg/kg. On day 29 of the experiment, the mice were euthanized, and after blood was collected from the heart, the whole lungs were collected, snap-frozen in liquid nitrogen, and then stored at -80 °C. The RAGE mRNA levels in the lung tissues of the mice were determined by the real-time quantitative PCR method; the primer information was the same as that in Example 20. The experimental results are shown in FIG. 3. Twenty-eight days after administration, TJR101401, TJR101468-11, and TJR101468-13 all exhibited significant mouse lung RAGE expression knockdown activity, and their mean mouse lung RAGE mRNA expression inhibition rates were 34.7%, 69.1%, and 60.9%, respectively. The data indicate that TJR101468-11 and TJR101468-13 knocked down the expression of RAGE in the lungs of mice more significantly than TJR101401.

### Example 26: In Vitro Activity

### Inhibitory activity against mouse MUC5B in MLE 12 cells

RNAi agents were subjected to a molecular-level simulation of endogenous cell activity screening in MLE 12 cells using 7 concentration gradients.

MLE 12 cells were cryopreserved in liquid nitrogen. For transfection, the MLE 12 cells were thawed and then seeded into a 96-well plate at a density of 2 × 10⁴ cells per well, with each well containing 100 µL of a culture medium. The cells were transfected with the RNAi agents using an RNAi MAX transfection reagent (ThermoFisher, 13778150) according to the instructions, with 0.3 µL of the RNAi MAX transfection reagent used per well. For the RNAi agents, a total of 7 concentration points were set. The highest concentration point final concentration was 100 nM, and was serially diluted 5-fold to obtain 100.0000 nM, 20 nM, 4 nM, 0.8 nM, 0.16 nM, 0.032 nM, and 0.0064 nM, and two duplicate wells were set for each concentration. After 48 h of treatment, total cell RNA extraction was performed using a high-throughput cell RNA extraction kit, and RNA reverse transcription and quantitative real-time PCR detection were performed to determine the mRNA level of mouse MUC5B. The mRNA level of mouse MUC5B was corrected based on the GAPDH internal reference gene level.

For the real-time quantitative PCR detection, a probe Q-PCR detection experiment was used, and its primer information is shown in Table 9. The primers were ordered from Sangon Biotech (Shanghai) Co., Ltd.

**Table 9. Taqman primers**

| Primer name | SEQ ID NO: | Primer sequence |
|---|---|---|
| Mouse-MUC5B-F | 45 | AGGTGCTCTGTTGTAGTGTTAG |
| Mouse-MUC5B-R | 46 | GTGTTGGGAAGAGGCATAGAA |
| Mouse-MUC5B-probe | 47 | CAATGACAGGATCAGCAACTACTGCCA |
| Mouse-GAPDH-F | 48 | AACAGCAACTCCCACTCTTC |
| Mouse-GAPDH-R | 49 | CCTGTTGCTGTAGCCGTATT |
| Mouse-GAPDH-probe | 50 | AAAGTTGTCATTGAGAGCAATGCCAGC |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-ΔΔCt}, where ΔΔCt = [(Ct experimental group target gene - Ct experimental group internal reference) - (Ct control group target gene - Ct control group internal reference)]. The remaining levels of target gene expression were calculated.

The results are expressed relative to the remaining percentage of mouse MUC5B mRNA expression in cells treated with the control siRNA. The results are shown in Table 10.

**Table 10. The activity of siRNAs in MLE 12 cells**

| Double strand No. | IC50 |
|---|---|
| TJR103427-11 | 0.206 |
| TJR103427-13 | 0.182 |
| TJR103427-8 | 0.092 |
| TJR103428-1 | 0.426 |

### Example 27: In Vivo Activity of TJR103427-11 and TJR103428-1

C57BL/6J male mice aged 7 weeks (purchased from Zhejiang Vital River Laboratory Animal Technology Co., Ltd.) were acclimatized for 3-7 days after entering an animal facility. Fifteen mice were weighed and divided into 3 groups of 5: G1 was a normal saline group, and G2-3 were TJR103427-11 and TJR103428-1, respectively. On day 1 of the experiment, the mice were weighed, divided into groups, anesthetized with isoflurane, and then dosed transtracheally using a mouse pulmonary drug delivery device (Yuyan Instruments); the single-dose injection dose was 3 mg/kg. On day 29 of the experiment, the mice were euthanized, and after blood was collected from the heart, the whole lungs were collected, snap-frozen in liquid nitrogen, and then stored at -80 °C. The MUC5B mRNA levels in the lung tissues of the mice were determined by the real-time quantitative PCR method; the primer information was the same as that in Example 26. The experimental results are shown in FIG. 4. Twenty-eight days after administration, TJR103427-11 exhibited significant mouse lung MUC5B expression knockdown activity, and its mean inhibition rate was 58.1%, whereas TJR103428-1 exhibited no knockdown activity.

### Example 28: In Vivo Activity of TJR103427-1, TJR103700-1, and TJR103701-1

C57BL/6J male mice aged 7 weeks (purchased from Zhejiang Vital River Laboratory Animal Technology Co., Ltd.) were acclimatized for 3-7 days after entering an animal facility. Forty mice were weighed and divided into 4 groups of 10: G1 was a normal saline group, and G2-4 were TJR103427-1, TJR103700-1, and TJR103701-1, respectively. On day 1 of the experiment, the mice were weighed, divided into groups, anesthetized with isoflurane, and then dosed transtracheally using a mouse pulmonary drug delivery device (Yuyan Instruments); the single-dose injection dose was 3 mg/kg. On day 15 of the experiment, the mice were euthanized, and after blood was collected from the heart, the whole lungs were collected, snap-frozen in liquid nitrogen, and then stored at -80 °C. The MUC5B mRNA levels in the lung tissues of the mice were determined by the real-time quantitative PCR method; the primer information was the same as that in Example 26. The experimental results are shown in FIG. 5. Fourteen days after administration, TJR103427-1, TJR103700-1, and TJR103701-1 all exhibited significant mouse lung MUC5B expression knockdown activity, and their mean inhibition rates were 24.5%, 32.1%, and 33.2%, respectively.

### Example 29: In Vitro Activity

### Inhibitory activity against APP in A172 cells

RNAi agents were subjected to a molecular-level simulation of endogenous cell activity screening in A172 cells using 7 concentration gradients.

A172 cells were cultured in a DMEM + 10% FBS complete culture medium at 37 °C with 5% CO₂. Twenty-four hours before transfection, the A172 cells were seeded into a 96-well plate at a density of 1 × 10⁴ cells/well, with each well containing 100 µL of the culture medium.

The cells were transfected with the RNAi agents using an RNAi MAX transfection reagent (ThermoFisher, 13778150) according to the instructions, with 0.3 µL of the RNAi MAX transfection reagent used per well. For the RNAi agents, a total of 7 concentration points were set. The highest concentration point final concentration was 10 nM, and was serially diluted 7-fold to obtain 10.0000 nM, 1.428 nM, 0.204 nM, 0.0291 nM, 0.004 nM, 0.00059 nM, and 0.00008 nM, and two duplicate wells were set for each concentration. After 48 h of treatment, total cell RNA extraction was performed using a high-throughput cell RNA extraction kit, and RNA reverse transcription and quantitative real-time PCR detection were performed to determine the mRNA level of human APP. The mRNA level of human APP was corrected based on the GAPDH internal reference gene level.

For the real-time quantitative PCR detection, a probe Q-PCR detection experiment was used, and its primer information is shown in Table 11.

**Table 11. Taqman primers**

| Primer name | SEQ ID NO: | Primer sequence |
|---|---|---|
| Human-APP-F | 51 | GACAGACAGCACACCCTAAA |
| Human-APP-R | 52 | CACACGGAGGTGTGTCATAA |
| Human-APP-probe | 53 | ATCCCAAGAAAGCCGCTCAGATCC |
| Human-GAPDH-F | 54 | CCAGGTGGTCTCCTCTGACTTC |
| Human-GAPDH-R | 55 | GTGGTCGTTGAGGGCAATG |
| Human-GAPDH-probe | 56 | ACAGCGACACCCACTCCTCCACCTT |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-ΔΔCt}, where ΔΔCt = [(Ct experimental group target gene - Ct experimental group internal reference) - (Ct control group target gene - Ct control group internal reference)]. The remaining levels of target gene expression were calculated.

The results are expressed relative to the remaining percentage of human APP mRNA expression in cells treated with the control siRNA. The results are shown in Table 12.

**Table 12. The activity of siRNAs in A172 cells**

| Double strand No. | IC₅₀ (nM) |
|---|---|
| TJR103788-8 | 0.895 |
| TJR103788-11 | 1.182 |
| TJR103788-13 | 1.556 |

## Claims

1. A targeting ligand represented by formula III or a pharmaceutically acceptable salt thereof, 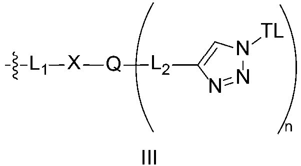
wherein L₁ is -(CH₂)_{q1}-, -(CH₂)_{q2}-NH-C(=O)-(CH₂)_{q3}-, or -(CH₂)_{q4}-O-(CH₂)_{q5}-, and q1, q2, q3, q4, and q5 are each independently an integer from 1 to 10;
X is a bond, -C(=O), -C(=O)-NH-, or -NH-C(=O)-;
TL are each independently a targeting group;
n is 3 or 4;
Q and L₂ are any one of the following combinations:
combination 1: Q is 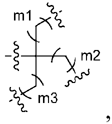 and L₂ are each independently -O-(CH₂)ₚ₁-C(=O)-NH-(CH₂CH₂O)ₚ₂-(CH₂)ₚ₃;
combination 2: Q is 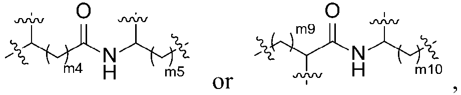 and L₂ are each independently -C(=O)-NH-(CH₂CH₂O)ₚ₄-(CH₂)ₚ₅;
combination 3: Q is 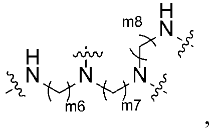 and L₂ are each independently -C(=O)-(CH₂CH₂O)ₚ₆-(CH₂)ₚ₇; and
combination 4: Q is 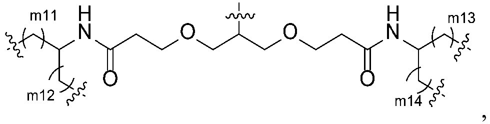 and L₂ are each independently -O-(CH₂)ₚ₁-C(=O)-NH-(CH₂CH₂O)ₚ₂-(CH₂)ₚ₃;
m1, m2, m3, m4, m5, m6, m7, m8, m9, m10, m11, m12, m13, and m14 are each independently 0, 1, 2, 3, 4, 5, or 6;
p1, p2, p3, p4, p5, p6, and p7 are each independently 0, 1, 2, 3, or 4.

2. The targeting ligand represented by formula III or the pharmaceutically acceptable salt thereof according to claim 1, wherein:
L₁ is -(CH₂)₆-, -(CH₂)₆-NH-C(=O)-(CH₂)₃-, or -(CH₂)₆-O-CH₂-.

3. The targeting ligand represented by formula III or the pharmaceutically acceptable salt thereof according to claim 1 or 2, wherein the targeting group TL is formula (III-d): 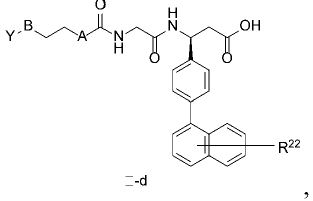
wherein R²² is formula (L) 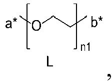 wherein a* represents a point of attachment to naphthyl, b* represents a point of attachment to triazolyl in formula III, and n1 is selected from the group consisting of integers from 2 to 20;
Y is 5-14 membered heteroaryl, and the 5-14 membered heteroaryl is optionally substituted with one or more groups as follows: halogen, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl, wherein the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl, or 5- to 6-membered heteroaryl is optionally substituted with one or more substituents selected from the group consisting of halogen, hydroxy, oxo, nitro, and cyano;
B is selected from the group consisting of -CH₂-, -NH-, 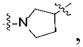 and absent;
A is selected from the group consisting of -CH₂-, -NH-, -O-, and absent.

4. The targeting ligand represented by formula III or the pharmaceutically acceptable salt thereof according to claim 3, wherein Y is selected from the group consisting of

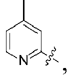

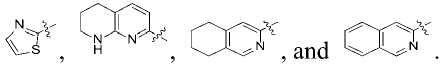

5. The targeting ligand represented by formula III or the pharmaceutically acceptable salt thereof according to claim 3 or 4, wherein the targeting group TL is formula (III-e-1) or formula (III-e-2): 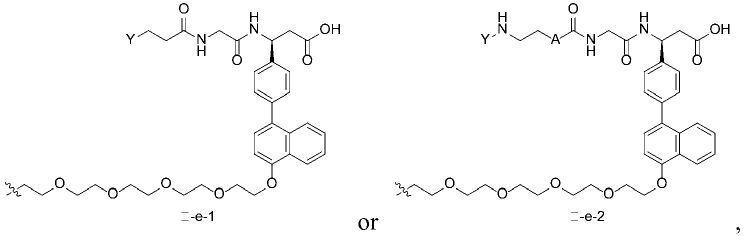 wherein Y is as defined in claim 3 or 4, and A is as defined in claim 3.

6. The targeting ligand represented by formula III or the pharmaceutically acceptable salt thereof according to any one of claims 1-5, wherein the targeting group TL is selected from the group consisting of (III-f)-(III-p): 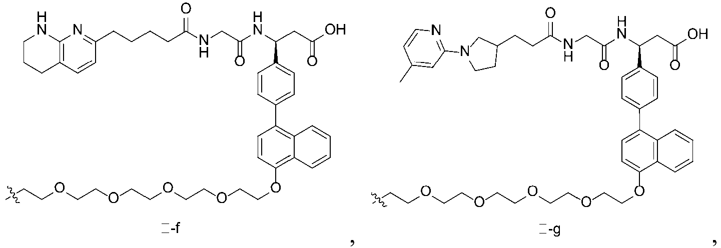 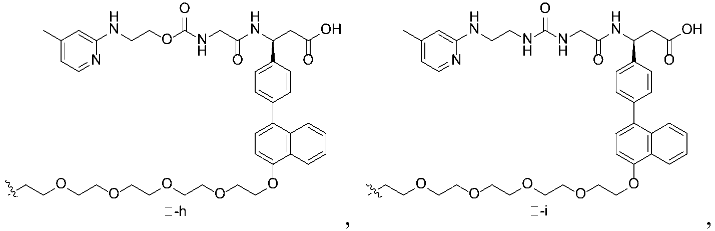 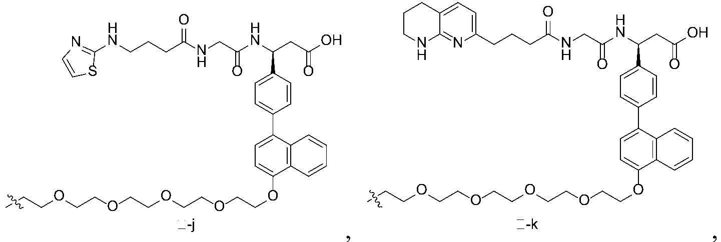 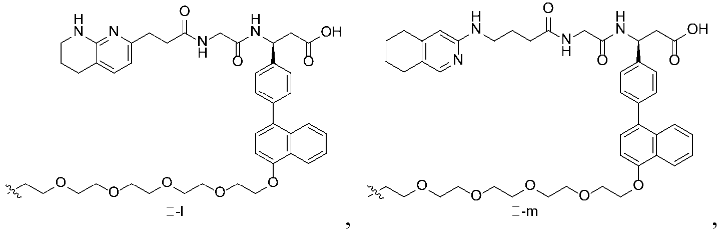 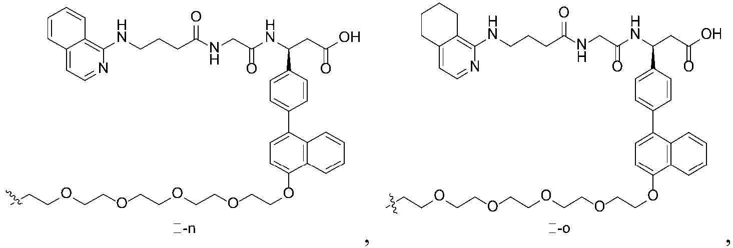 and

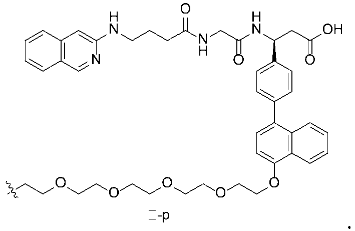

7. The targeting ligand represented by formula III or the pharmaceutically acceptable salt thereof according to any one of claims 1-6, wherein the targeting ligand is selected from the group consisting of the following structures: 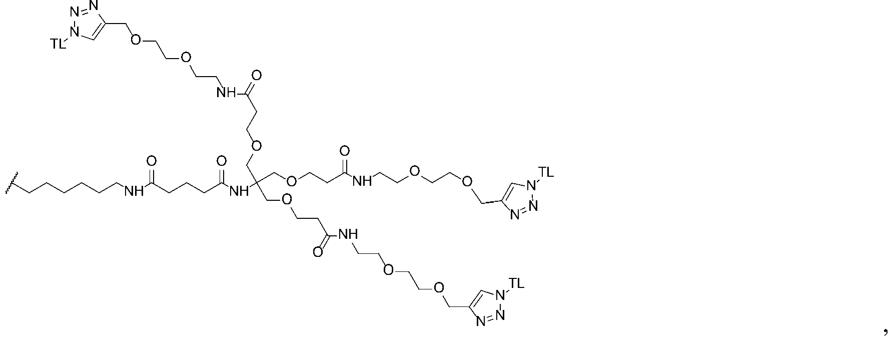 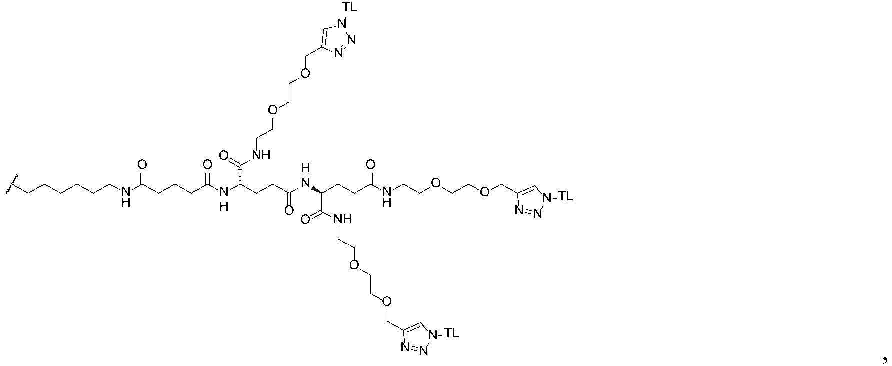 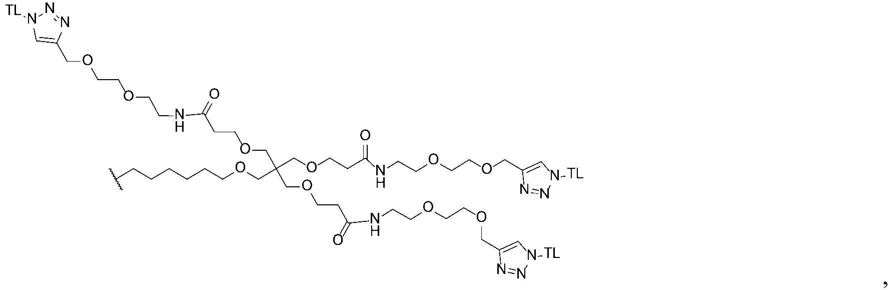 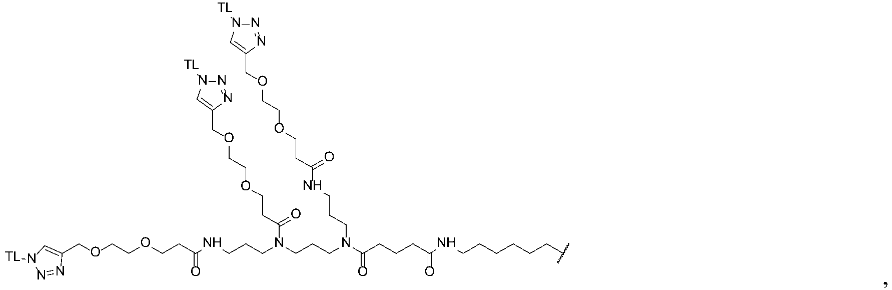 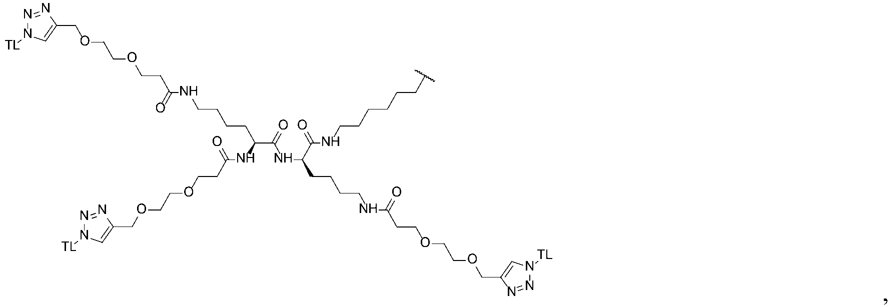 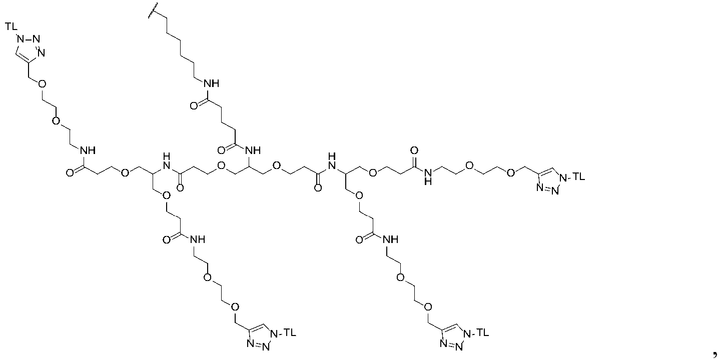 and 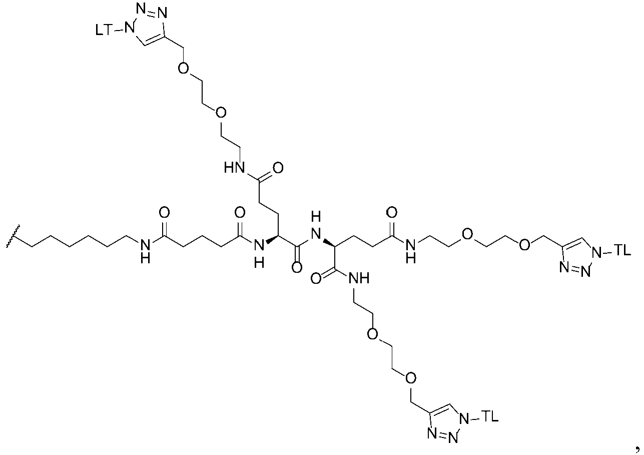 wherein TL is as defined in any one of claims 1-6.

8. The targeting ligand represented by formula III or the pharmaceutically acceptable salt thereof according to any one of claims 1-6, wherein the targeting ligand is selected from the group consisting of the following structures: 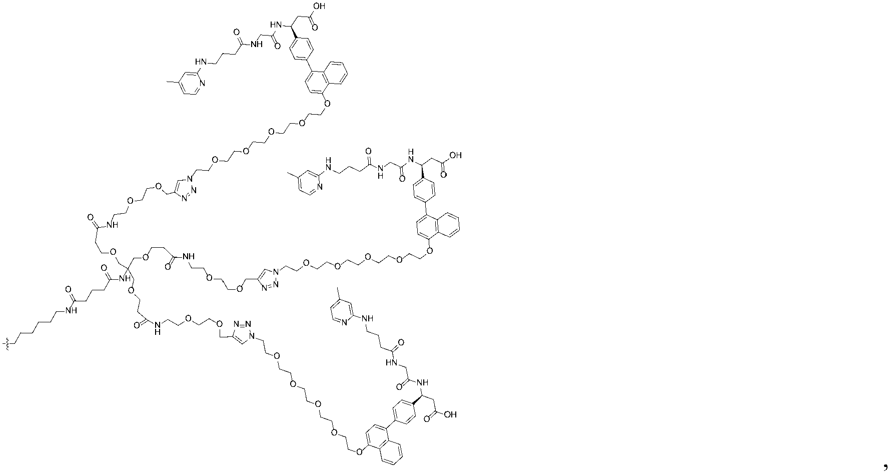 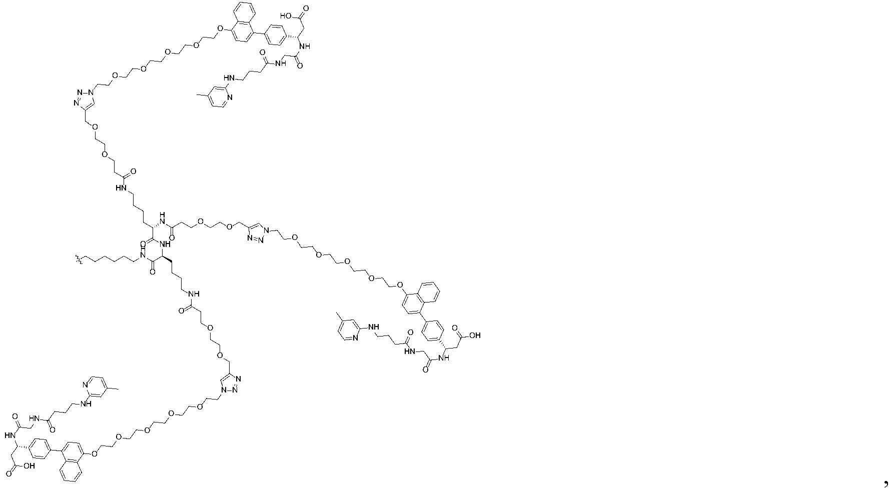 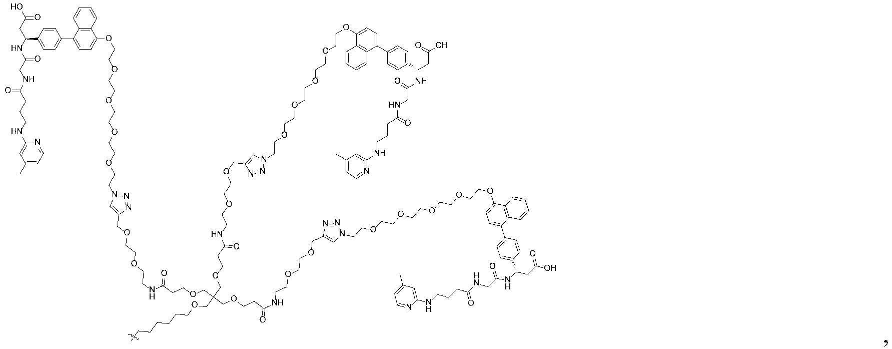 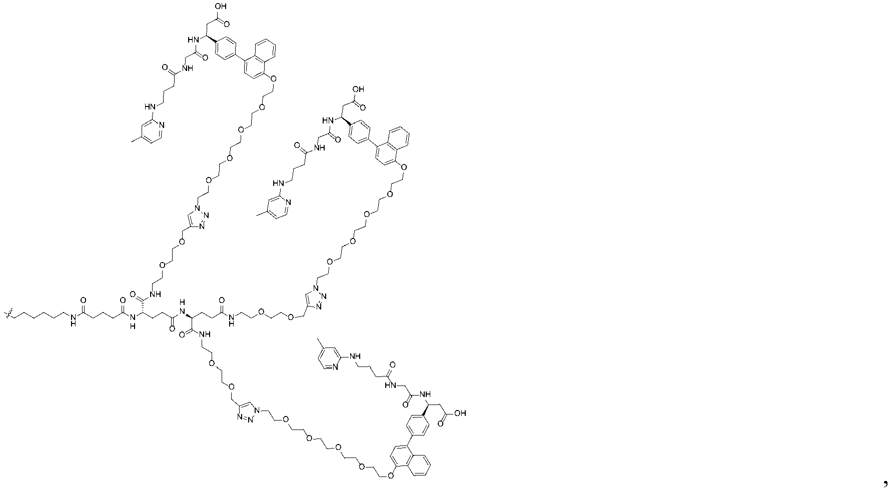 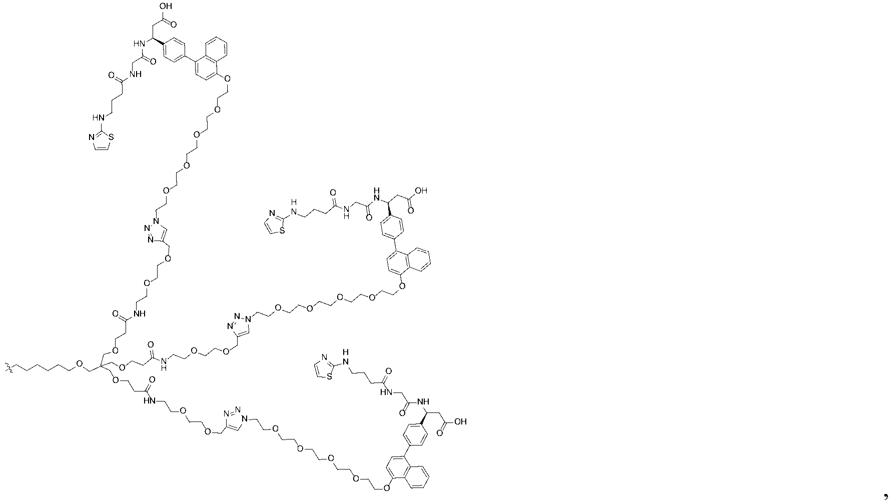 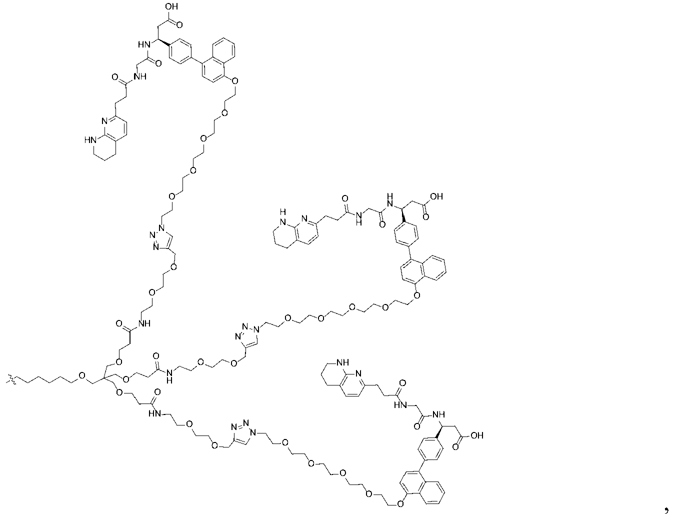 and

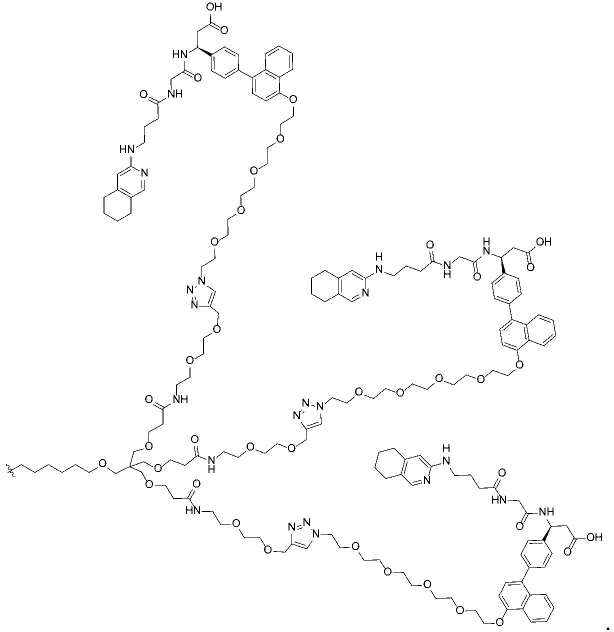

9. A nucleic acid-targeting ligand conjugate or a pharmaceutically acceptable salt thereof, wherein the nucleic acid-targeting ligand conjugate comprises a nucleic acid and one or more targeting ligands conjugated to the nucleic acid; the targeting ligands are as described in any one of claims 1-8, and each of the targeting ligands is identical or different.

10. The nucleic acid-targeting ligand conjugate or the pharmaceutically acceptable salt thereof according to claim 9, wherein the nucleic acid is an siRNA comprising a sense strand and an antisense strand that are partially complementary;
preferably, the nucleic acid is an siRNA targeting RAGE, an siRNA targeting MUC5B, or an siRNA targeting APP;
preferably, the 5' end of the sense strand in the siRNA is conjugated to a targeting ligand.

11. The nucleic acid-targeting ligand conjugate or the pharmaceutically acceptable salt thereof according to claim 9 or 10, wherein the nucleic acid-targeting ligand conjugate is represented by formula V, 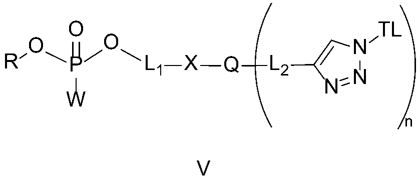
wherein R is the nucleic acid; W is OH, SH, O⁻, or S⁻;
L₁, L₂, X, Q, and TL are as defined in any one of claims 1-6.

12. The nucleic acid-targeting ligand conjugate or the pharmaceutically acceptable salt thereof according to any one of claims 9-11, wherein the nucleic acid-targeting ligand conjugate is selected from the group consisting of the following structures: 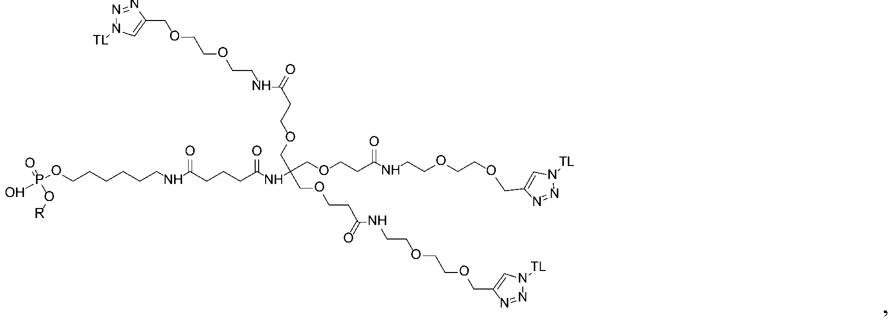 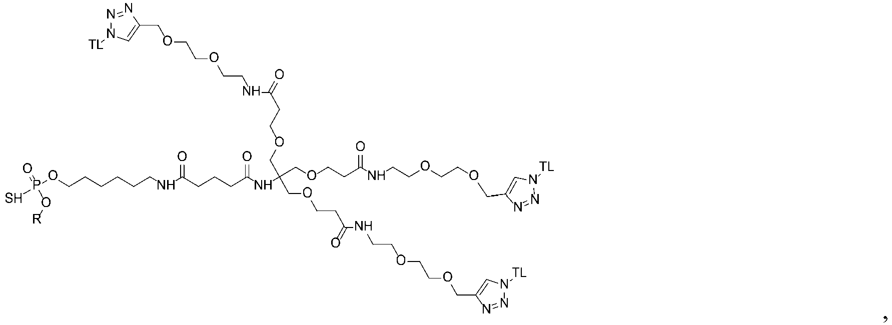 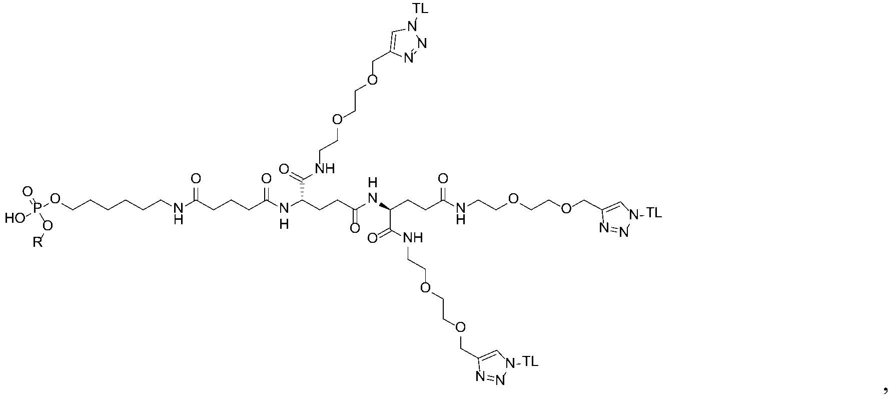 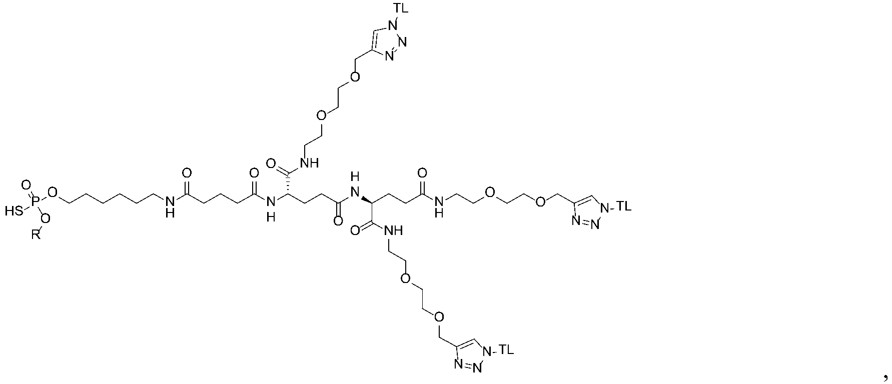 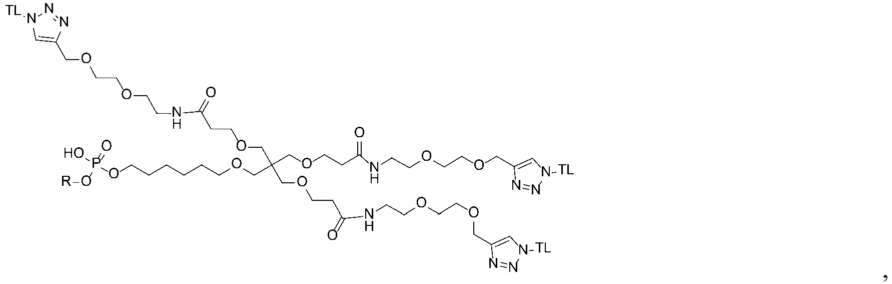 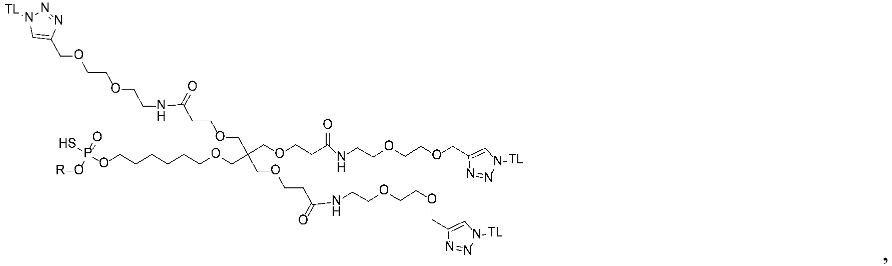 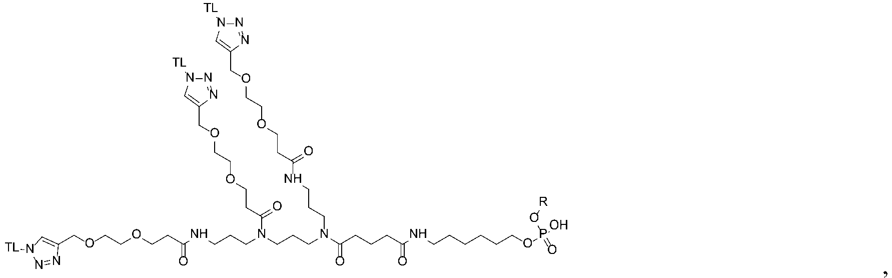 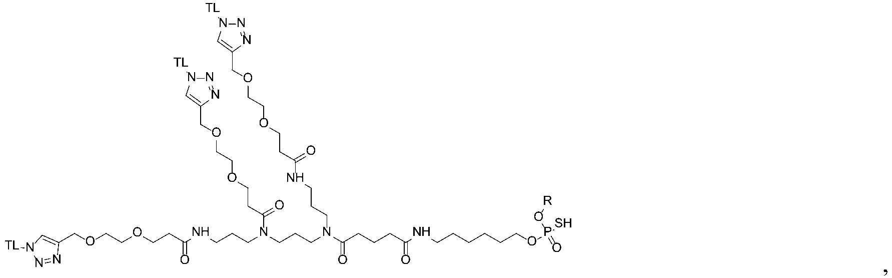 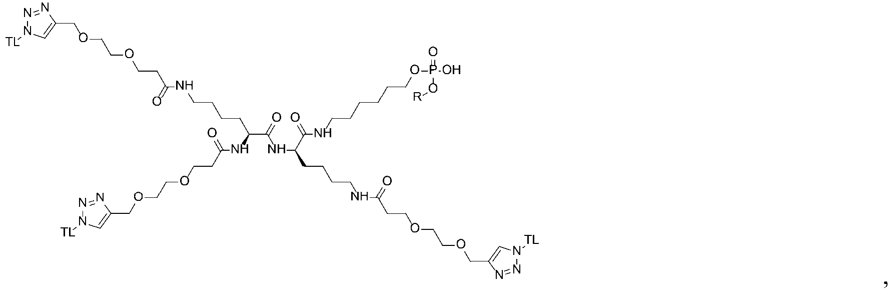 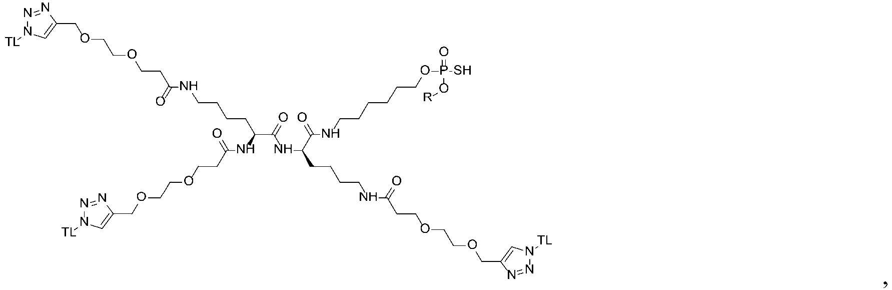 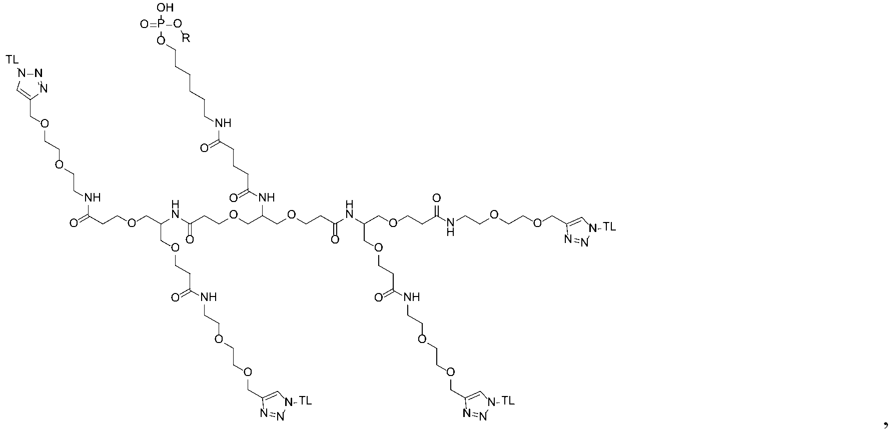 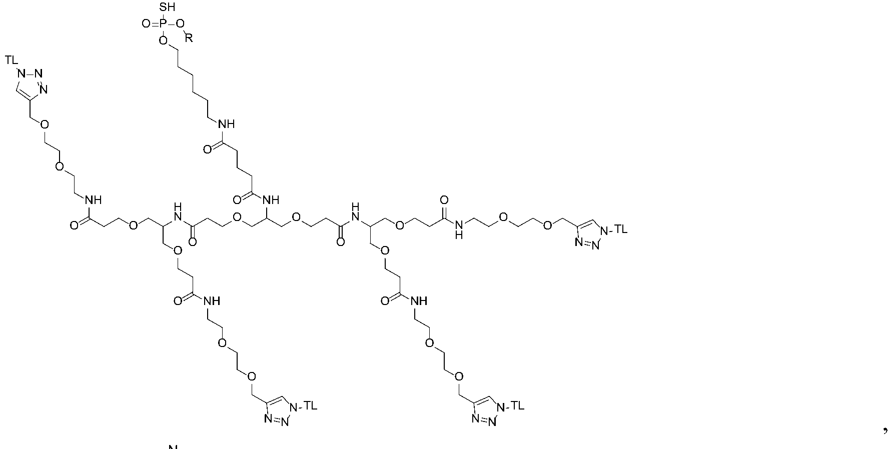 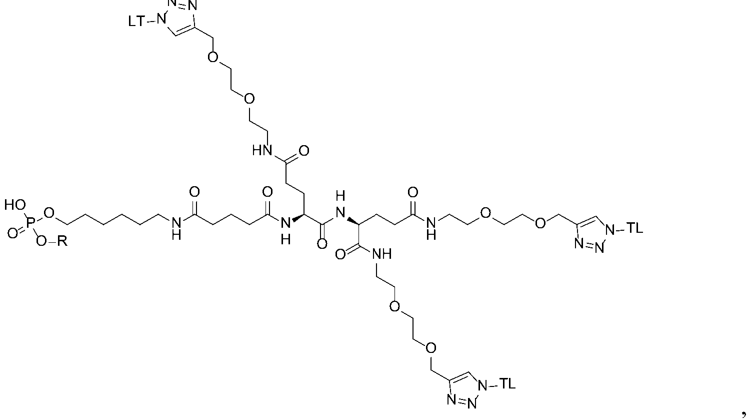 and 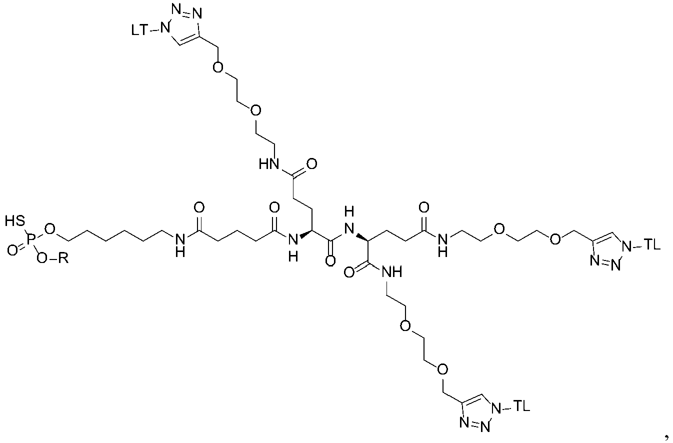 wherein R is a nucleic acid, and the nucleic acid is as defined in claim 10; TL is as defined in any one of claims 1-6.

13. A pharmaceutical composition, comprising the nucleic acid-targeting ligand conjugate or the pharmaceutically acceptable salt thereof according to any one of claims 9-12, and one or more pharmaceutically acceptable excipients.

14. Use of the nucleic acid-targeting ligand conjugate or the pharmaceutically acceptable salt thereof according to any one of claims 9-12 or the pharmaceutical composition according to claim 13 in the manufacture of a medicament for preventing and/or treating a respiratory disease.

15. A preparation method for the nucleic acid-targeting ligand conjugate according to any one of claims 9-12, comprising a step of preparing the nucleic acid-targeting ligand conjugate from a nucleic acid.
